# EUROPEAN PATENT SPECIFICATION

(11) **EP 3 927 284 B1**
(45) Date of publication and mention of the grant of the patent: **09.07.2025**
(21) Application number: 20712143.5
(22) Date of filing: 19.02.2020
(51) Int. Cl.: A61F 2/24, A61M 25/01

(54) **COUNTERFLEXING STEERABLE CATHETER FOR TRANSCATHETER HEART VALVE THERAPY**
GEGENBIEGENDER LENKBARER KATHETER FÜR DIE TRANSKATHETERHERZKLAPPENTHERAPIE
CATHÉTER ORIENTABLE À CONTRE-FLEXION POUR THÉRAPIE DE VALVULE CARDIAQUE PAR TRANSCATHÉTER

(30) Priority: 20.02.2019 US 201962808200 P
(43) Date of publication of application: 29.12.2021
(73) Proprietor: Edwards Lifesciences Corporation, Irvine, CA 92614 (US)
(72) Inventor: DIXON, Eric, Robert, Irvine, CA 92614 (US)
(74) Representative: Eisenführ Speiser
(86) International application number: PCT/US2020/018751
(87) International publication number: WO 2020/172224

(56) References cited:
- WO-A1-2018/112429
- US-A1- 2010 331 776
- US-A1- 2014 135 685
- US-A1- 2018 071 492

## Description

### CROSS-REFERENCE TO RELATED APPLICATIONS

The present application claims the benefit of US Provisional Patent Application Serial No. 62/808,200, filed February 20, 2019 titled "Counterflexing Steerable Catheter for Transcatheter Heart Valve Therapy."

### BACKGROUND OF THE INVENTION

The native heart valves (i.e., the aortic, pulmonary, tricuspid, and mitral valves) serve critical functions in assuring the forward flow of an adequate supply of blood through the cardiovascular system. These heart valves can be damaged, and thus rendered less effective, for example, by congenital malformations, inflammatory processes, infectious conditions, disease, etc. Such damage to the valves can result in serious cardiovascular compromise or death. Damaged valves can be surgically repaired or replaced during open heart surgery. However, open heart surgeries are highly invasive, and complications may occur. Transvascular techniques can be used to introduce and implant prosthetic devices in a manner that is much less invasive than open heart surgery. As one example, a transvascular technique useable for accessing the native mitral and aortic valves is the trans-septal technique. The trans-septal technique comprises advancing a catheter into the right atrium (e.g., inserting a catheter into the right femoral vein, up the inferior vena cava and into the right atrium). The septum is then punctured, and the catheter passed into the left atrium. A similar transvascular technique can be used to implant a prosthetic device within the tricuspid valve that begins similarly to the trans-septal technique but stops short of puncturing the septum and instead turns the delivery catheter toward the tricuspid valve in the right atrium.

A healthy heart has a generally conical shape that tapers to a lower apex. The heart is four-chambered and comprises the left atrium, right atrium, left ventricle, and right ventricle. The left and right sides of the heart are separated by a wall generally referred to as the septum. The native mitral valve of the human heart connects the left atrium to the left ventricle. The mitral valve has a very different anatomy than other native heart valves. The mitral valve includes an annulus portion, which is an annular portion of the native valve tissue surrounding the mitral valve orifice, and a pair of cusps, or leaflets, extending downward from the annulus into the left ventricle. The mitral valve annulus can form a "D"-shaped, oval, or otherwise out-of-round cross-sectional shape having major and minor axes. The anterior leaflet can be larger than the posterior leaflet, forming a generally "C"-shaped boundary between the abutting sides of the leaflets when they are closed together.

When operating properly, the anterior leaflet and the posterior leaflet function together as a one-way valve to allow blood to flow only from the left atrium to the left ventricle. The left atrium receives oxygenated blood from the pulmonary veins. When the muscles of the left atrium contract and the left ventricle dilates (also referred to as "ventricular diastole" or "diastole"), the oxygenated blood that is collected in the left atrium flows into the left ventricle. When the muscles of the left atrium relax and the muscles of the left ventricle contract (also referred to as "ventricular systole" or "systole"), the increased blood pressure in the left ventricle urges the sides of the two leaflets together, thereby closing the one-way mitral valve so that blood cannot flow back to the left atrium and is instead expelled out of the left ventricle through the aortic valve. To prevent the two leaflets from prolapsing under pressure and folding back through the mitral annulus toward the left atrium, a plurality of fibrous cords called chordae tendineae tether the leaflets to papillary muscles in the left ventricle.

Valvular regurgitation involves the valve improperly allowing some blood to flow in the wrong direction through the valve. For example, mitral regurgitation occurs when the native mitral valve fails to close properly and blood flows into the left atrium from the left ventricle during the systolic phase of heart contraction. Mitral regurgitation is one of the most common forms of valvular heart disease. Mitral regurgitation can have many different causes, such as leaflet prolapse, dysfunctional papillary muscles, stretching of the mitral valve annulus resulting from dilation of the left ventricle, more than one of these, etc. Mitral regurgitation at a central portion of the leaflets can be referred to as central jet mitral regurgitation and mitral regurgitation nearer to one commissure (i.e., location where the leaflets meet) of the leaflets can be referred to as eccentric jet mitral regurgitation. Central jet regurgitation occurs when the edges of the leaflets do not meet in the middle and thus the valve does not close, and regurgitation is present.

WO2018112429A1 describes various configurations of a delivery catheter configured to deliver an anchoring device to a native valve annulus of a patient's heart, where the anchoring device can better secure a prosthesis at the native annulus. The delivery catheter includes a distal section that includes one or more spines or is laser cut to assume a particular shape for better facilitating delivery of the anchoring device at the implant site and can include a flexible tube, a first pull wire, and a second pull wire. The flexible tube can comprise a bore extending between the first end and the second end sized for passing the anchoring device therethrough. The distal portion can comprise a first flexing section and a second flexing section. Actuation of the first pull wire and the second pull wire can cause the flexible tube to move from a first configuration to a second configuration.

### SUMMARY

This summary is meant to provide some examples and is not intended to be limiting of the scope of the invention in any way. For example, any feature included in an example of this summary is not required by the claims, unless the claims explicitly recite the features.

The claimed invention as defined in independent claim 1 relates to a delivery device for delivering an implant to a native valve of a patient's heart. The delivery device includes a flexible hollow tube extending from a proximal end to a distal end. A first flexible portion extends from the proximal end to a middle portion. A second flexible portion extends from the middle portion to the distal end. A first pull wire attaches to the middle portion such that applying tension to the first pull wire causes the first flexible portion to bend in a first bend direction. A second pull wire attaches to the distal end such that applying tension to the second pull wire causes the second flexible portion to bend in a second bend direction. The first bend direction being offset from the second bend direction by about 160 to about 200 degrees.

Some preferred configurations of the claimed device are defined in dependent claims 2 to 14.

The claimed invention as defined in independent claim 15 relates to a method of manufacturing a device for delivering an implant to a native valve of a patient's heart. The method comprises providing a flexible hollow tube. The flexible hollow tube extends from a proximal end to a distal end. The flexible hollow tube includes a middle portion disposed between the proximal end and distal end. A plurality of first slots are cut into the flexible hollow tube between the proximal end and the middle portion to form a first flexible portion extending from the proximal end to the middle portion. A plurality of second slots are cut into the flexible hollow tube between the middle portion and the distal end to form a second flexible portion extending from the middle portion to the distal end. A first wire is attached to the middle portion. The first pull wire extending through the first flexible portion along a first side of the flexible hollow tube. Applying tension to the first pull wire in a proximal direction creates a first bend in the first flexible portion in a first direction. A second pull wire is attached to the distal end. The second pull wire extends through the first and second flexible portions along a second side of the flexible hollow tube. Applying tension to the second pull wire in a proximal direction creates a second bend in the second flexible portion in a second direction. The second side is offset from the first side by an offset angle so that the second direction of the second bend is also offset from the first direction of the first bend by the offset angle. The offset angle is in a range of about 160 degrees to about 200 degrees.

In one example method of delivering a heart valve implant using a delivery catheter, the delivery catheter is advanced into an atrium (e.g., to the right atrium, across a septum to the left atrium, etc.). The delivery catheter comprises a distal end, a first flexible portion, a second flexible portion, and a middle portion connecting the first flexible portion and the second flexible portion. A first curve is created in the first flexible portion by tensioning a first pull wire connected to the middle portion. Creating the first curve moves the distal end of the delivery catheter away from a native valve. A second curve in the proximal section is created by tensioning a second pull wire connected to the distal end. Creating the second curve moves the distal end toward the native valve. This method can be performed on a living animal or on a simulation, such as on a cadaver, cadaver heart, simulator (e.g. with the body parts, heart, tissue, etc. being simulated), etc.

In one example embodiment, a device for delivering an implant to a native valve of a patient's heart includes a flexible hollow tube, a first pull wire, and a second pull wire. The flexible hollow tube has a proximal region and a distal region. The distal region of the flexible hollow tube comprises a distal section and a proximal section. A first pull wire is fixed to the distal end of the distal section. A second pull wire is fixed to a distal end of the proximal section. The second pull wire is offset from the first pull wire. In some embodiments, the second pull wire is offset from the first pull wire by 180 degrees or about 180 degrees. Applying tension to the first pull wire in a proximal direction creates a first bend in the distal section in a first direction. Applying tension to the second pull wire in a proximal direction creates a second bend in the proximal section in a second direction different from the first direction. In some embodiments, the second direction is opposite or substantially opposite to the first direction.

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of embodiments of the present disclosure, a more particular description of the certain embodiments will be made by reference to various aspects of the appended drawings. It is appreciated that these drawings depict only typical embodiments of the present disclosure and are therefore not to be considered limiting of the scope of the disclosure. Moreover, while the figures can be drawn to scale for some embodiments, the figures are not necessarily drawn to scale for all embodiments. Embodiments and other features and advantages of the present disclosure will be described and explained with additional specificity and detail through the use of the accompanying drawings in which:
Figure 1 illustrates a cutaway view of the human heart in a diastolic phase;
Figure 2 illustrates a cutaway view of the human heart in a systolic phase;
Figure 3 illustrates a cutaway view of the human heart in a diastolic phase, in which the chordae tendineae are shown attaching the leaflets of the mitral and tricuspid valves to ventricle walls;
Figure 4 illustrates a healthy mitral valve with the leaflets closed as viewed from an atrial side of the mitral valve;
Figure 5 illustrates a dysfunctional mitral valve with a visible gap between the leaflets as viewed from an atrial side of the mitral valve;
Figure 6 illustrates a mitral valve having a wide gap between the posterior leaflet and the anterior leaflet;
Figure 7 illustrates a tricuspid valve viewed from an atrial side of the tricuspid valve;
Figures 8-14 show an example embodiment of an implantable prosthetic device, in various stages of deployment;
Figure 11A shows an example embodiment of an implantable prosthetic device that is similar to the device illustrated by Figure 11, but where the paddles are independently controllable;
Figures 15-20 show the implantable prosthetic device of Figures 8-14 being delivered and implanted within the native mitral valve;
Figures 17A, 18A, and 19A show the implantable prosthetic device of Figures 8-14 being delivered and implanted within the native mitral valve by an example counterflexing delivery device;
Figures 21-32 show an example embodiment of an implantable prosthetic device being delivered and implanted within the native mitral valve;
Figures 24A, 25A, and 32A show the implantable prosthetic device of Figures 21-32 being delivered and implanted within the native mitral valve by a counterflexing delivery device in accordance with an example embodiment;
Figure 33 shows a perspective view of an example distal section of a delivery catheter used as part of an example delivery device for implanting an anchoring device;
Figure 34 is a cross-sectional view of several links of the distal section of Figure 33;
Figure 35 is a perspective view of the distal section of the delivery catheter in a bent or curved configuration;
Figure 36 is a flat view of an example laser cut sheet that can be used for forming a distal section of a delivery catheter;
Figure 37 is a flat view of another example laser cut sheet that can be used for forming a distal section of a delivery catheter;
Figure 38 is a flat view of another example laser cut sheet that can be used for forming a distal section of a delivery catheter;
Figure 39 shows a perspective view of a bent or curved configuration of a distal segment of a delivery catheter having two flexed sections usable for implanting an anchoring device at a native valve, e.g., using a transseptal technique;
Figure 40 shows a schematic side view of an example distal section of a delivery catheter with an example two control wire or pull wire system that can be used in various delivery catheters or delivery devices herein;
Figure 41 shows a schematic perspective view of the delivery catheter of Figure 40 in a partially actuated state;
Figure 42 shows a schematic perspective view of the delivery catheter of Figure 40 in a fully actuated state;
Figure 43A is an end view of another example embodiment of a delivery catheter;
Figure 43B is a sectional view taken along the plane indicated by lines B-B in Figure 43A;
Figure 43C is a sectional view taken along the plane indicated by lines D-D in Figure 43B:
Figure 43D is a sectional view taken along the plane indicated by lines C-C in FIG. 43B;
Figure 44A shows a schematic perspective view of a distal section of the delivery catheter of Figures 43A-43D in a partially actuated state;
Figure 44B shows a schematic perspective view of the distal section of the delivery catheter of Figures 43A-43D in a more actuated state or more curved actuated state;
Figure 45A is a partial view of the delivery catheter of Figures 43A-43D;
Figures 45B-45D show cross-sectional views of the delivery catheter shown in Figure 45A, the cross-sections taken in a plane perpendicular to a longitudinal axis of the delivery catheter;
Figure 46 shows a schematic view of an example two pull wire system for the delivery catheter shown in Figures 43A-43D;
Figure 47A shows a top view of a distal section of a delivery catheter used as part of the delivery device for implanting a valve repair or replacement device, according to an example embodiment;
Figure 47B shows a side view of the distal section of a delivery catheter illustrated in Figure 47A;
Figure 47C shows a bottom view of a distal section of a delivery catheter illustrated in Figure 47A;
Figure 47D shows an end view of the distal section of a delivery catheter illustrated in Figure 47A;
Figure 48A shows a flat view of a laser cut sheet for the distal section of the delivery catheter according to the example embodiment of Figure 47A;
Figure 48B shows a close-up view of a portion of the laser cut sheet of Figure 48A;
Figure 49A shows a side view of a distal shaft of a catheter according to an example embodiment;
Figure 49B shows an end view of the distal shaft of the steerable catheter, according to the embodiment of Figure 49A;
Figure 50A shows a side view of the distal section of a delivery catheter with control wires running therethrough, according to an example embodiment;
Figure 50B shows a side view of the distal section of a delivery catheter of Figure 50A and how it moves when a control wire is pulled, according to an example embodiment;
Figure 51 shows a cross-section view of the steerable catheter of Figure 49A taken along line BB-BB of Figure 49A;
Figure 52A shows a cross-section view of the steerable catheter of Figure 49A taken along line CC-CC of Figure 49A;
Figure 52B shows a close up of a portion of the cross-section view of Figure 52A, having a control wire;
Figure 53 shows a cross-section view of the steerable catheter of Figure 49A taken along line DD-DD of Figure 49A;
Figure 54 shows a cross-section view of the steerable catheter of Figure 49A taken along line EE-EE of Figure 49A;
Figure 55A shows a flat view of a proximal anchor ring for the distal portion of the delivery catheter, according to an example embodiment;
Figure 55B shows a close-up view of a portion of the anchor ring as shown in Figure 55A, having weld holes;
Figure 56A shows a top view of the anchor ring of Figure 55A with a hypotube anchor according to an example embodiment;
Figure 56B shows a side view of the anchor ring of Figure 56A;
Figure 56C shows an end view of the anchor ring of Figure 56A;
Figure 57A shows a flat view of a proximal coil stopper according to an example embodiment;
Figure 57B shows a close-up of weld holes in the proximal coil stopper of Figure 57A;
Figure 58A shows a top view of a proximal coil stopper with a portion of a coil, according to an example embodiment;
Figure 58B shows a cross-section view of the proximal coil stopper and coil taken along line X-X of Figure 58A according to an example embodiment;
Figure 58C shows an end view of the proximal end of the proximal coil stopper and coil of Figure 58A;
Figure 59A shows a schematic of a counterflexing steerable catheter having two pull wire controls, in a straight configuration in accordance with an example embodiment;
Figure 59B shows a schematic of the counterflexing steerable catheter of Figure 59A with a flexed distal segment, in accordance with an example embodiment;
Figure 59C shows a schematic of a counterflexing steerable catheter of Figure 59A with flexed distal and proximal segments, in accordance with an example embodiment;
Figure 60A shows a schematic of a counterflexing steerable catheter having a clutch-type pull wire control mechanism in a disengaged configuration, in accordance with an example embodiment;
Figure 60B shows the schematic of a counterflexing steerable catheter of Figure 60A having a clutch-type control mechanism in an engaged configuration;
Figure 61 shows the schematic of a counterflexing steerable catheter having one pull wire control with slack to control when the proximal pull wire is tensioned, in accordance with an example embodiment;
Figures 62A-62C shows a schematic of a counterflexing steerable catheter having different stiffness properties along the length and circumference of the catheter to control the flex, in accordance with an example embodiment;
Figure 63 shows a schematic of a counterflexing steerable catheter having a different flex/wheel turn ratio for each of the pull wires to pull them at different rates, in accordance with an example embodiment;
Figure 64 shows a schematic of a counterflexing steerable catheter having a different screw pitch for each of the pull wires to pull them at different rates, in accordance with an example embodiment;
Figure 65 shows a schematic of a counterflexing steerable catheter having a single pull wire to curve each of two flex regions in two different direction, in accordance with an example embodiment;
Figure 66 shows a top view of a distal section of a delivery catheter used as part of the delivery device for implanting a valve repair or replacement device, according to an example embodiment;
Figure 67 shows a side view of the distal section of a delivery catheter illustrated in Figure 66;
Figure 68 shows a bottom view of a distal section of a delivery catheter illustrated in Figure 66;
Figure 69 shows an end view of the distal section of a delivery catheter illustrated in Figure 66;
Figure 70 shows a side view of the distal section of a delivery catheter with control wires running therethrough, according to an example embodiment; and
Figure 71 shows a side view of the distal section of a delivery catheter of Figure 70 and how it moves when a control wire is pulled, according to an example embodiment.

### DETAILED DESCRIPTION

The following description refers to the accompanying drawings, which illustrate specific embodiments of the present disclosure. Other embodiments having different structures and operation do not depart from the scope of the present disclosure.

Example embodiments of the present disclosure are directed to devices and methods for repairing a defective heart valve. It should be noted that various embodiments of native valve reparation devices and systems for delivery are disclosed herein, and any combination of these options can be made unless specifically excluded. In other words, individual components of the disclosed devices and systems can be combined unless mutually exclusive or otherwise physically impossible.

As described herein, when one or more components are described as being connected, joined, affixed, coupled, attached, or otherwise interconnected, such interconnection may be direct as between the components or may be indirect such as through the use of one or more intermediary components. Also as described herein, reference to a "member," "component," or "portion" shall not be limited to a single structural member, component, or element but can include an assembly of components, members, or elements. Also as described herein, the terms "substantially" and "about" are defined as at least close to (and includes) a given value or state (preferably within 10% of, more preferably within 1% of, and most preferably within 0.1% of).

Example embodiments of a catheter that can flex down to point at the native valve, such as the mitral valve, and also raise up to give more height to an implant above the native valve, during a transseptal procedure of implanting the implant. The implant can take a wide variety of different forms. In some embodiments, the implant can be configured as a valve repair device, such as the PASCAL system produced by Edwards Lifesciences Corporation, are described herein. Various implementation and user interface controls are described herein. This system and method of using the system can be used when a transseptal puncture is lower than an optimal or target position. The lower position reduces the space between the end of the catheter and the native valve annulus (e.g., mitral valve annulus, tricuspid valve anulus, etc.). The system and method can be used to create more space between the end of the catheter where the implant attaches and the valve leaflets. By raising the level of the implant, procedural steps such as clocking the implant, grasping the leaflets, disengaging the leaflets from the paddles, and repositioning the leaflets can be performed more easily and/or efficiently.

The example embodiments described herein can have a steerable catheter with a distal region, having a flex section at the distalmost end of the catheter in the distal region, referred to herein as the distal flex section, and a flex section located in the distal region proximal to the distal flex section, referred to herein as the proximal flex section. The distal flex section can flex to form a bend of 170 degrees or about 170 degrees. The proximal flex section can flex to form a bend of 60 degrees or about 60 degrees. The distal flex section can have a pull wire (which is also referred to herein as a control wire) on the inferior side of the catheter which directs the flex to bend inferior toward the valve. The proximal flex section has a pull wire on the superior side of the catheter which directs the flex to bend away from the valve. When both the distal flex section and proximal flex section are flexed, the proximal flex section can direct the distal region of the catheter in an upward direction, and the distal flex section can direct the distal end of the catheter in an inferior direction, down towards the heart valve. These two flexes result in an overall shape similar to a "question mark" that ultimately points in an inferior direction, with a superior bend to gain height above the valve. In the "question mark" configuration, the distal end is directed in an inferior direction by the distal flex section bend and raised superior to the native valve by the proximal flex section bend.

The distal flex section and proximal flex section can be controlled by a single flex wheel or by two independently controllable flex wheels. The flexing can be performed in any order, such as the distal flex section bending first, followed by a proximal flex section bend, or vice versa. The two flex sections can also bend simultaneously. In the example embodiments described herein, the simultaneous flexing of the proximal and distal flex sections can be used to maintain the direction that the distal end of the catheter is pointed while the height of the distal end is raised. Simultaneous flexing causes the position and trajectory changes of the distal end to be compensated for by additional flex on the distal flex section.

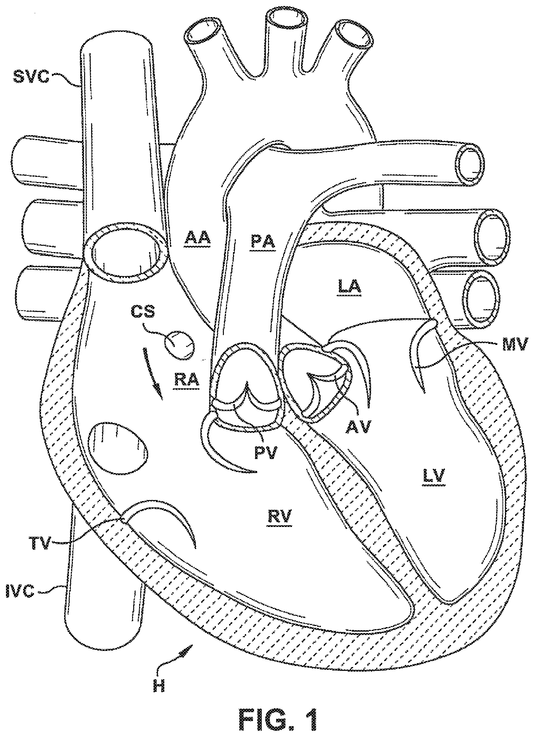

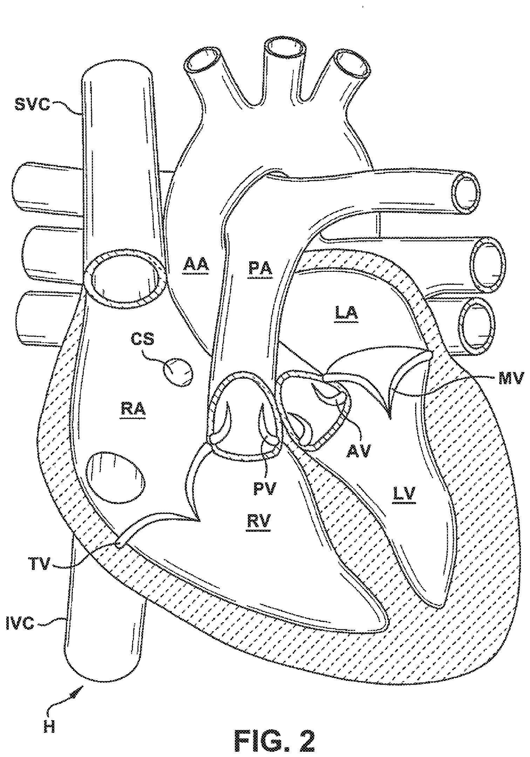

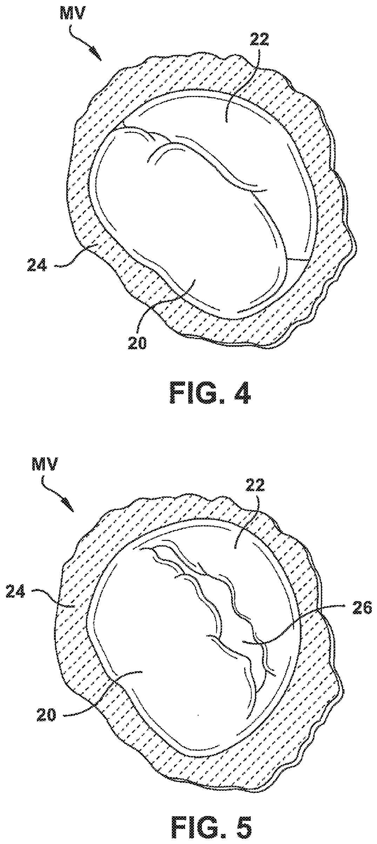

Figures 1 and 2 are cutaway views of the human heart H in diastolic and systolic phases, respectively. The right ventricle RV and left ventricle LV are separated from the right atrium RA and left atrium LA, respectively, by the tricuspid valve TV and mitral valve MV; i.e., the atrioventricular valves. Additionally, the aortic valve AV separates the left ventricle LV from the ascending aorta AA, and the pulmonary valve PV separates the right ventricle from the pulmonary artery PA. Each of these valves has flexible leaflets (e.g., leaflets 20, 22 shown in Figures 4 and 5) extending inward across the respective orifices that come together or "coapt" in the flow stream to form the one-way, fluid-occluding surfaces. The native valve repair systems of the present application are described primarily with respect to the mitral valve MV. Therefore, anatomical structures of the left atrium LA and left ventricle LV will be explained in greater detail. It should be understood that the devices described herein may also be used in repairing other native valves, e.g., the devices can be used in repairing the tricuspid valve TV, the aortic valve AV, and the pulmonary valve PV.

The left atrium LA receives oxygenated blood from the lungs. During the diastolic phase, or diastole, seen in Figure 1, the blood that was previously collected in the left atrium LA (during the systolic phase) moves through the mitral valve MV and into the left ventricle LV by expansion of the left ventricle LV. In the systolic phase, or systole, seen in Figure 2, the left ventricle LV contracts to force the blood through the aortic valve AV and ascending aorta AA into the body. During systole, the leaflets of the mitral valve MV close to prevent the blood from regurgitating from the left ventricle LV and back into the left atrium LA, and blood is collected in the left atrium from the pulmonary vein. In one example embodiment, the devices described by the present application are used to repair the function of a defective mitral valve MV. That is, the devices are configured to help close the leaflets of the mitral valve to prevent blood from regurgitating from the left ventricle LV and back into the left atrium LA.

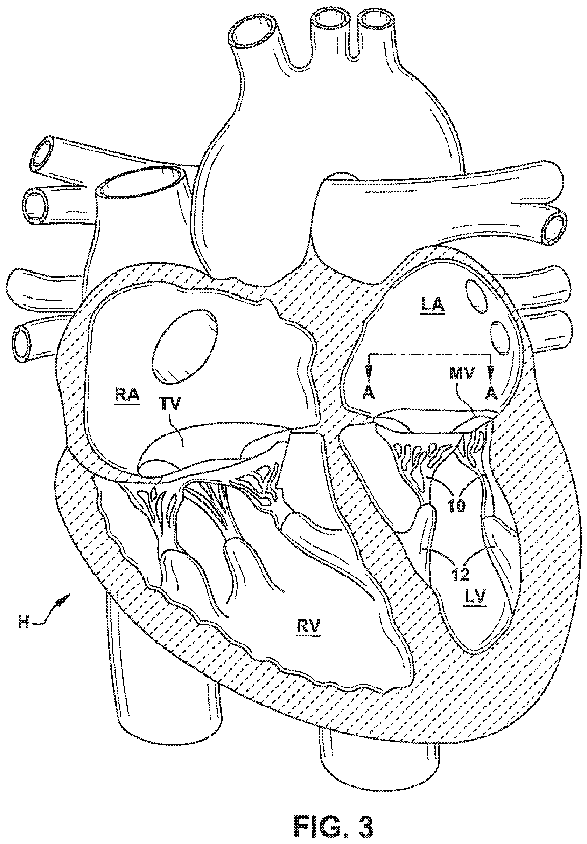

Referring now to Figures 1-7, the mitral valve MV includes two leaflets, the anterior leaflet 20 and the posterior leaflet 22. The mitral valve MV also includes an annulus 24, which is a variably dense fibrous ring of tissues that encircles the leaflets 20, 22. Referring to Figure 3, the mitral valve MV is anchored to the wall of the left ventricle LV by chordae tendineae 10. The chordae tendineae 10 are cord-like tendons that connect the papillary muscles 12 (i.e., the muscles located at the base of the chordae tendineae and within the walls of the left ventricle) to the leaflets 20, 22 of the mitral valve MV. The papillary muscles 12 serve to limit the movements of the mitral valve MV and prevent the mitral valve from being reverted. The mitral valve MV opens and closes in response to pressure changes in the left atrium LA and the left ventricle LV. The papillary muscles do not open or close the mitral valve MV. Rather, the papillary muscles brace the mitral valve MV against the high pressure needed to circulate blood throughout the body. Together the papillary muscles and the chordae tendineae are known as the subvalvular apparatus, which functions to keep the mitral valve MV from prolapsing into the left atrium LA when the mitral valve closes.

Various disease processes can impair proper function of one or more of the native valves of the heart H. These disease processes include degenerative processes (e.g., Barlow's Disease, fibroelastic deficiency), inflammatory processes (e.g., Rheumatic Heart Disease), and infectious processes (e.g., endocarditis). In addition, damage to the left ventricle LV or the right ventricle RV from prior heart attacks (i.e., myocardial infarction secondary to coronary artery disease) or other heart diseases (e.g., cardiomyopathy) can distort a native valve's geometry, which can cause the native valve to dysfunction. However, the majority of patients undergoing valve surgery, such as surgery to the mitral valve MV, suffer from a degenerative disease that causes a malfunction in a leaflet (e.g., leaflets 20, 22) of a native valve (e.g., the mitral valve MV), which results in prolapse and regurgitation.

Generally, a native valve may malfunction in two different ways: (1) valve stenosis; and (2) valve regurgitation. Valve stenosis occurs when a native valve does not open completely and thereby causes an obstruction of blood flow. Typically, valve stenosis results from buildup of calcified material on the leaflets of a valve, which causes the leaflets to thicken and impairs the ability of the valve to fully open to permit forward blood flow.

The second type of valve malfunction, valve regurgitation, occurs when the leaflets of the valve do not close completely thereby causing blood to leak back into the prior chamber (e.g., causing blood to leak from the left ventricle to the left atrium). There are three main mechanisms by which a native valve becomes regurgitant-or incompetent-which include Carpentier's type I, type II, and type III malfunctions. A Carpentier type I malfunction involves the dilation of the annulus such that normally functioning leaflets are distracted from each other and fail to form a tight seal (i.e., the leaflets do not coapt properly). Included in a type I mechanism malfunction are perforations of the leaflets, as are present in endocarditis. A Carpentier's type II malfunction involves prolapse of one or more leaflets of a native valve above a plane of coaption. A Carpentier's type III malfunction involves restriction of the motion of one or more leaflets of a native valve such that the leaflets are abnormally constrained below the plane of the annulus. Leaflet restriction can be caused by rheumatic disease (Ma) or dilation of a ventricle (IIIb).

Referring to Figure 4, when a healthy mitral valve MV is in a closed position, the anterior leaflet 20 and the posterior leaflet 22 coapt, which prevents blood from leaking from the left ventricle LV to the left atrium LA. Referring to Figure 5, regurgitation occurs when the anterior leaflet 20 and/or the posterior leaflet 22 of the mitral valve MV is displaced into the left atrium LA during systole. This failure to coapt causes a gap 26 between the anterior leaflet 20 and the posterior leaflet 22, which allows blood to flow back into the left atrium LA from the left ventricle LV during systole. As set forth above, there are several different ways that a leaflet (e.g. leaflets 20, 22 of mitral valve MV) may malfunction, which can thereby lead to regurgitation.

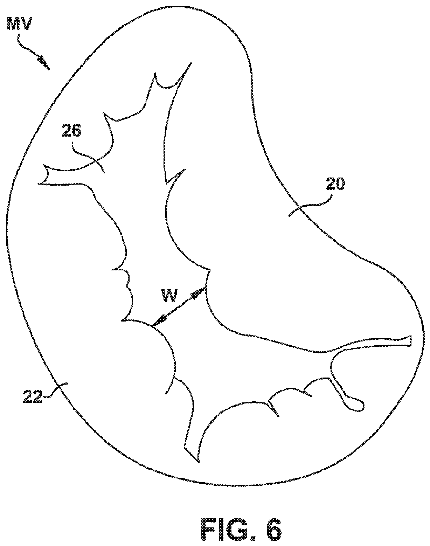

Referring to Figure 6, in certain situations, the mitral valve MV of a patient can have a wide gap 26 between the anterior leaflet 20 and the posterior leaflet 22 when the mitral valve is in a closed position (i.e., during the systolic phase). For example, the gap 26 can have a width W between about 2.5 mm and about 17.5 mm, such as between about 5 mm and about 15 mm, such as between about 7.5 mm and about 12.5 mm, such as about 10 mm. In some situations, the gap 26 can have a width W greater than 15 mm. In any of the above-mentioned situations, a valve repair device is desired that is capable of engaging the anterior leaflet 20 and the posterior leaflet 22 to close the gap 26 and prevent regurgitation of blood through the mitral valve MV.

Although stenosis or regurgitation can affect any valve, stenosis is predominantly found to affect either the aortic valve AV or the pulmonary valve PV, and regurgitation is predominantly found to affect either the mitral valve MV or the tricuspid valve TV. Both valve stenosis and valve regurgitation increase the workload of the heart H and may lead to very serious conditions if left un-treated; such as endocarditis, congestive heart failure, permanent heart damage, cardiac arrest, and ultimately death. Because the left side of the heart (i.e., the left atrium LA, the left ventricle LV, the mitral valve MV, and the aortic valve AV) is primarily responsible for circulating the flow of blood throughout the body, malfunction of the mitral valve MV or the aortic valve AV is particularly problematic and often life threatening. Accordingly, because of the substantially higher pressures on the left side of the heart, dysfunction of the mitral valve MV or the aortic valve AV is often more problematic.

Malfunctioning native heart valves may either be repaired or replaced. Repair typically involves the preservation and correction of the patient's native valve. Replacement typically involves replacing the patient's native valve with a biological or mechanical substitute. Typically, the aortic valve AV and pulmonary valve PV are more prone to stenosis. Because stenotic damage sustained by the leaflets is irreversible, some treatments for a stenotic aortic valve or stenotic pulmonary valve are removal and replacement of the valve with a surgically implanted heart valve, or displacement of the valve with a transcatheter heart valve. The mitral valve MV and the tricuspid valve TV are more prone to deformation of leaflets, which, as described above, prevents the mitral valve or tricuspid valve from closing properly and allows for regurgitation or back flow of blood from the ventricle into the atrium (e.g., a deformed mitral valve MV may allow for regurgitation or back flow from the left ventricle LV to the left atrium LA). The regurgitation or back flow of blood from the ventricle to the atrium results in valvular insufficiency. Deformations in the structure or shape of the mitral valve MV or the tricuspid valve TV are often repairable. In addition, regurgitation can occur due to the chordae tendineae 10 becoming dysfunctional (e.g., the chordae tendineae may stretch or rupture), which allows the anterior leaflet 20 and the posterior leaflet 22 to be reverted such that blood is regurgitated into the left atrium LA. The problems occurring due to dysfunctional chordae tendineae 10 can be repaired by repairing the chordae tendineae or the structure of the mitral valve (e.g., by securing the leaflets 20, 22 at the affected portion of the mitral valve).

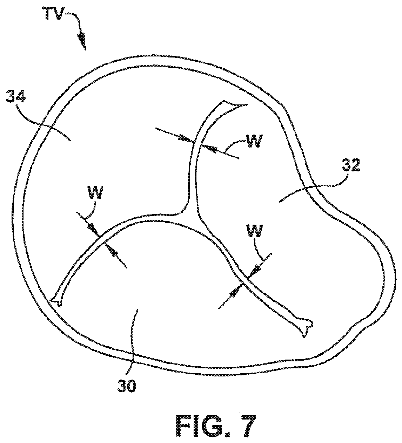

The devices and procedures disclosed herein often make reference to repairing a mitral valve for illustration. However, it should be understood that the devices and concepts provided herein can be used to repair any native valve, as well as any component of a native valve. For example, referring now to Figure 7, any of the devices and concepts provided herein can be used to repair the tricuspid valve TV. For example, any of the devices and concepts provided herein can be used between any two of the anterior leaflet 30, septal leaflet 32, and posterior leaflet 34 to prevent regurgitation of blood from the right ventricle into the right atrium. In addition, any of the devices and concepts provided herein can be used on all three of the leaflets 30, 32, 34 together to prevent or inhibit regurgitation of blood from the right ventricle to the right atrium. That is, the valve repair devices provided herein can be centrally located between the three leaflets 30, 32, 34.

An example implantable prosthetic device has one or more anchors and an optional coaption element (e.g., spacer, coaptation element, etc.). The coaption element can be configured to be positioned within the native heart valve orifice to help fill the space between native leaflets and form a more effective seal, thereby reducing or preventing regurgitation described above. The coaption element can have a structure that is impervious or resistant to blood and that allows the native leaflets to close around the coaption element during ventricular systole to block blood from flowing from the left or right ventricle back into the left or right atrium, respectively. The prosthetic device can be configured to seal against two or three native valve leaflets; that is, the device may be used in the native mitral (bicuspid) and tricuspid valves. The coaption element is sometimes referred to herein as a spacer because the coaption element can fill a space between improperly functioning native mitral or tricuspid leaflets that do not close completely.

The coaption element (e.g., spacer, coaptation element, etc.) can have various shapes. In some embodiments, the coaption element can have an elongated cylindrical shape having a round cross-sectional shape. In some embodiments, the coaption element can have an oval cross-sectional shape, a crescent cross-sectional shape, a rectangular cross-sectional shape, or various other non-cylindrical shapes. The coaption element can have an atrial portion positioned in or adjacent to the left atrium, a ventricular or lower portion positioned in or adjacent to the left ventricle, and a side surface that extends between the native mitral leaflets. In embodiments configured for use in the tricuspid valve, the atrial or upper portion is positioned in or adjacent to the right atrium, and the ventricular or lower portion is positioned in or adjacent to the right ventricle, and the side surface that extends between the native tricuspid leaflets.

The anchor can be configured to secure the device to one or both of the native leaflets such that the coaption element is positioned between the two native leaflets. In embodiments configured for use in the tricuspid valve, the anchor is configured to secure the device to one, two, or three of the tricuspid leaflets such that the coaption element is positioned between the three native leaflets. In some embodiments, the anchor can attach to the coaption element at a location adjacent the ventricular portion of the coaption element. In some embodiments, the anchor can attach to an actuation element, such as a shaft or actuation wire, to which the coaption element is also attached. In some embodiments, the anchor and the coaption element can be positioned independently with respect to each other by separately moving each of the anchor and the coaption element along the longitudinal axis of the actuation element (e.g., actuation shaft, actuation rod, actuation wire, actuation tube, etc.). In some embodiments, the anchor and the coaption element can be positioned simultaneously by moving the anchor and the coaption element together along the longitudinal axis of the actuation element, e.g., shaft or actuation wire. The anchor can be configured to be positioned behind a native leaflet when implanted such that the leaflet is grasped by the anchor.

The prosthetic device can be configured to be implanted via a delivery sheath or delivery catheter. The coaption element and the anchor can be compressible to a radially compressed state and can be self-expandable to a radially expanded state when compressive pressure is released. The device can be configured for the anchor to be expanded radially away from the still-compressed coaption element initially in order to create a gap between the coaption element and the anchor. A native leaflet can then be positioned in the gap. The coaption element can be expanded radially, closing the gap between the coaption element and the anchor and capturing the leaflet between the coaption element and the anchor. In some embodiments, the anchor and coaption element are optionally configured to self-expand. The implantation methods for various embodiments can be different and are more fully discussed below with respect to each embodiment. Additional information regarding these and other delivery methods can be found in U.S. Pat. No. 8,449,599, International Patent Application No. PCT/US2019/055320, U.S. Patent Application Publication Nos. 2014/0222136, 2014/0067052, and 2016/0331523. These methods can be performed on a living animal or on a simulation, such as on a cadaver, cadaver heart, simulator (e.g. with the body parts, heart, tissue, etc. being simulated), etc. *mutatis mutandis.*

The disclosed prosthetic devices can be configured such that the anchor is connected to a leaflet, taking advantage of the tension from native chordae tendineae to resist high systolic pressure urging the device toward the left atrium. During diastole, the devices can rely on the compressive and retention forces exerted on the leaflet that is grasped by the anchor.

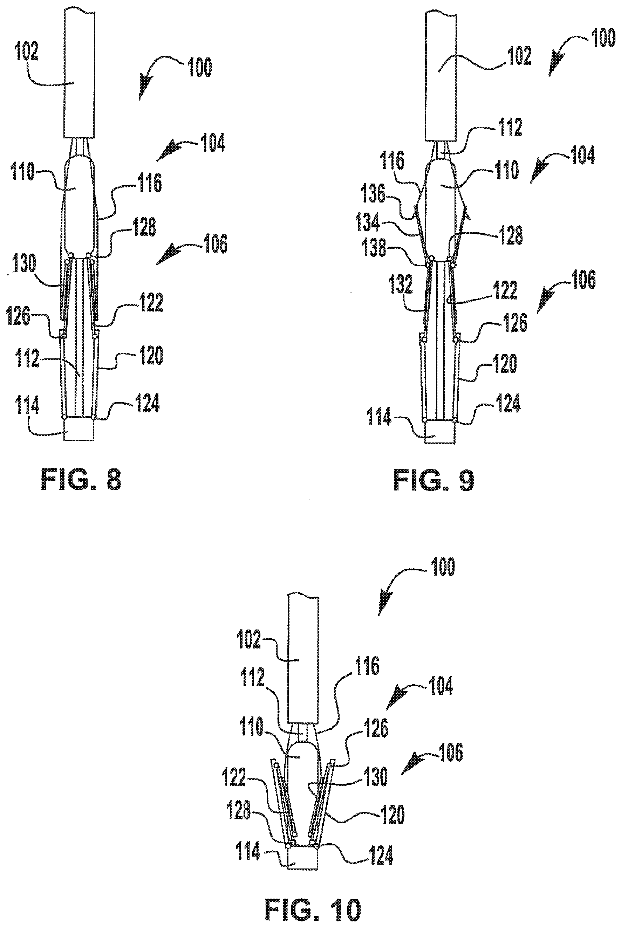

Referring now to Figures 8-14, a schematically illustrated example implantable prosthetic device 100 (e.g., a prosthetic spacer device, etc.) is shown in various stages of deployment. The device 100 can include any other features for an implantable prosthetic device discussed in the present application, and the device 100 can be positioned to engage valve tissue 20, 22 as part of any suitable valve repair system (e.g., any valve repair system disclosed in the present application). Other implants and devices of other configurations and designs can also be used with the inventions herein.

The device 100 is deployed from a delivery sheath or means for delivery 102 and includes a coapting portion or coaptation portion 104 and an anchor portion 106. In some embodiments, the coaptation portion 104 of the device 100 includes a coaption element or means for coapting 110 that is adapted to be implanted between the leaflets of a native valve (e.g., a native mitral valve, tricuspid valve, etc.) and is slidably attached to an actuation element 112 (e.g., actuation wire, actuation shaft, actuation tube, etc.). The anchor portion 106 is actuatable between open and closed conditions and can take a wide variety of forms, such as, for example, paddles, gripping elements, or the like. Actuation of the actuation element or means for actuating 112 opens and closes the anchor portion 106 of the device 100 to grasp the native valve leaflets during implantation. The actuation element or means for actuation 112 (as well as other actuation elements and means for actuation herein) can take a wide variety of different forms (e.g., as a wire, rod, shaft, tube, screw, suture, line, combination of these, etc.). As one example, the actuation element can be threaded such that rotation of the actuation element moves the anchor portion 106 relative to the coaption portion 104. Or, the actuation element can be unthreaded, such that pushing or pulling the actuation element 112 moves the anchor portion 106 relative to the coaption portion 104.

The anchor portion 106 and/or anchors of the device 100 include outer paddles 120 and inner paddles 122 that are, in some embodiments, connected between a cap 114 and the coaption element or means for coapting 110 by portions 124, 126, 128. The connection portions 124, 126, 128 can be jointed and/or flexible to move between all of the positions described below. The interconnection of the outer paddles 120, the inner paddles 122, the coaption element or means for coapting 110, and the cap 114 by the portions 124, 126, and 128 can constrain the device to the positions and movements illustrated herein.

In some implementations, the actuation element or means for actuating 112 (e.g., actuation wire, actuation shaft, etc.) extends through the delivery sheath and the coaption element or means for coapting 110 to the cap 114 at the distal connection of the anchor portion 106. Extending and retracting the actuation element or means for actuating 112 increases and decreases the spacing between the coaption element or means for coapting 110 and the cap 114, respectively. A collar or other attachment element removably attaches the coaption element or means for coapting 110 to the delivery sheath or means for delivery 102 so that the actuation element or means for actuating 112 slides through the collar or other attachment element and through the coaption element or means for coapting 110 during actuation to open and close the paddles 120, 122 of the anchor portion 106.

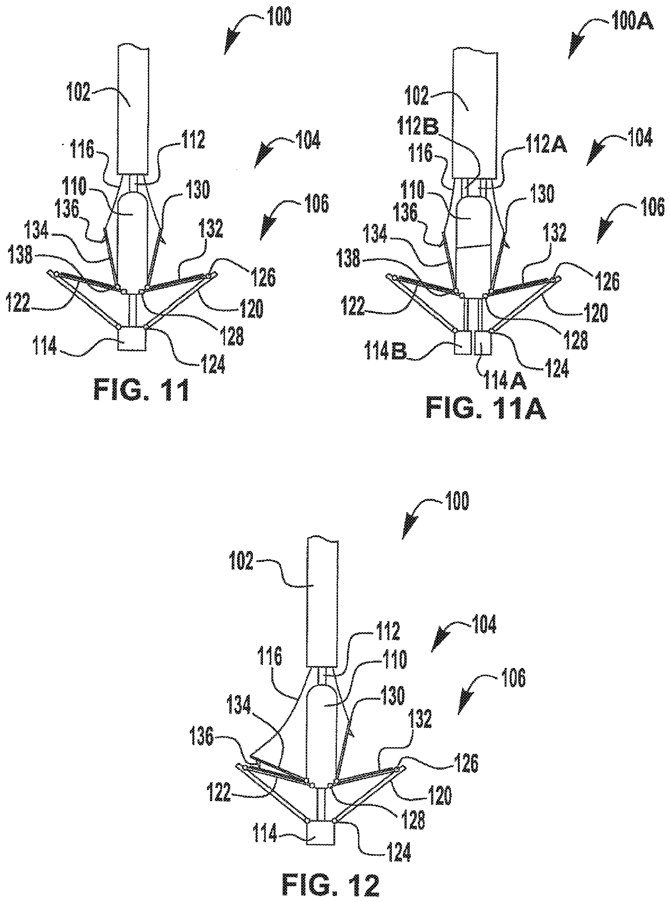

Referring now to Figure 11, the anchor portion 106 and/or anchors include attachment portions or gripping members. The illustrated gripping members comprise clasps 130 that include a base or fixed arm 132, a moveable arm 134, optional barbs or other means for securing 136, and a joint portion 138. The fixed arms 132 are attached to the inner paddles 122. In some embodiments, the fixed arms 132 are attached to the inner paddles 122 with the joint portion 138 disposed proximate the coapting or coaption element 110 or means for coapting 110. The clasps or barbed clasps have flat surfaces and do not fit in a recess of the inner paddle. Rather, the flat portions of the clasps are disposed against the surface of the inner paddle 122. The joint portion 138 provides a spring force between the fixed and moveable arms 132, 134 of the clasp 130. The joint portion 138 can be any suitable joint, such as a flexible joint, a spring joint, a pivot joint, or the like. In some embodiments, the joint portion 138 is a flexible piece of material integrally formed with the fixed and moveable arms 132, 134. The fixed arms 132 are attached to the inner paddles 122 and remain stationary relative to the inner paddles 122 when the moveable arms 134 are opened to open the clasps 130 and expose the barbs, friction-enhancing elements, or means for securing 136. In some implementations, the clasps 130 are opened by applying tension to actuation lines 116 attached to the moveable arms 134, thereby causing the moveable arms 134 to articulate, flex, or pivot on the joint portions 138. Other actuation mechanisms are also possible.

During implantation, the paddles 120, 122 can be opened and closed, for example, to grasp the native leaflets (e.g., native mitral valve leaflets, etc.) between the paddles 120, 122 and/or between the paddles 120, 122 and a coaption element or means for coapting 110. The clasps 130 can be used to grasp and/or further secure the native leaflets by engaging the leaflets with barbs, friction-enhancing elements, or means for securing 136 and pinching the leaflets between the moveable and fixed arms 134, 132. The barbs, friction-enhancing elements, or other means for securing 136 of the clasps or barbed clasps 130 increase friction with the leaflets or may partially or completely puncture the leaflets. The actuation lines 116 can be actuated separately so that each clasp 130 can be opened and closed separately. Separate operation allows one leaflet to be grasped at a time, or for the repositioning of a clasp 130 on a leaflet that was insufficiently grasped, without altering a successful grasp on the other leaflet. The clasps 130 can be opened and closed relative to the position of the inner paddle 122 (as long as the inner paddle is in an open position), thereby allowing leaflets to be grasped in a variety of positions as the particular situation requires.

The clasps 130 can be opened separately by pulling on an attached actuation line 116 that extends through the delivery sheath or means for delivery 102 to the clasp 130. The actuation line 116 can take a wide variety of forms, such as, for example, a line, a suture, a wire, a rod, a catheter, or the like. The clasps 130 can be spring loaded so that in the closed position the clasps 130 continue to provide a pinching force on the grasped native leaflet. This pinching force remains constant regardless of the position of the inner paddles 122. Barbs or means for securing 136 of the barbed clasps 130 can pierce the native leaflets to further secure the native leaflets.

Referring now to Figure 8, the device 100 is shown in an elongated or fully open condition for deployment from the delivery sheath. The device 100 is loaded in the delivery sheath in the fully open position, because the fully open position takes up the least space and allows the smallest catheter to be used (or the largest device 100 to be used for a given catheter size). In the elongated condition the cap 114 is spaced apart from the coaption element or means for coapting 110 such that the paddles 120, 122 are fully extended. In some embodiments, an angle formed between the interior of the outer and inner paddles 120, 122 is 180 degrees or approximately 180 degrees. The clasps 130 are kept in a closed condition during deployment through the delivery sheath or means for delivery 102 so that the barbs or means for securing 136 (Fig. 11) do not catch or damage the sheath or tissue in the patient's heart.

Referring now to Figure 9, the device 100 is shown in an elongated detangling condition, similar to Figure 8, but with the clasps 130 in a fully open position, ranging from about 140 degrees to about 200 degrees, from about 170 degrees to about 190 degrees, or about 180 degrees between fixed and moveable portions of the clasps 130. Fully opening the paddles 120, 122 and the clasps 130 has been found to improve ease of detanglement or detachment from anatomy of the patient, such as the chordae tendineae, during implantation of the device 100.

Referring now to Figure 10, the device 100 is shown in a shortened or fully closed condition. The compact size of the device 100 in the shortened condition allows for easier maneuvering and placement within the heart. To move the device 100 from the elongated condition to the shortened condition, the actuation element or means for actuating 112 is retracted to pull the cap 114 towards the coaption element or means for coapting 110. The connection portion(s) 126 (e.g., joint(s), flexible connection(s), etc.) between the outer paddle 120 and inner paddle 122 are constrained in movement such that compression forces acting on the outer paddle 120 from the cap 114 being retracted towards the coaption element or means for coapting 110 cause the paddles or gripping elements 120, 122 to move radially outward. During movement from the open to closed position, the outer paddles 120 maintain an acute angle with the actuation element or means for actuating 112. The outer paddles 120 can optionally be biased toward a closed position. The inner paddles 122 during the same motion move through a considerably larger angle as they are oriented away from the coaption element or means for coapting 110 in the open condition and collapse along the sides of the coaption element or means for coapting 110 in the closed condition. In some embodiments, the inner paddles 122 are thinner and/or narrower than the outer paddles 120, and the connection portions 126, 128 (e.g., joints, flexible connections, etc.) connected to the inner paddles 122 can be thinner and/or more flexible. For example, this increased flexibility can allow more movement than the connection portion 124 connecting the outer paddle 120 to the cap 114. In some embodiments, the outer paddles 120 are narrower than the inner paddles 122. The connection portions 126, 128 connected to the inner paddles 122 can be more flexible, for example, to allow more movement than the connection portion 124 connecting the outer paddle 120 to the cap 114. In some embodiments, the inner paddles 122 can be the same or substantially the same width as the outer paddles.

Referring now to Figures 11-13, the device 100 is shown in a partially open, grasp-ready condition. To transition from the fully closed to the partially open condition, the actuation element or means for actuating 112 (e.g., actuation wire, actuation shaft, etc.) is extended to push the cap 114 away from the coaption element or means for coapting 110, thereby pulling on the outer paddles 120, which in turn pull on the inner paddles 122, causing the anchors or anchor portion 106 to partially unfold. The actuation lines 116 are also retracted to open the clasps 130 so that the leaflets can be grasped. In the example illustrated by Figure 11, the pair of inner and outer paddles 122, 120 are moved in unison, rather than independently, by a single actuation element or means for actuating 112. Also, the positions of the clasps 130 are dependent on the positions of the paddles 122, 120. For example, referring to Figure 10 closing the paddles 122, 120 also closes the clasps.

Figure 11A illustrates an example embodiment where the paddles 120, 122 are independently controllable. The device 100A illustrated by Figure 11A is similar to the device illustrated by Figure 11, except the device 100A includes an actuation element that is configured as two independent actuation elements or actuation wires 112A, 112B, which are coupled to two independent caps 114A, 114B. To transition a first inner paddle and a first outer paddle from the fully closed to the partially open condition, the actuation element or means for actuating 112A is extended to push the cap 114A away from the coaption element or means for coapting 110, thereby pulling on the outer paddle 120, which in turn pulls on the inner paddle 122, causing the first anchor portion 106 to partially unfold. To transition a second inner paddle and a second outer paddle from the fully closed to the partially open condition, the actuation element or means for actuating 112B is extended to push the cap 114 away from the coaption element or means for coapting 110, thereby pulling on the outer paddle 120, which in turn pulls on the inner paddle 122, causing the second anchor portion 106 to partially unfold. The independent paddle control illustrated by Figure 11A can be implemented on any of the devices disclosed by the present application.

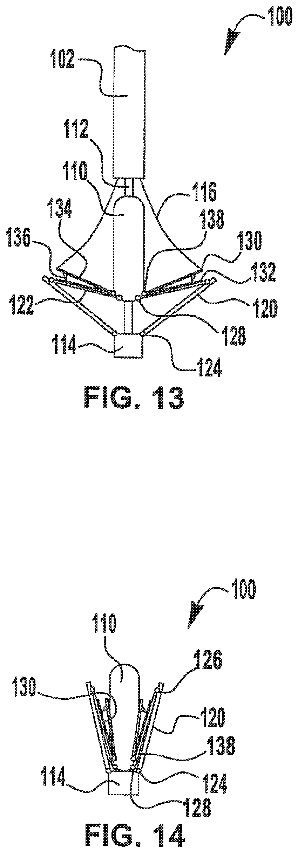

Referring now to Figure 12, one of the actuation lines 116 is extended to allow one of the clasps 130 to close. Referring now to Figure 13, the other actuation line 116 is extended to allow the other clasp 130 to close. Either or both of the actuation lines 116 can be repeatedly actuated to repeatedly open and close the clasps 130.

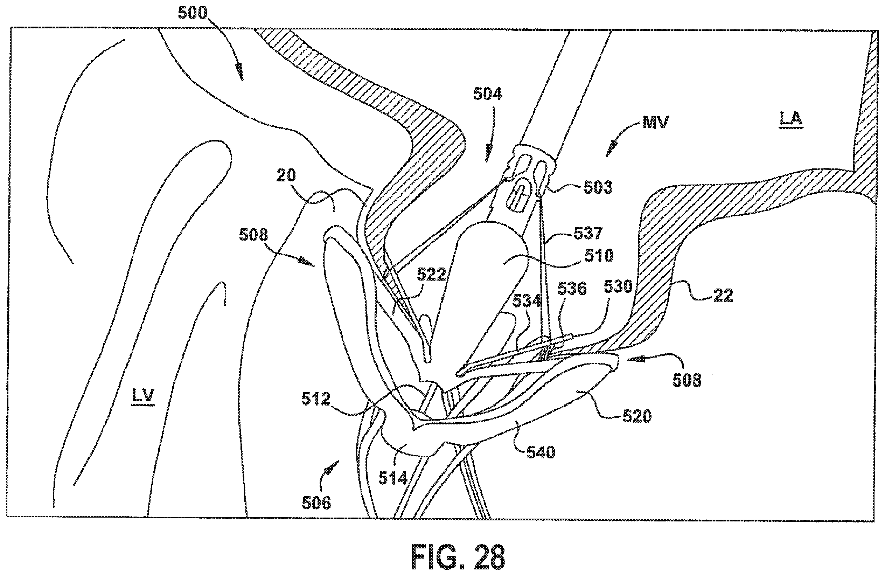

Referring now to Figure 14, the device 100 is shown in a fully closed and deployed condition. The delivery sheath or means for delivery 102 and actuation element or means for actuating 112 is/are retracted and the paddles 120, 122 and clasps 130 remain in a fully closed position. Once deployed, the device 100 can be maintained in the fully closed position with a mechanical latch or can be biased to remain closed through the use of spring materials, such as steel, other metals, plastics, composites, etc. or shape-memory alloys such as Nitinol. For example, the connection portions 124, 126, 128, the joint portion(s) 138, and/or the inner and outer paddles 122, 120 and/or an additional biasing component (see component 524 in Figure 28) can be formed of metals such as steel or shape-memory alloy, such as Nitinol-produced in a wire, sheet, tubing, or laser sintered powder-and are biased to hold the outer paddles 120 closed around the coaption element or means for coapting 110 and the clasps 130 pinched around native leaflets. Similarly, the fixed and moveable arms 132, 134 of the clasps 130 are biased to pinch the leaflets. In certain embodiments, the attachment or connection portions 124, 126, 128, joint portion(s) 138, and/or the inner and outer paddles 122, 120 and/or an additional biasing component (see component 524 in Figure 28) can be formed of any other suitably elastic material, such as a metal or polymer material, to maintain the device in the closed condition after implantation.

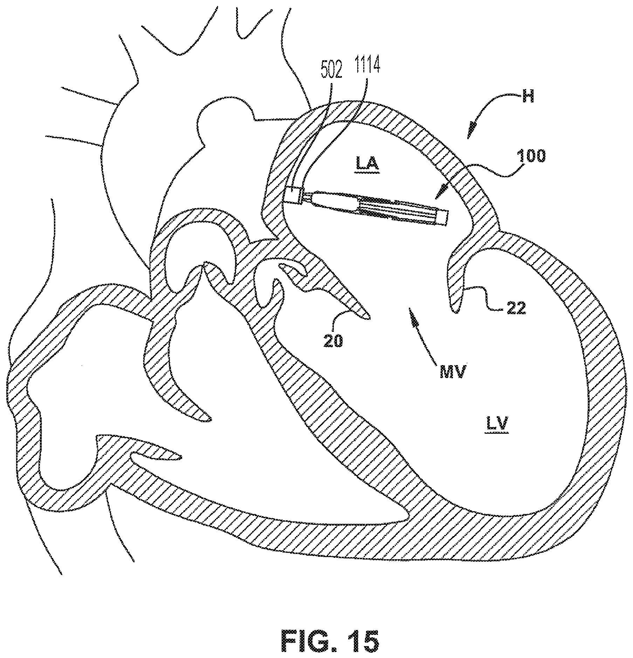

Referring now to Figures 15-20, the implantable device 100 of Figures 8-14 is shown being delivered and implanted within the native mitral valve MV of the heart H. The methods and steps shown and/or discussed can be performed on a living animal or on a simulation, such as on a cadaver, cadaver heart, simulator (e.g. with the body parts, heart, tissue, etc. being simulated), etc.

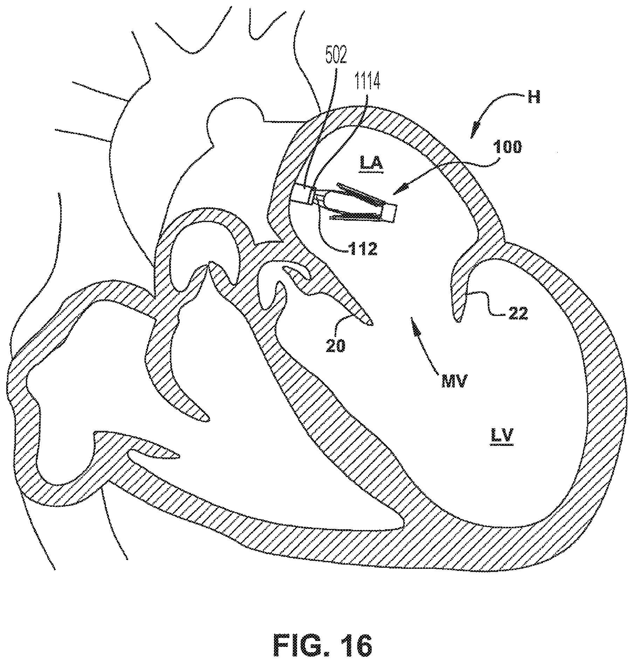

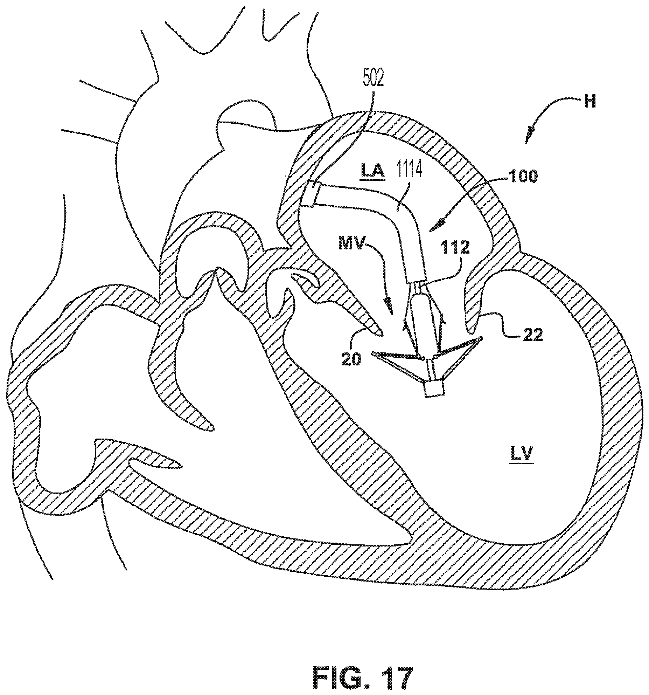

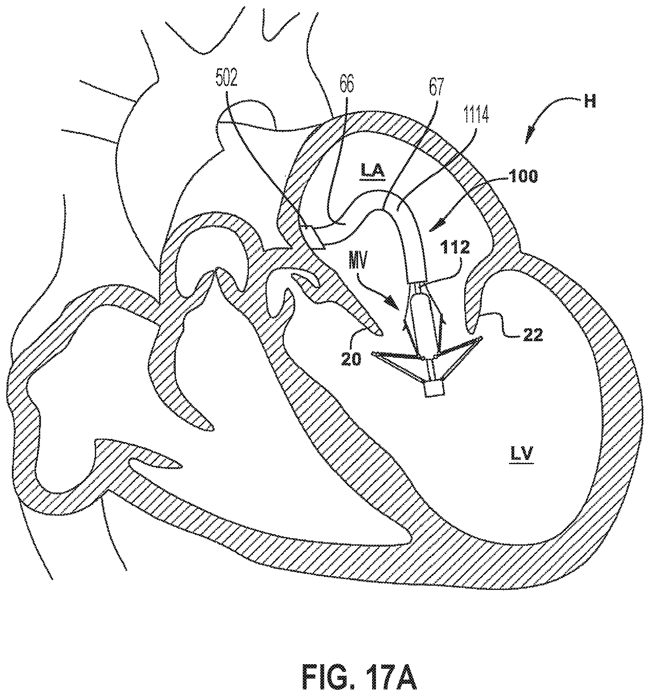

Referring now to Figure 15, the delivery sheath 102, which can be positioned inside a guide sheath 502, is inserted into the left atrium LA through the septum and the device 100 is deployed from the delivery sheath in the fully open condition. The guide sheath 502 is optional. The actuation element or means for actuating 112 is then retracted to move the device 100 into the fully closed condition shown in Figure 16. As can be seen in Figure 17, the device 100 is moved into position within the mitral valve MV into the ventricle LV and partially opened so that the leaflets 20, 22 can be grasped. An actuation line 116 can be extended to close one or more of the clasps 130, capturing one or more leaflets.

Referring now to Figure 17A, an illustration is provided of a delivery sheath 102, extending from a guide sheath 502 inserted into the left atrium LA through the septum at a location low enough in the septum such that a single bend 67 in the distal flex section 115 as illustrated in Figure 17 would position the device 100 lower than a target or optimal deployment position in the left atrium relative to the mitral valve MV. In Figure 17A, the delivery sheath 102 has a distal section bend 67 and also has a proximal section bend 66 in a proximal flex section 116 in a direction counter to that of the distal section bend 67, to pull the overall height of the distal end of the delivery sheath upward in the atrium. As will be explained in more detail below, this provides greater flexibility in positioning the delivery sheath 102 and device 1000.

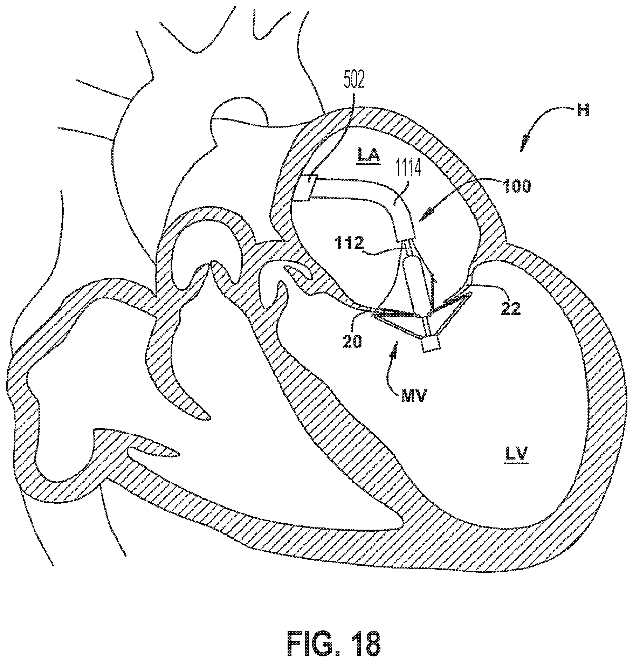

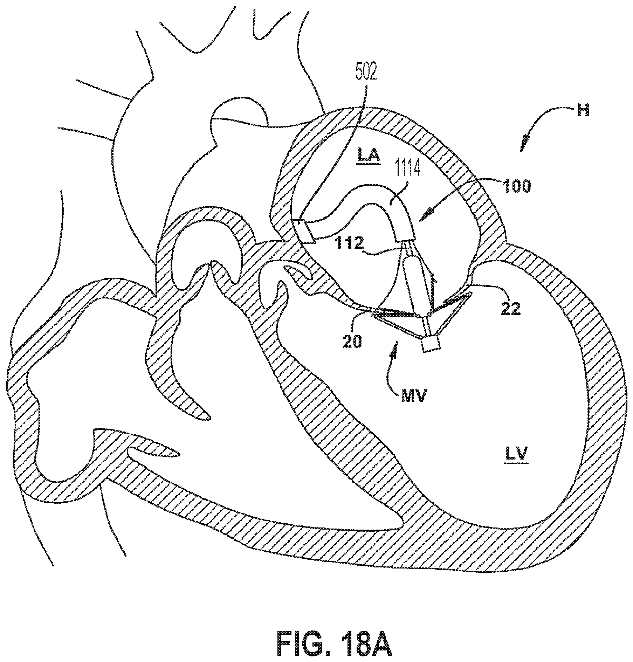

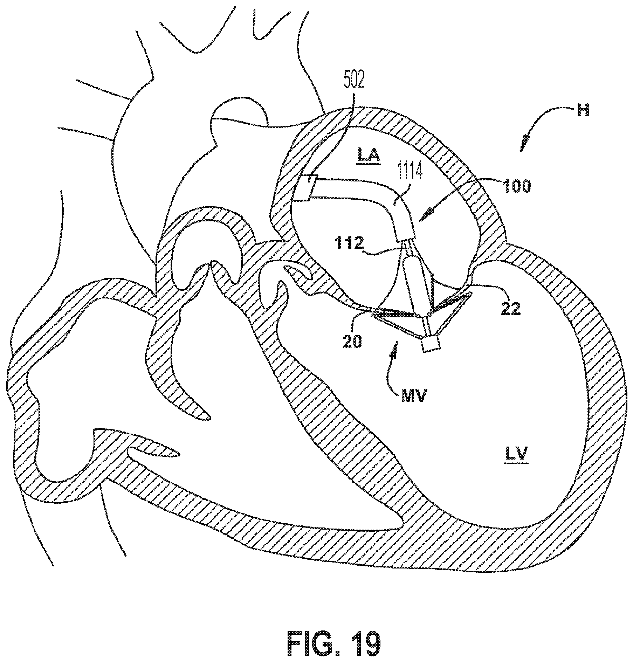

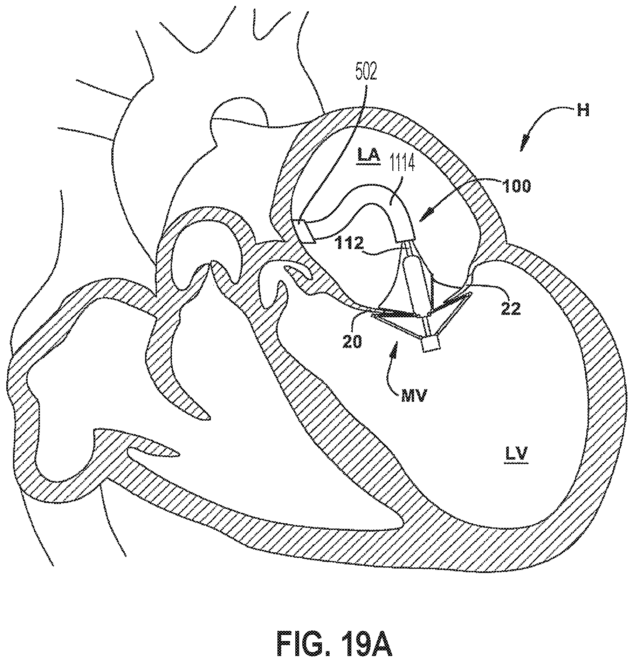

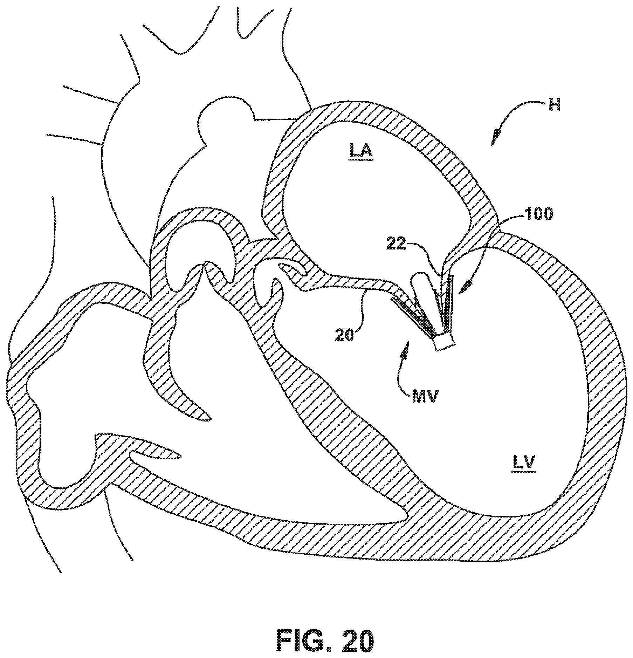

Referring now to Figures 18 and 18A, an actuation line 116 is extended to close one of the clasps 130, capturing a leaflet 20. Figures 19 and 19A show the other actuation line 112 being then extended to close the other clasp 130, capturing the remaining leaflet 22. Lastly, as can be seen in Figure 20, the delivery sheath or means for delivery 102 and actuation wire or means for actuating 112 are then retracted and the device 100 is fully closed and deployed in the native mitral valve MV.

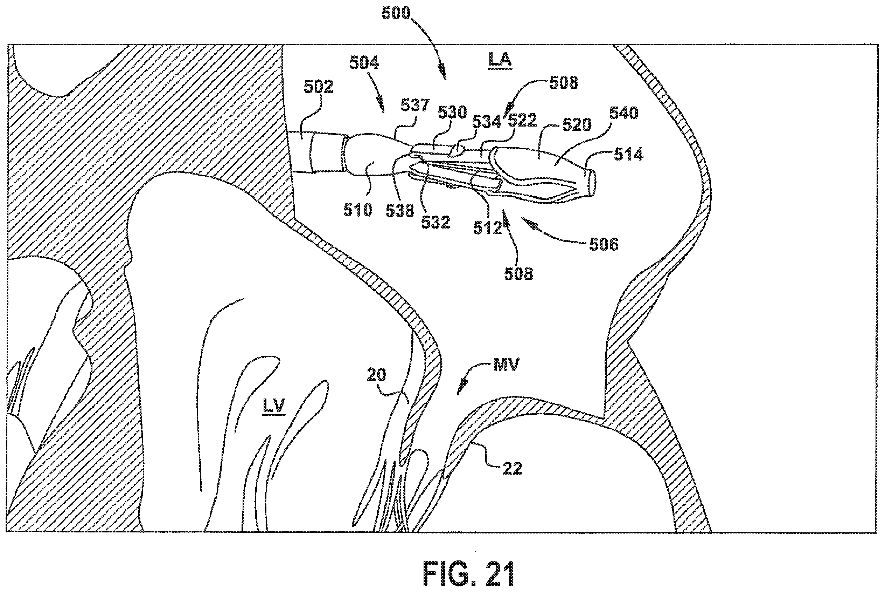

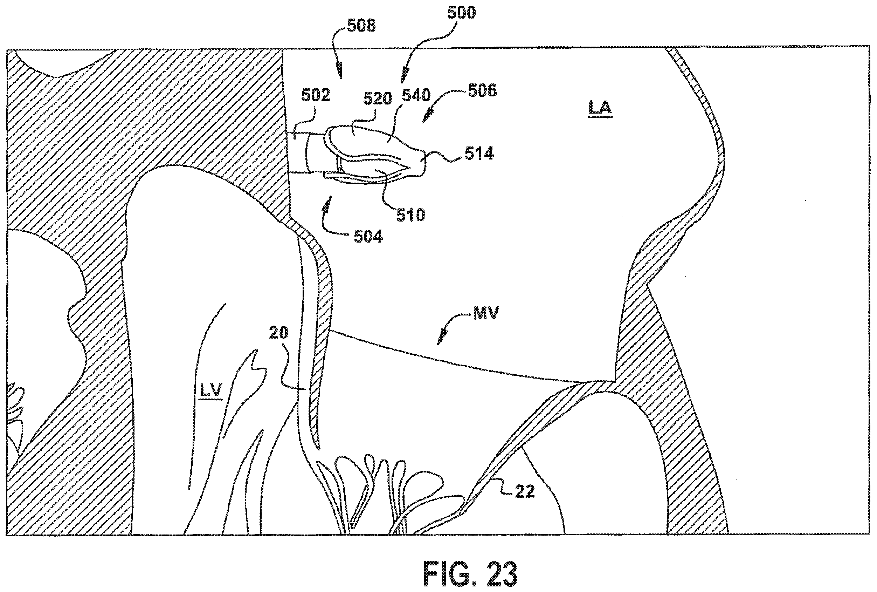

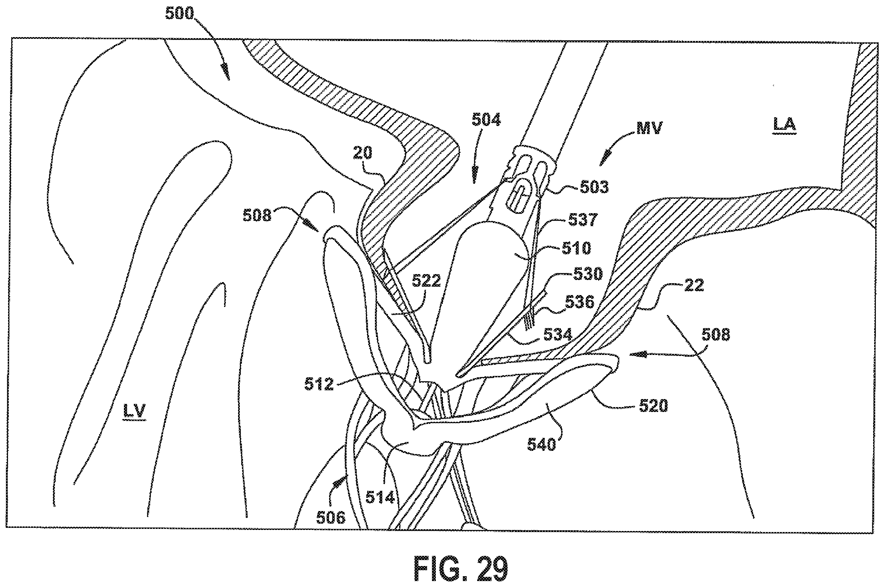

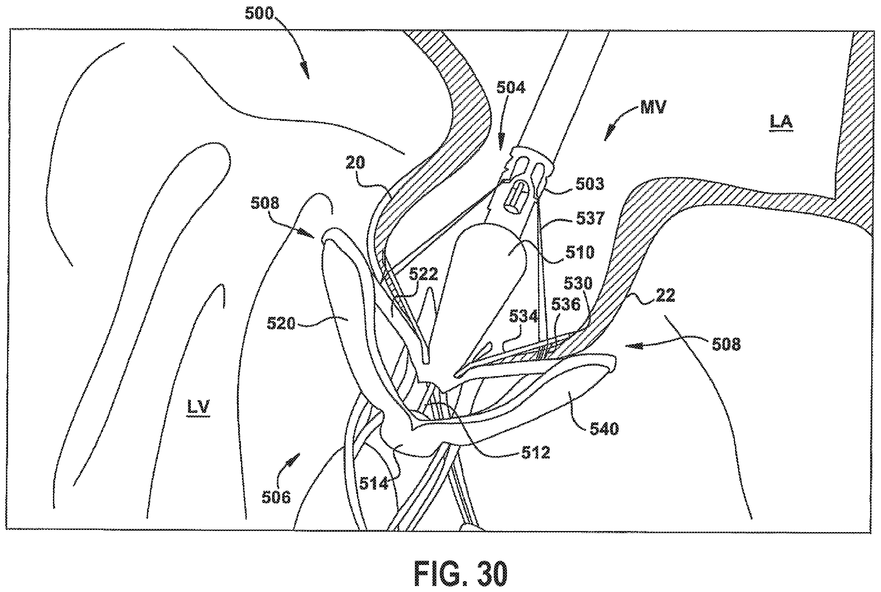

Referring now to Figures 21-32A, an example embodiment of an implantable device 500 is shown being delivered and implanted within the native mitral valve MV of the heart H. The device 500 can have a covering 540 over the coaption element 510, clasps 530, inner paddles 522 and/or the outer paddles 520. The device 500 is deployed from a guide sheath 502 and includes a coaption portion 504 and an anchor portion 506 including a plurality of anchors 508 (i.e., two in the illustrated embodiment). The coaption portion 504 of the device includes a coaption element 510 for implantation between the leaflets 20, 22 of the native mitral valve MV that is slidably attached to an actuation wire or shaft 512. Actuation of the actuation wire or shaft 512 opens and closes the anchors 508 of the device 500 to grasp the mitral valve leaflets 20, 22 during implantation.

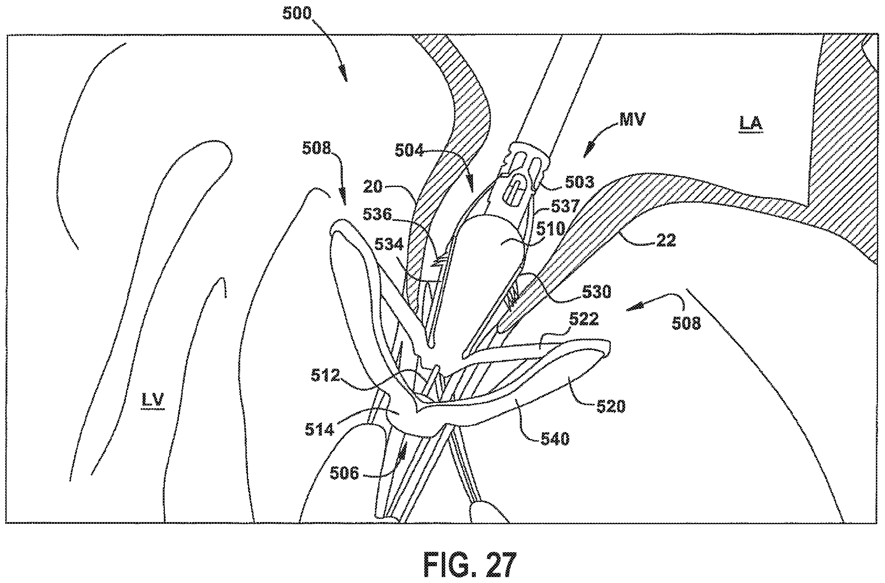

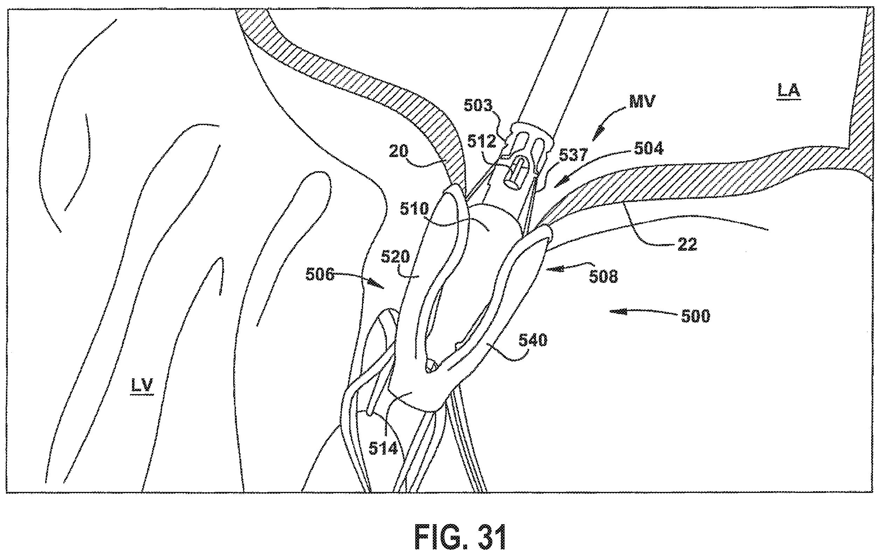

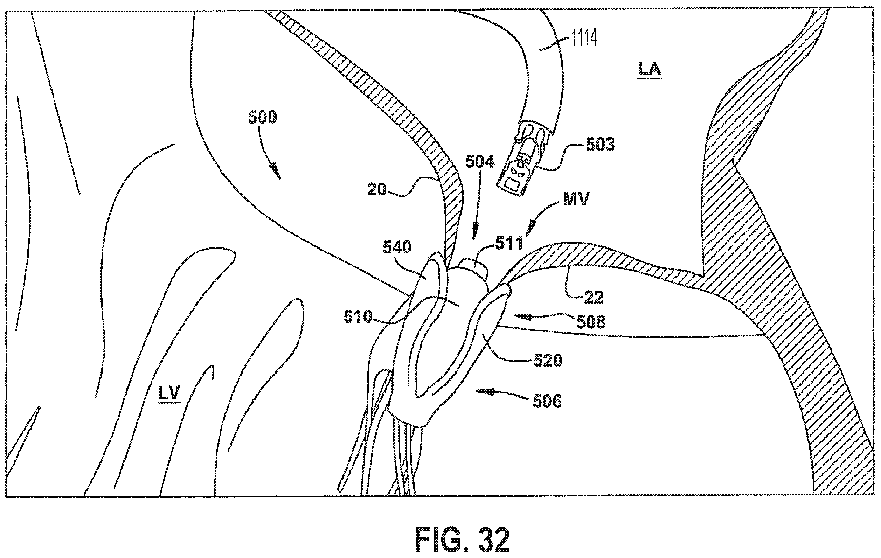

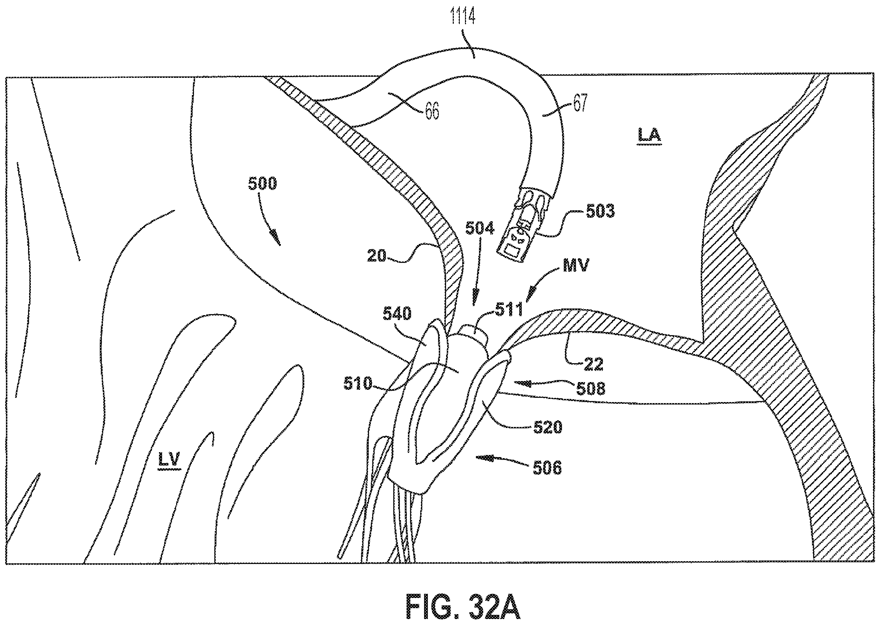

The anchors 508 of the device 500 include outer paddles 520 and inner paddles 522 that are flexibly connected to the cap 514 and the coaption element 510. The actuation element 512 extends through a capture mechanism 503 (see Figure 27), guide sheath 502, and the coaption element 510 to the cap 514 connected to the anchor portion 506. Extending and retracting the actuation element 512 increases and decreases the spacing between the coaption element 510 and the cap 514, respectively. In the example illustrated by Figures 21-37A, the pair of inner and outer paddles 522, 520 are moved in unison, rather than independently, by a single actuation element 512. Also, the positions of the clasps 530 are dependent on the positions of the paddles 522, 520. For example, referring to Figure 31 closing the paddles 522, 520 also closes the clasps. In one example embodiment, the device 500 can be made to have the paddles 520, 522 be independently controllable in the same manner as the Figure 11A embodiment. Figure 32 illustrates the device with the paddles closed on the leaflets of the mitral valve and the delivery sheath 102 is separated from the device 500. Figure 32A illustrates the same deployed position of the device 500 with a delivery sheath having a distal section bend 67 and a proximal section bend 66.

Fingers of the capture mechanism 503 removably attach the collar 511 to the guide sheath 502. The collar 511 and the coaption element 510 slide along the actuation element 512 during actuation to open and close the anchors 508 of the anchor portion 506. In some embodiments, the capture mechanism 503 is held closed around the collar 511 by the actuation element 512, such that removal of the actuation element 512 allows the fingers of the capture mechanism 503 to open, releasing the collar 511, and thus the coaption element 510.

The coaption element 510 and paddles 520, 522 can be formed from a flexible material that may be a metal fabric, such as a mesh, woven, braided, or formed in any other suitable way or a laser cut or otherwise cut flexible material. The flexible material may be cloth, shape-memory alloy wire-such as Nitinol-to provide shape-setting capability, or any other flexible material suitable for implantation in the human body.

The barbed clasps 530 include a base or fixed arm 532, a moveable arm 534, barbs 536 (*see* Figure 27), and a joint portion 538. The fixed arms 532 are attached to the inner paddles 522, with the joint portions 538 disposed proximate the coaption element 510. Sutures (not shown) attach the fixed arms 532 to the inner paddles 522. The fixed arms 532 may be attached to the inner paddles 522 with any suitable means, such as screws or other fasteners, crimped sleeves, mechanical latches or snaps, welding, adhesive, or the like. The fixed arms 532 remain stationary or substantially stationary when the moveable arms 534 are opened to open the barbed clasps 530 and expose the barbs 536. The barbed clasps 530 are opened by applying tension to actuation lines 537 attached to the moveable arms 534, thereby causing the moveable arms 534 to articulate, pivot, or flex on the joint portions 538.

During implantation, the anchors 508 are opened and closed to grasp the native mitral valve leaflets between the paddles 520, 522 and the coaption element 510. The outer paddles 520 have a wide curved shape that fits around the curved shape of the coaption element 510 to more securely grip the leaflets 20, 22. The curved shape and rounded edges of the outer paddle 520 also prohibits tearing of the leaflet tissue. The barbed clasps 530 further secure the native leaflets by engaging the leaflets with barbs 536 and pinching the leaflets between the moveable and fixed arms 534, 532. The barbs 536 of the barbed clasps 530 increase friction with the leaflets or may partially or completely puncture the leaflets. The actuation lines can be actuated separately so that each barbed clasp 530 can be opened and closed separately. Separate operation allows one leaflet to be grasped at a time, or for the repositioning of a clasp 530 on a leaflet that was insufficiently grasped, without altering a successful grasp on the other leaflet. The barbed clasps 530 can be fully opened and closed when the inner paddle 522 is not closed, thereby allowing leaflets to be grasped in a variety of positions as the particular situation requires.

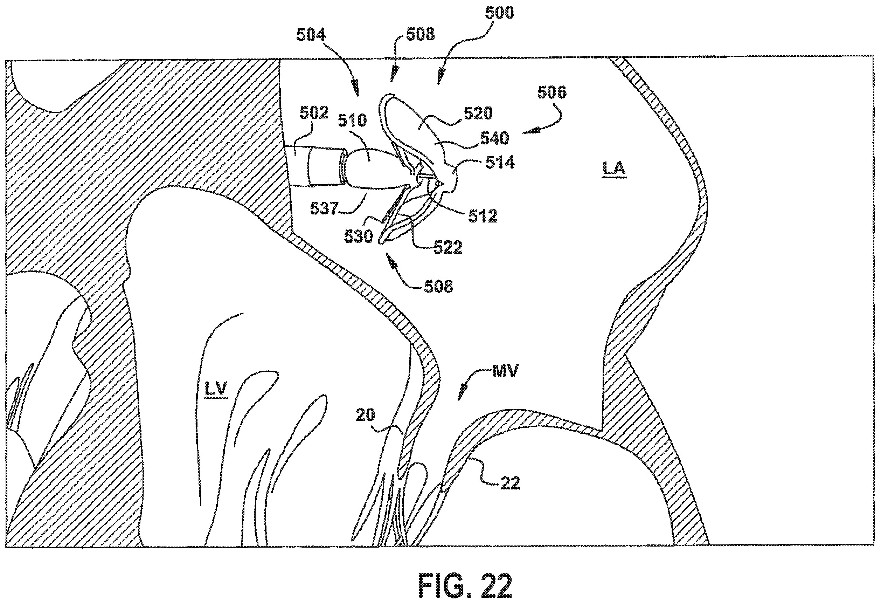

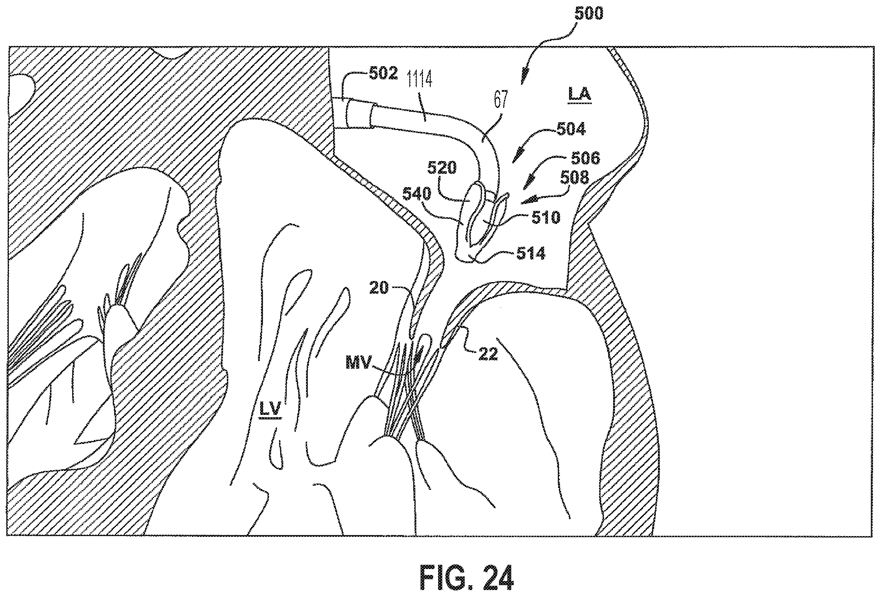

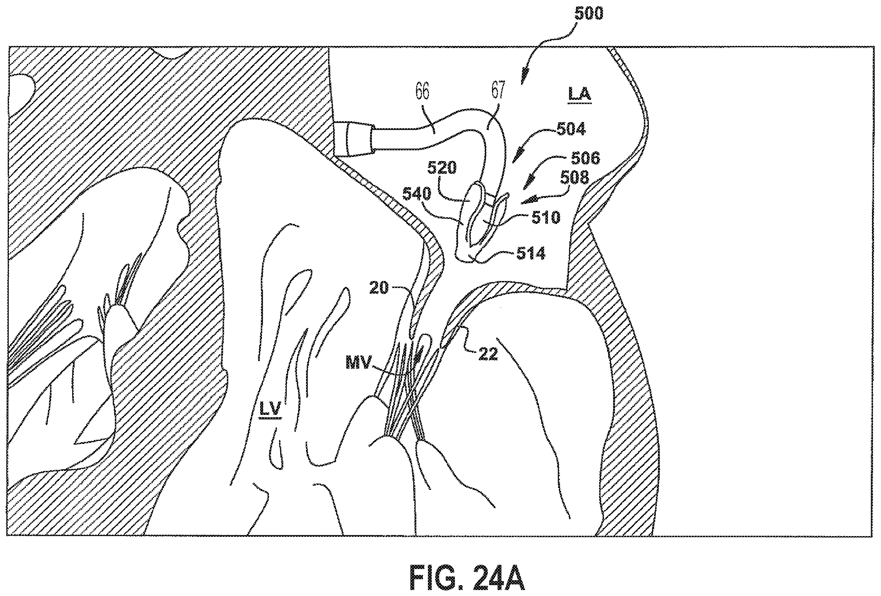

The device 500 is loaded in the delivery sheath in the fully open position, because the fully open position takes up the least space and allows the smallest catheter to be used (or the largest device 500 to be used for a given catheter size). Referring now to Figure 21, the delivery sheath is inserted into the left atrium LA through the septum and the device 500 is deployed from the guide sheath 502 in the fully open condition. The actuation element 512 is then retracted to move the device 500 into the fully closed condition shown in Figures 22-23 and then maneuvered towards the mitral valve MV as shown in Figure 24.

Figure 24A illustrates the device 500 in the same position and fully closed condition as illustrated in Figure 24, positioned as such by a guide sheath 502 having a distal flex section 115 with a distal bend 67 and a proximal flex section 116. As explained above with respect to Figure 17A, the proximal flex section 116 pulls the overall height of the distal end 118 of the delivery sheath up in the left atrium so that it can be properly positioned for delivery of the device 500 into the native valve in the event the delivery sheath passes through the septum at a position low than a target position for a single distal section bend 67 to position the device 500 at an optimal position.

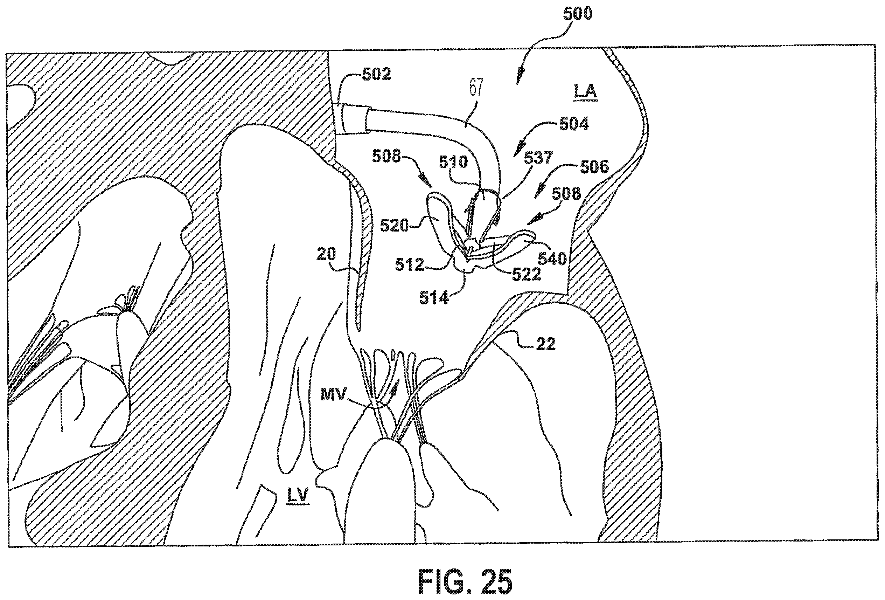

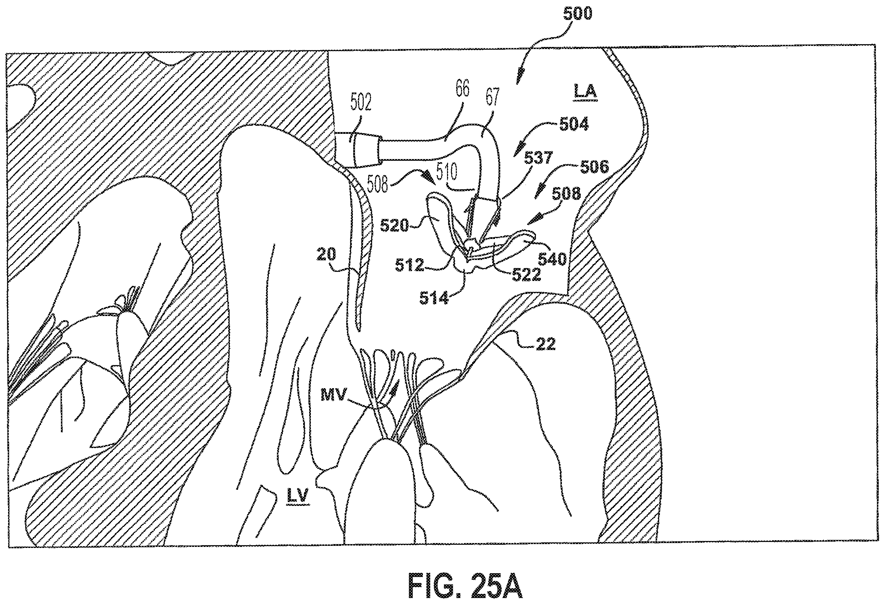

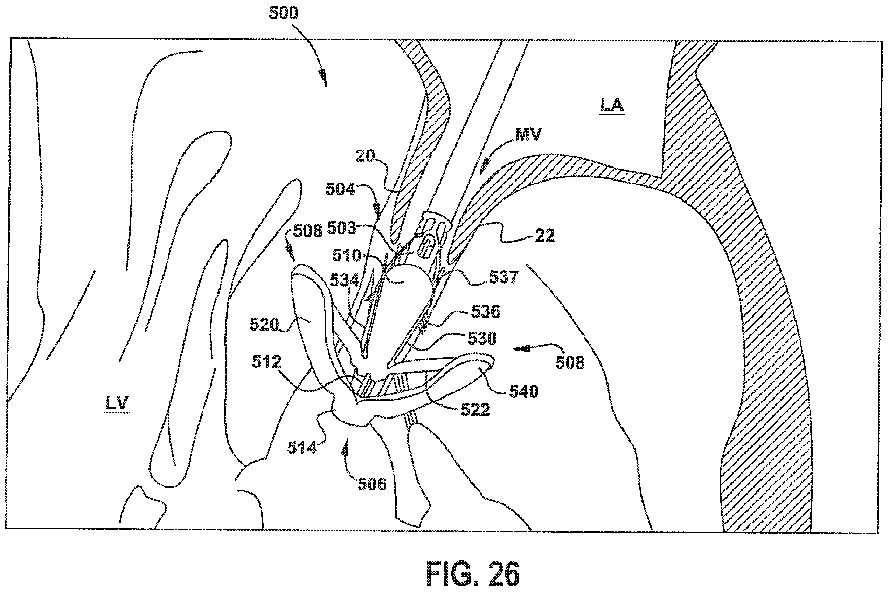

Referring now to Figure 25, when the device 500 is aligned with the native valve, the actuation element 512 can be extended to open the paddles 520, 522 into the partially opened position and the actuation lines 537 are retracted to open the barbed clasps 530 to prepare for leaflet grasp. Figure 25A illustrates the same position and condition of the device 500, with a delivery sheath 102 having both a distal section bend 67 and a proximal section bend 66. Figure 25A also illustrates an optional guide sheath 502. Next, as shown in Figures 26-27, the partially open device 500 is inserted through the native valve until leaflets 20, 22 are properly positioned in between the inner paddles 522 and the coaption element 510 and inside the open barbed clasps 530.

Figure 28 shows the device 500 with both clasps 530 closed, though the barbs 536 of one clasp 530 missed one of the leaflets 22. As can be seen in Figures 28-30, the out of position clasp 530 is opened and closed again to properly grasp the missed leaflet 22. When both leaflets 20, 22 are grasped properly, the actuation element 512 is retracted to move the device 500 into the fully closed position shown in Figure 31. With the device 500 fully implanted in the native valve, the actuation element 512 is withdrawn to release the capture mechanism 503 from the proximal collar 511. Once deployed, the device 500 can be maintained in the fully closed position with a mechanical means such as a latch or can be biased to remain closed through the use of spring material, such as steel, and/or shape-memory alloys such as Nitinol. For example, the paddles 520, 522 can be formed of steel or Nitinol shape-memory alloy-produced in a wire, sheet, tubing, or laser sintered powder-and are biased to hold the outer paddles 520 closed around the inner paddles 522, coaption element 510, and the barbed clasps 530 pinched around native leaflets 20, 22.

The device 500 can have a wide variety of different shapes and sizes. Referring to Figure 21, in an example embodiment, the coaption element 510 functions as a gap filler in the valve regurgitant orifice, such as the gap 26 in the mitral valve MV illustrated by Figure 6. Since the coaption element 510 is deployed between two opposing valve leaflets 20, 22, the leaflets will not coapt against each other in the area of the coaption element 510, but coapt against the coaption element 510 instead. This reduces the distance the leaflets 20, 22 need to be approximated. A reduction in leaflet approximation distance can result in several advantages. For example, the coaption element and resulting reduced approximation can facilitate repair of severe mitral valve anatomies, such as large gaps in functional valve disease (See for example, Figure 6). Since the coaption element 510 reduces the distance the native valves have to be approximated, the stress in the native valves can be reduced or minimized. Shorter approximation distance of the valve leaflets 20, 22 can require less approximation forces which can result in less tension of the leaflets and less diameter reduction of the valve annulus. The smaller reduction of the valve annulus (or no reduction of the valve annulus) can result in less reduction in valve orifice area as compared to a device without a spacer. As a result, the coaption element 510 can reduce the transvalvular gradients.

The various delivery catheters, delivery sheaths, means for delivery herein can be used with the example implants and devices described herein and/or with other types of implants/devices (e.g., prosthetic heart valves, docking devices, stents, repair devices, replacement devices, etc.).

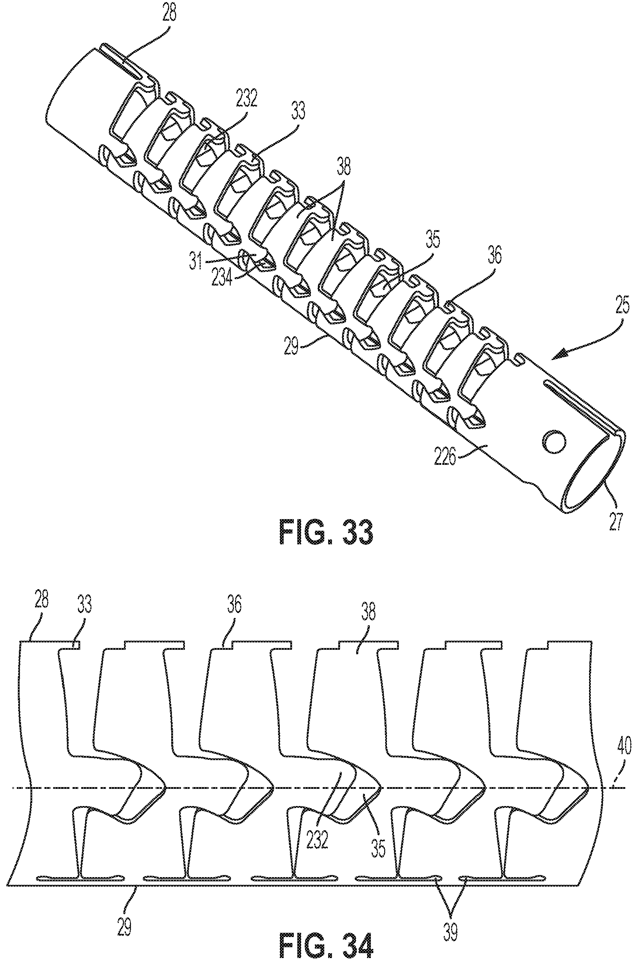

Figure 33 shows a perspective view of an example flex segment 25 that can be used in a delivery catheter or delivery sheath 24. The flex segment 25 includes two opposite ends, two opposite sides 226 and 27, a top 28, and a bottom 29 extending between the two ends. These have been labelled "top" and "bottom" for ease of description and understanding and are not intended to limit the orientation of the distal flex segment 25. The orientation of the flex segment 25 can be arranged so that the top 28 is aligned with the desired inner curve of a catheter when it is in a flexed configuration. The flex segment 25 of Figure 33 forms a generally cylindrical hollow tube that can include a plurality of links 38. Each link 38 has the shape of a cylindrical segment and each link 38 is aligned with and connected to adjacent links 38 to form the cylindrical tube shape of the flex segment 25. While the distal flex segment 25 is cylindrical in this embodiment, other shapes, such as ovular distal flex segments, are also possible. Each link 38 of the flex segment 25 can have a greater width at the bottom 29 than at the top 28, giving the links 38 the general shape of an acute trapezoid when viewed from the side, as best seen in Figure 34. The bottom of each link 38 can have slits 39 to allow for more flexing of the links 38 relative to one another.

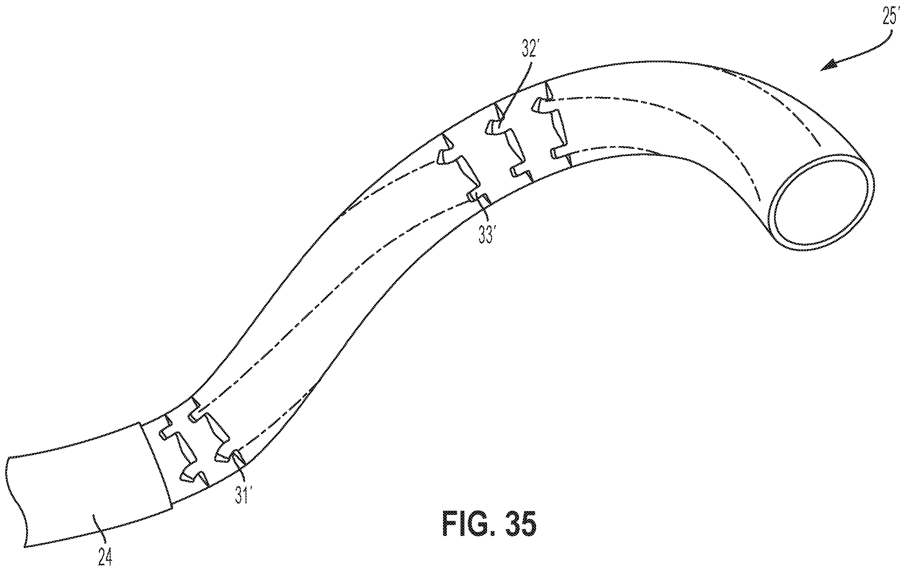

The flex segment 25 can include a double guiding pattern forming a hybrid bending section that incorporates both side teeth 31, 232 and top teeth 33. To this effect, each link 38 can include two side teeth 31, 232 on opposite sides of the link 38 and a top tooth 33. With respect to the flex segment 25, the two rows of side teeth 31, 232 of the links 38 can run the length of the sides 226, 27 of the flex segment 25, respectively, and the top teeth 33 can run the length of the flex segment 25 on the top 28, as best seen in Figure 33. While the rows of side teeth 31, 232 and top teeth 33 are shown to run straight along the length of the distal section 25 in this illustrated embodiment, other embodiments can have different configurations. For example, in some embodiments, the rows of side teeth 31, 232 and top teeth 33 can spiral around the tube of the flex segment 25, to effect specific bending shapes of the flex segment 25 when the flex segment 25 is actuated. In some embodiments, the side teeth 31, 232 can be mirror images of each other to allow analogous bending on opposite sides 226, 27 of the flex segment 25. In some embodiments, the side teeth 31, 232 can have different shapes and/or sizes in comparison to each other. The teeth 31, 232, 33 can take any other suitable shape and/or size that allows the distal region to move to a flexed configuration while delivering an anchoring device. While the teeth 31, 232, 33 are all right-facing teeth in the illustrated embodiment (e.g., directed to the right in the view shown in Figure 34), in other embodiments, the teeth can be left-facing teeth (see, for example, Figure 35) or the top and side teeth can face different directions, for example.

Adjacent to each side tooth 31, 232 and each top tooth 33 is a corresponding side slot or groove 234, 35 and top slot or groove 36, respectively, on an adjacent link 38. Each slot 234, 35, 36 can have a shape complementary to the side tooth 31, 232 or top tooth 33 to which it is adjacent. When the flex segment 25 is in a straightened configuration, the side teeth 31, 232 are partially inserted into the side slots 234, 35 and the top teeth 33 are separated from their adjacent top slots 36 by a gap. Having the side teeth 31, 232 partially within the side slots 234, 35 in this straightened configuration provides additional torque resistance to the flex segment 25 when the flex segment 25 of the delivery catheter 1114 is not fully flexed. However, in some embodiments, the side teeth 31, 232 may not be positioned partially within the side slots 234, 35 when the flex segment 25 is in the straightened configuration.

When the flex segment 25 is bent, each side tooth 31, 232 moves further into its corresponding side slot 234, 35 and each top tooth 33 moves closer to and then into its corresponding top slot 36. The addition of the top teeth 33 and top slots 36 provides enhanced torqueability and torque resistance to the distal section 25 when it is in the fully flexed configuration. Further, having both side teeth 31, 232 and top teeth 33 provides additional guiding control and structural support when adjusting the flex segment 25 from its straightened to its flexed configuration.

Figure 34 is a detailed cross-sectional view of several links 38 of the flex segment 25 of Figure 33. While Figure 34 is described with respect to the side teeth 232, this description equally applies to side teeth 31 on the opposite side of the flex segment 25. Side teeth 232 are shown as being positioned along a tooth line 40 that is low relative to the top 28 of the flex segment 25. This positioning causes the side teeth 232 to have a smaller displacement, i.e., the distance the side teeth 232 move into the adjacent slot 35 is much shorter or less than if the side teeth 232 were positioned closer to the top 28 of the flex segment 25. For example, in the illustrated embodiment, the distance that the side teeth 31, 232 move during flexing is smaller compared to the distance that the top teeth 33 move. In other words, the top teeth 33 move a greater distance relative to adjacent links 38 when the flex segment 25 is adjusted to a fully bent configuration, as compared to the side teeth 31, 232. This arrangement allows the use of shorter side teeth 31, 232 (e.g., to have side teeth with shorter longitudinal lengths), which can in turn be incorporated into shorter bending sections in the flex segment 25.

Further, the low tooth line also provides more space for wider tooth slots 234, 35 to accommodate, for example, even larger side teeth since the tooth slots 234, 35 are located at the wider lower portions of the links 38. Having more space to house larger and/or more appropriate or robust tooth slots 234, 35 for the side teeth 31, 232 can enhance guiding of the teeth 31, 232 into the slots 234, 35, for example, during bending. The low tooth line also allows for the above discussed robust tooth design that can still provide structural support while bending the links away from each other, i.e., in the opposite direction of the bending configuration. Therefore, when bending the links away from each other, the side teeth can still maintain their interface with the adjacent side slots, and this maintained tooth-slot interface can provide for more structural support and torqueability.

Figure 35 is a perspective view of a flex segment 25' in a bent configuration according to a modification of the first embodiment. The flex segment 25' in Figure 35 is similar to the flex segment 25 of Figure 33, except that in Figure 35, the rows of top teeth 33' and the rows of side teeth 31', 232' are shifted laterally around the tube-shaped flex segment 25' instead of continuing in a straight line down the length of the flex segment. This positioning of the rows of teeth 31', 232', 33' along, for example, a spiral line allows the flex segment 25' to bend in three dimensions, as opposed to a single plane as would occur in Figure 33. As seen in Figure 35, the example flex segment 25' has a three-dimensional curved shape. Various embodiments of distal sections can be laser cut (e.g., into a sheet or tube) so that the top and side teeth follow a pattern that will form a desired shape during bending. For example, patterns can be cut that create a distal section having a bent shape that, when used in surgery, allows the flex segment to be positioned at the mitral or other valve, such that an anchoring device can be advanced from the distal section and accurately positioned at the valve.

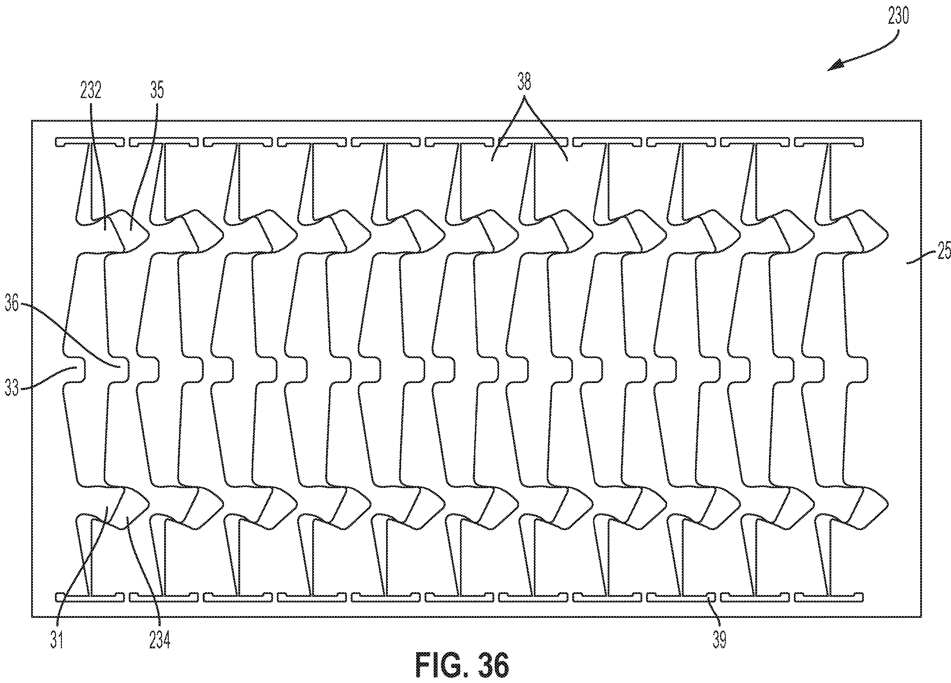

Flex segments 25, 25' can be manufactured by cutting, for example, by laser cutting a flat metal strip or sheet with the desired pattern and then rolling the patterned metal strip or sheet into a hypotube. Optionally, the desired pattern (e.g., the same or similar patterns to those shown in various figures herein) could be cut directly into a tube (e.g., a hypotube) without using a sheet or having to roll the material. As an example, Figure 36 shows a flat view of an example laser cut file or sheet 230 that can be used for the flex segment 25 of Figure 33. This laser cut sheet 230 includes both the top teeth 33 and their associated slots 36 and the side teeth 31, 232 and their associated slots 234, 35 arranged in straight rows along the length of the flex segment 25. However, as noted above, this laser cut file 230 can be modified to have the teeth 31, 232, 33 and their associated slots 234, 35, 36 arranged in other different paths or configurations, for example, in rows of spiral lines, in order to create a curved or spiral bent flex segment 25' similar to the one shown in Figure 35. In some embodiments, various patterns can be cut that provide flex segments that can bend in other shapes or configurations that help accurately navigate and deploy an anchoring device into position at the implant site during surgery.

Many types of sheets capable of being folded into tubing can be used for making the cut flex segments. Further many types of tubes can be cut into the desired pattern(s). For example, Nitinol and stainless steel can be used, as well as various other suitable metals known in the art, as materials for the sheets or tubes.

While the above embodiments include both top and side teeth, such that each link 38 has three teeth total, other embodiments may only include one of either the top or side teeth, or no teeth at all.

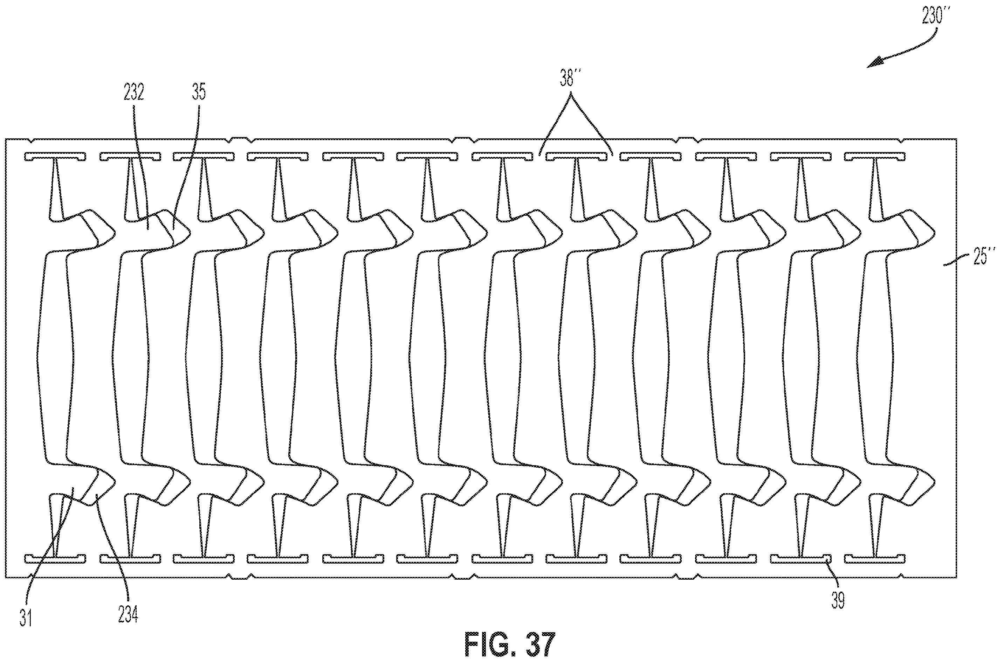

Figure 37 is a flat view of another example laser cut sheet 230" for a flex segment 25" of a delivery catheter. The flex segment 25" of Figure 37 is similar to the flex segment 25 of Figure 36, however, links 38" of the flex segment 25" only include the two side teeth 31, 232 and their associated slots 234, 35, and do not include any top teeth or corresponding slots.

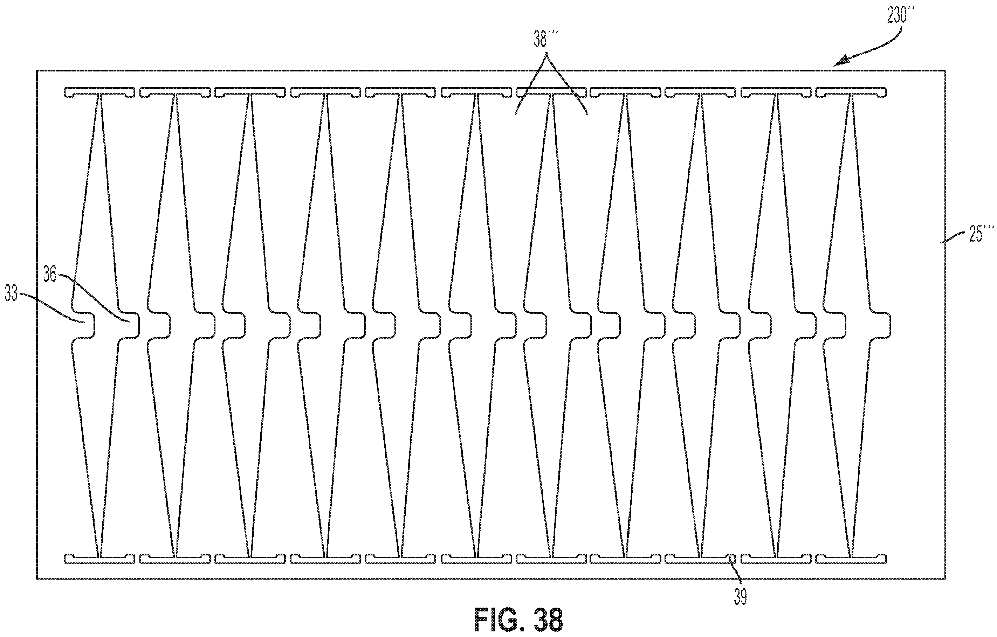

Figure 38 is a flat view of another example laser cut sheet 230"' for a flex segment 25‴ of a delivery catheter. The flex segment 25‴ of Figure 38 is also similar to the flex segment 25 of Figure 36, however, each of the links 38"' of the flex segment 25‴ only includes the single top tooth 33 and its associated slot 36 and do not include any side teeth or corresponding slots.

In some embodiments, more or less than three teeth in any combination can be included on each link. Meanwhile, while Figures 36 and 37 are shown with teeth arranged in straight rows along the length of the flex segments 25", 25‴, respectively, the laser cut sheets 230", 230"' can also be modified to include various tooth patterns and arrangements in order to have flex segments capable of bending in specific desired shapes, similarly as discussed above.

Various sheath and catheter designs can be used to effectively deploy the anchoring device at the implant site. The sheath and catheter designs, as well as the deployment tools such as actuation pull wire systems, and methods described in U.S. Patent Application Serial No. 15/984661 and U.S. Patent Application Serial No. 62/770071.

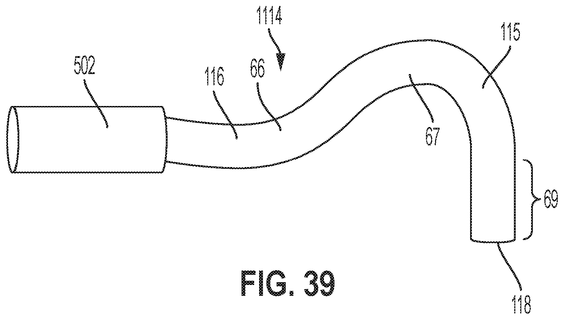

Referring to Figure 39, the delivery catheter can have two flexing segments; a distal flex section 115 and a proximal flex section 116. The location of the distal flex section 115 in the delivery catheter is where the distal section bend 67 occurs. The location of the proximal flex section 116 in the delivery catheter is where the proximal section bend 66 will occur. The distal section bend 67 can be achieved by one of the embodiments described herein, for example, with the laser cut sheets as described above and illustrated in Figures 33-37. The slots 36 in the sheet, when rolled into a tubular shape, are aligned along each of the distal flex section 115 and the proximal flex section 116 and will curve in towards itself, to reduce the space between the slots in each of the sections.

In the embodiments having two flex segments, there can be a second sheet with slots and teeth cut in it, and the second sheet is rolled into a tube. The second sheet can be positioned and secured to the first sheet such that a distal end of the first sheet is secured to a proximal end of the first sheet. The slots are aligned so that when the connected tubes are in a straight configuration, the center of each slot in the second tube is offset from the center of each slot in the first tube by 180 degrees. In some embodiments, the center of the slots can be offset by 90 degrees, or 45 degrees. In some embodiments, instead of having two sheets connected together, the tube can be made from one longer sheet, but laser cut to have the slots offset from each other.

In one example embodiment, the distal section bend, provided by the first tube, is used to point the distal end of the delivery catheter toward the mitral valve. The proximal section bend, provided by the second tube, is used in scenarios of delivering a valve implant where the delivery catheter has punctured the septal wall at a location that is lower than a target location for the implant to be positioned above the native valve using only a distal section flex. The proximal flex section, for example, can pull the distal section of the delivery catheter upwards (with the top of the left atrium defined as "up").

In some embodiments, the catheter itself can also be positioned to pass below a plane of the annulus of a native valve or to extend into a ventricle. The catheter can be positioned in any suitable manner that allows the device to be deployed at an implant site, such as the mitral valve.

Referring again to Figure 39, the delivery catheter 1114 is in a curved configuration. The illustrated configuration can be used for implanting an anchoring device at a native valve (e.g., at a native mitral valve using, for example, a transseptal technique). In this configuration, the distal portion of the delivery catheter extending from a transseptal sheath 502 has a distal section bend 67, a proximal section bend 66, and a straight distal portion 69, located distal to the distal section bend 67. The straight distal portion 69 can be flexible. The shapes of these subsections allow the delivery catheter to navigate the delivery catheter into position at a native valve (e.g., a native mitral valve) and accurately deploy an anchoring device at the native valve (e.g., at the mitral position). The catheter 1114 can take any suitable form that allows the distal region to take the flexed configuration described above, such as, for example, any form described in the present application.

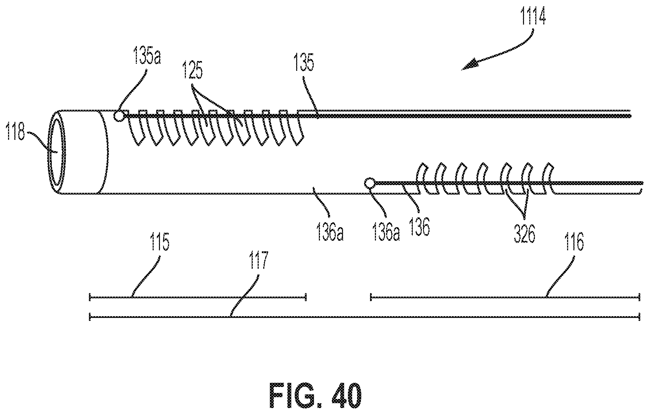

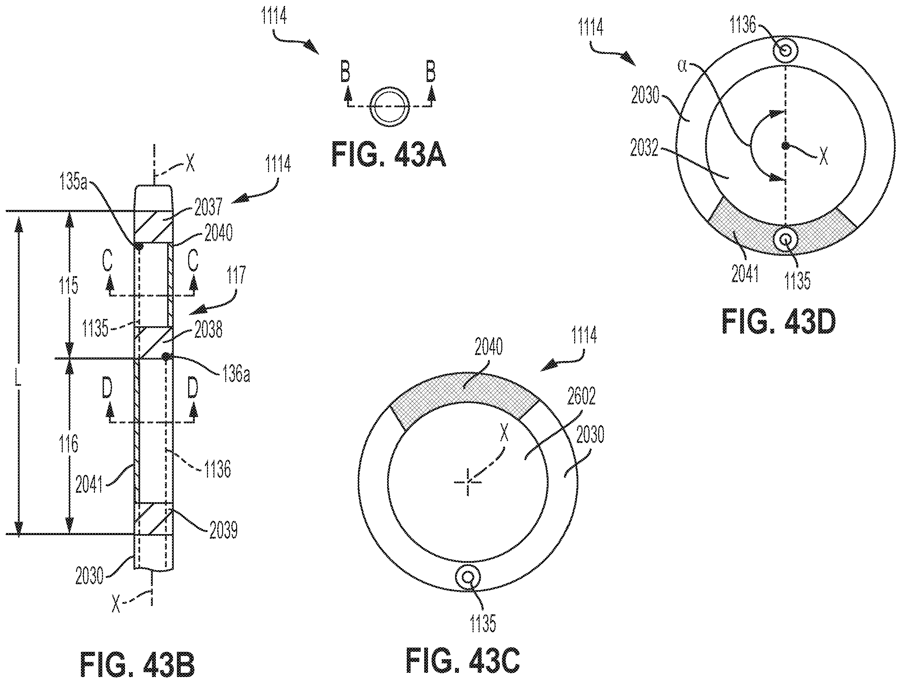

The guide sheaths and/or the distal sections of the various delivery catheters herein can include one or multiple pull wires (e.g., 2-6 pull wires) to control or actuate the delivery catheters to desired configurations. For example, distal sections of the various delivery catheters herein can have a two-pull wire system (e.g., the two-pull wire system described in Figures 43A-46). For example, the configuration shown in Figure 39 or any other configuration described in the present application can also be achieved by using a flexible tube catheter constructed with two pull rings positioned, for example, at or near the actuation points 135a, 136a (Figure 40). The pull rings can be engaged with or connected to respective pull wires. The pull wires can be positioned 180° away from one another in a circumferential direction around the delivery catheter. A first pull ring that is positioned, for example, at the distal end 65 of the delivery catheter 64, can be actuated by a first pull wire to pull the distal region of the delivery catheter including distal end portion. A second pull ring positioned proximally, for example between the distal flex section 115 and a proximal flex section 116, can be actuated by another pull wire to make the catheter curve in a different direction. For example, the second pull wire 136 can cause the catheter to upward towards the top of the left atrium. This upward flexing of the catheter can bring the distal section to a higher elevation within the left atrium, so that the distal end, and therefore valve implant, is directly above the native mitral valve.

In some embodiments, the two pull rings can be connected by a spine that is implemented on a radially opposite side of one of the pull wires, for example, opposite the pull wire for the distal-most pull ring. Such an added spine can restrict the relative movement between the pull rings, and better control the direction of deflection caused by pulling the pull wire for the distalmost pull ring, and preventing deflection of the flexible distal flex segment in a direction perpendicular to the mitral plane, or in otherwise unintended directions. While the embodiment described above can include three pull rings and two pull wires, it should be understood that any number of pull rings and/or pull wires can be used to create the various configurations described herein. In addition, it should be understood that any suitable number of spines can be used to restrict the relative movement between the pull rings.

In some embodiments, the distal flex section 115 can have one or more laser cut hypotubes (similar to the laser cut tubes described in Figures 34-37 above), arranged in a pattern such that, when bent, the distal section forms any of the various configurations described herein. Also as discussed, a laser cut distal section can have two or more actuation points that can be actuated independent from one another, for example, with separate pull wires that are, for example, controlled by separate controls (e.g., knobs, tabs, inputs, buttons, levers, switches, etc.) or other mechanisms, in order to effectuate the dual directional deflections in the distal end in the fully bent configuration (e.g., the one curve being towards the mitral plane, and the other curve being the circular portion that curves generally around the mitral annulus). In some embodiments, the separate pull wires can be controlled with a single control, as described in more detail below. In some embodiments, described below, both the distal section flex and the proximal section flex can be controlled with a single pull wire.

In some embodiments, the entire distal flex section 115 and/or the entire proximal flex section does not need to be constructed as a laser cut hypotube. For example, the proximal flexible section can be constructed, for example, with a polyether block amide (PEBAX) having a hardness of 50D or approximately 50D, that is coated over a coiled or braided tube. The distal flex section 115 and distal end portion 69 thereof, can also be constructed, for example, with PEBAX, with for example a hardness of 55D or approximately 55D, and that is also reflowed over a coiled or braided tube. Using this configuration can still yield a distal section 65 that can be shaped and actuated substantially similarly to the laser cut hypotube discussed above, without the need to form the entire distal section 65 as one or more laser cut hypotubes, or any portion from a laser cut hypotube.

While the delivery catheter 64 having a distal flex section 115 is described using the embodiments described above, it should be understood that the embodiment described above are only example. The delivery catheter 1114 can take any suitable form that is capable of creating the shape configurations described herein. In addition, the delivery catheter can be constructed with any suitable material that is capable of creating the shape configurations described herein.

Figure 40 shows a perspective view of an example distal region 117 of a delivery catheter 1114 (which can be the same as or similar to other delivery catheters described herein) for implanting a device at a native valve. For the mitral valve, this can be done using a transseptal technique. The delivery catheter is shown as assuming an example of a counterflexed configuration. In this configuration, the guide sheath 20 extends through the fossa ovalis in a direction parallel to the plane of the native valve annulus (e.g., the mitral plane). In this embodiment, the distal region 117 then exits the guide sheath 502 and is flexed with a distal bend so that its distal end 118 points down to the native valve. The user can adjust the height of the distal end, for example, by applying an upward tension on a second flex wire integrated into or attached to the catheter. For example, a pull ring located between the distal section and proximal section can be pulled to flex the proximal section upwards, thus shifting the entire distal section upward. Continued adjustment by applying additional tension to the distal section at the same time can change the distal section flex, so that the distal end remains pointing down. This can bring the distal end up to or just above the native valve annulus plane of the patient's heart.

In some embodiments, the distal region 117 can be one or more full laser cut hypotubes (similar to the laser cut catheter described in Figures 34-37 above) where the cuts are arranged in a pattern such that, when bent, the distal section forms the counterflexing configuration. In some embodiments, the counterflexed configuration of the laser cut hypotube is allowed to be shape set to a distally located downward flex and a proximally located upward flex that stretches or extends to the native valve annulus plane (e.g., that stretches or extends from the fossa ovalis to a position that is lower than the mitral plane). The distal tip of the catheter can be pulled up to position it along or just above the mitral plane, for example, by flexing or tensioning the flex wire for providing the upward flex in the proximal flex section as previously discussed. This feature allows the catheter tip to be adjusted to varying heights to accommodate different patient anatomies.

In one embodiment employing the delivery catheter 1114 with the counterflexing configuration, the distal section may not be laser cut or otherwise cut at all. For example, the distal flex section 115 of the delivery catheter 1114 can be formed by a flexible tube catheter constructed with pull rings, pull wires, and/or spines configured to move the delivery catheter 1114 into the counterflexed configuration.

While the delivery catheter 1114 having a distal flex section 115 is described using the embodiments described above, it should be understood that the embodiments described above are only example. The delivery catheter 74 can take any suitable form that is capable of creating the counterflex configuration. In addition, the delivery catheter can be constructed with any suitable material that is capable of creating the counterflex configuration (e.g., the distal flex section 115 can take the form of the delivery catheter 1114 shown in Figures 43A-46).

While in the above described embodiments, the delivery device is generally or mostly positioned above the native valve annulus plane (e.g., mitral plane), and the anchoring device is extended away from the delivery device while still on the atrial side, and advanced into the ventricle, in some embodiments, at least a portion of the delivery device itself can be positioned in the ventricle.

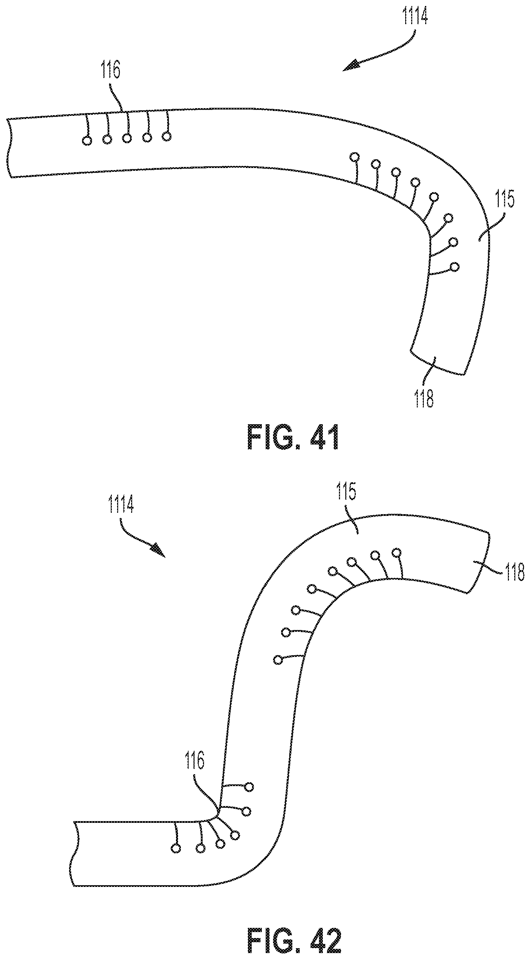

Referring to Figures 40-42, in one example embodiment, a distal region 117 of an example delivery catheter 1114 can be constructed of a hypotube having a first series of slots 125 and a second series of slots 326. The delivery catheter can also have a pull wire system (e.g., a two pull wire system that includes a first pull wire 135 and a second pull wire 136). Figure 40 shows a schematic side view of a distal region 117 of an example embodiment of a delivery catheter 1114. Figures 41 and 42 respectively show schematic perspective views of the delivery catheter 1114 of Figure 40 in partial and fully actuated states. Other delivery catheters that are deployed and used in different manners, for example, as shown in any of the embodiments discussed above, can also be constructed in a similar two pull wire system.

In one embodiment, the delivery catheter 1114 has a distal region 117 including two flexible sections 115, 116. In the descriptions provided herein, the term "first" can apply to components associated with the distal flex section 115 and flexing thereof, and the term "second" can apply to components associated with the proximal flex section 116 and the bending thereof. A first series of slots 125 can be arranged (e.g., linearly arranged or otherwise) along a first side of the distal region 117, corresponding and providing flexibility to the distal flex section 115, so that the distal flex section 115 can form a curved configuration when the delivery catheter is actuated. The configuration can be generally circular, or semi-circular, such that when positioned in the left atrium, it can pull the distal end 118 in a "downward" direction when positioned in the left atrium (as oriented in Figure 40, the distal flex section 115 curves up). A second series of slots 326 can be arranged linearly along a second side of the distal region 117, corresponding and providing flexibility to the proximal flex section 116, so that the proximal flex section 116 can form a second bend when the delivery catheter 1114 is actuated. When positioned in the left atrium, the second bend can be curved in an upward direction to pull the delivery catheter up closer to the top of the left atrium, thereby pulling the distal end 118 farther from the bottom of the left atrium (as oriented in Figure 40, the flexible section 116 curves down). The second series of slots 326 can be arranged so that they are positioned 180 degrees away from the first series of slots 125, in a circumferential direction around the delivery catheter.

The slots 125, 326 can be laser cut or formed similarly as discussed in previous embodiments, or can otherwise be formed in various other manners, so long as the slots 125, 326 contribute to the desired shaping of the delivery catheter 1114 upon actuation. The second series of slots 326 is positioned slightly proximal to the first series of slots 125 corresponding to the bending positions of the sections 115, 116, and can be offset in a circumferential direction, for example, by 180 degrees or approximately 180 degrees around the distal region 117, in order to allow for two bends in the region, where the respective radii of curvature and directions of articulation of the sections 115, 116 can be different from one another. In some embodiments, the sections 115, 116 can be offset in a circumferential direction by, for example, between about 155 degrees and about 205 degrees, such as between about 165 degrees and about 195 degrees, such as between about 170 degrees and about 190 degrees, such as between about 175 degrees and about 185 degrees, such as about 180 degrees.

In certain embodiments, each of the sections 115, 116 can have an associated pull wire 135, 136, for respectively controlling the bending of the sections 115, 116. The pull wire 135 can extend distally past the slots 125 and can be attached to the distal region 117, for example, via welding or other attachment means at connection point 135a and/or a pull ring. Similarly, the pull wire 136 can extend distally past the slots 326 and can be welded or otherwise attached to the distal region 117 at connection point 136a and/or a pull ring.

In practice, once the guide sheath 3 is arranged or positioned as desired (e.g., as shown or described elsewhere herein, for example, crossing the septum in a mitral procedure, distal regions of the delivery catheter (e.g., distal region 117 or any of the other distal regions described herein), and in some embodiments, a portion of a proximal section (e.g., proximal section 116), are advanced out of the distal opening of the guide sheath 502. The portions of the delivery catheter 1114 that extend out of the guide sheath 502 can be positioned in the left atrium before the delivery catheter is adjusted to its actuated configuration or final actuated configuration. In some cases, part of the delivery catheter can also extend into the left ventricle through the native mitral valve before the delivery catheter is adjusted to its final actuated configuration. The pull wires 135, 136 can be tensioned in order to actuate the distal region 117 and to gain articulation of the two bends, e.g., in sections 115, 116, at the distal portions of the delivery catheter 1114.

In one sequence, as shown in Figure 41, the first pull wire 135 can first be tensioned to bend section 115 to bring it to its rounded or curved actuated state, such that the curvature of section 115 brings the distal end 118 perpendicular or substantially perpendicular to the native annulus of the native valve. (e.g., the mitral plane). Then, as shown in Figure 42, the second pull wire 136 can be tensioned to its actuated or curved state, to bring the curved portion of section 115 upward to a higher position in the left atrium. Additional tensioning of the first pull wire can create additional curvature in section 115 so that the distal end 118 can remain or be moved to perpendicular or substantially perpendicular to the native annulus of the native valve.

In some embodiments, the second pull wire 136 can first be tensioned in order to bend section 116. Then, the first pull wire 135 can then be tensioned, to bend section 115 to its rounded or curved actuated state, as shown in Figure 42. In some embodiments, the first and second pull wires can initially be tensioned simultaneously. Section 115 can be flexed until the bend is at an angle from about zero degrees to about 200 degrees, or within a range of about zero degrees to about 180 degrees, or within a range of about zero degrees to about 90 degrees. Section 116 can be flexed until the bend is about zero degrees to about 60 degrees.

Figures 43A-43E, 45A-45D, and 46 illustrate an example embodiment of a delivery catheter that can operate in the same or similar manner as the delivery catheters 64, 1114 described above. Any of the components, mechanisms, functions, elements, etc. (e.g., the steering or actuation mechanism or pull wire system, pull wires, rings, spines, etc.) of this embodiment can be incorporated into other delivery catheters (and even guide sheaths) described herein.

In the example illustrated by Figures 43A-43E, 45A-45D, and 46, the distal region 117 of the delivery catheter 1114 can be constructed of flexible tube 2030. The delivery catheter has a steering/actuation mechanism or pull wire system that can be used to actuate and curve the distal region of the catheter. A steering/actuation mechanism or pull wire system herein can have one or more pull wires (e.g., 1-6 or more pull wires), one or more rings or pull rings (e.g., 1-7 or more rings), one or more spines, and/or other components.

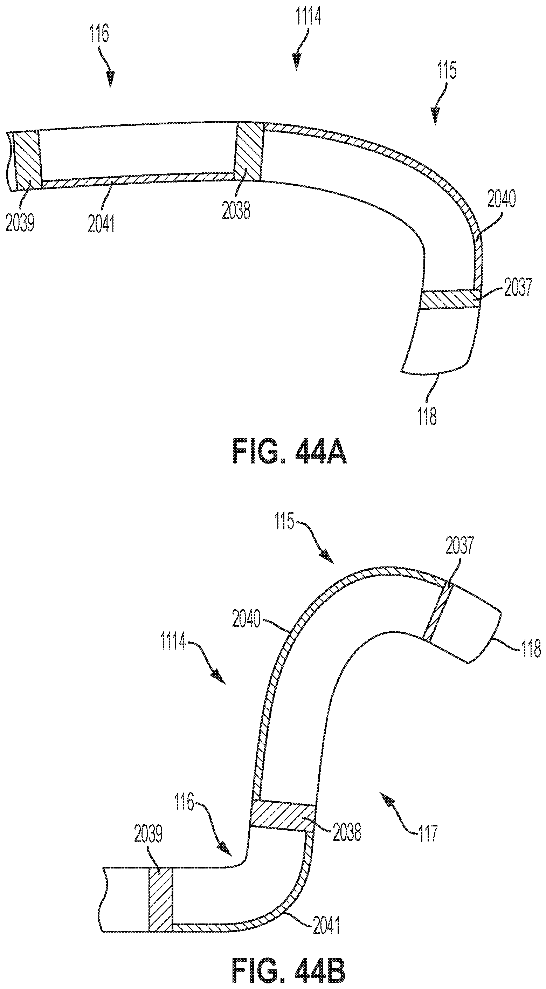

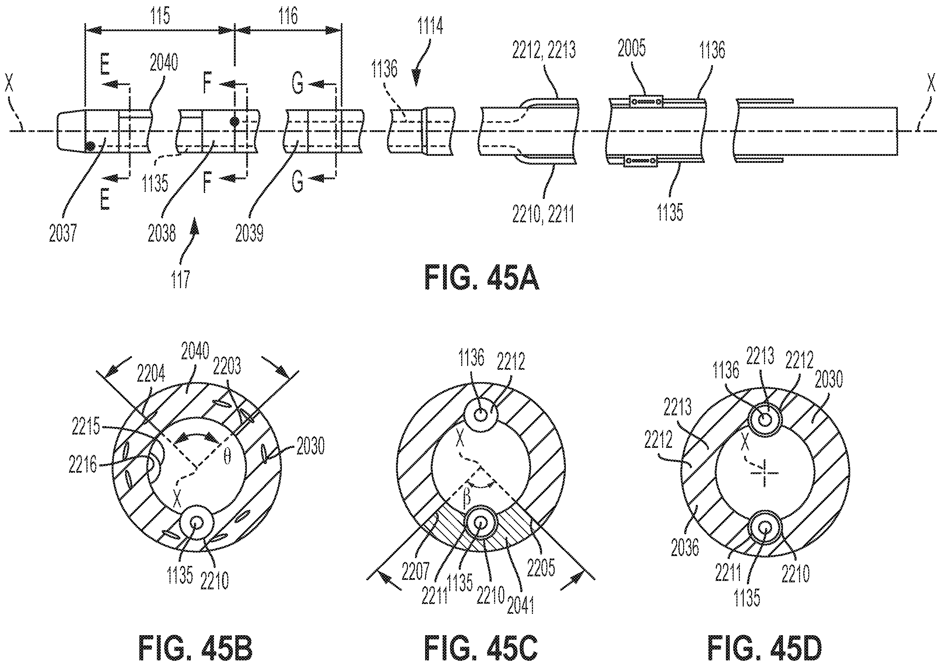

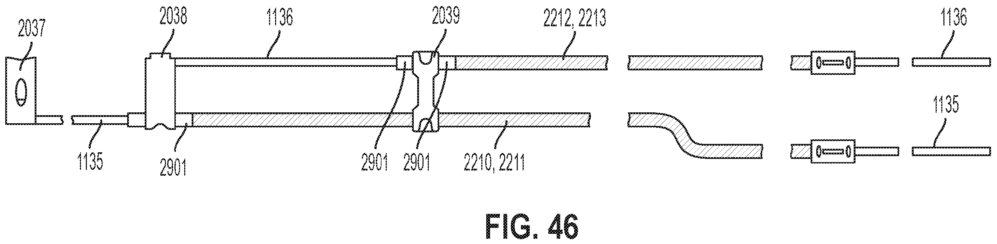

In the illustrated embodiment, the delivery catheter has a two pull wire system that includes a first pull wire 1135, a second pull wire 1136, three rings which can be pull rings and/or anchor rings (i.e., a first ring 2037, a second ring 2038, a third ring 2039), a first spine 2040, and a second spine 2041. Here, the first pull wire refers to the pull wire that actuates the distal flex section. Figure 43A shows an end view of a distal region 117 of the delivery catheter 1114. Figure 43B is a sectional view of the delivery catheter 1114 taken along the plane indicated by lines B-B. Figure 43C shows a cross sectional view of the delivery catheter 1114 taken along the plane indicated by lines C-C in Figure 43B. Figure 43D is taken along the plane indicated by a cross-sectional view of the delivery catheter 1114 taken along the plane indicated by lines D-D in Figure 43A. Figures 44A and 44B are schematic perspective views of the delivery catheter 1114 in partially and fully actuated states, respectively, similar to the views of Figures 41 and 42. Figure 45A is a partial view of the delivery catheter 1114. Figures 45B-45D show cross-sectional views of the delivery catheter taken along the planes indicated by lines B-B, C-C, and D-D, respectively, in Figure 45A. Figure 46 is a side view of the two-pull wire system for the delivery catheter 1114. Other delivery catheters or sheaths that are deployed and used in different manners, for example, as shown in any of the embodiments discussed above, can also be constructed with a similar two pull wire system. While the illustrated embodiments show the delivery catheter 1114 having rings 2037, 2038, 2039 and spines 2040, 2041, it should be understood that the delivery catheter 1114 can be constructed having any number of rings and/or spines, or without any rings or spines.

In the illustrated embodiment, the delivery catheter 1114 has a distal region 117 including the two flex sections 115, 116. Referring to Figure 43B, the distal flex section 115 extends between the first ring 2037 and the second ring 2038. A first pull wire 1135 is attached to the first ring 2037 at connection point A, and actuation of the first pull wire 1135 causes the distal flex section 115 to form a curved configuration such as that shown in Figure 41. Referring to Figs. 43B and 43C and 45A and 45B, an optional spine 2040 is connected between the first ring 2037 and the second ring 2038. The spine 2040 is made of a stiffer material than the flexible tube 2030 and, therefore, is configured to restrict the movement, such as compression, between the rings 2037, 2038 when the first pull wire 1135 is actuated. The spine 2040 can be made of, for example, stainless steel, plastic, or any other suitable material that is stiffer than the flexible tube. The flexible tube 2030 can be made out of, for example, nitinol, steel, and/or plastic, or any other suitable material or combination of materials that allow the delivery catheter 1114 to be moved to a flexed configuration. In some embodiments, the ratio of Shore D hardness for the spine 2040 to Shore D hardness of the flexible tube 2030 is about 3:1. In certain embodiments, the ratio of shore D hardness of the spine 2040 to the flexible tube 2030 is between about 1.5:1 and about 5:1, such as between about 2:1 and about 4:1, such as between about 2.5:1 and about 3.5:1. In some embodiments, the ratio of Shore D hardness of the spine 2040 to the flexible tube 2030 is greater than 5:1 or less than 1.5:1. The stiffness of the spine 2040 can be varied by varying the durometer of the spine. The stiffness can be determined by using a braid, varying the coil, or by using a stent material. The stiffness can also vary along the length of the spine by using one of these options.

In the illustrated embodiment, the spine 2040 is disposed opposite or substantially opposite the first pull wire 1135 such that a center of the spine 2040 is circumferentially offset from the first pull wire 1135 by 180 degrees or approximately 180 degrees. A center of the spine 2040 can be circumferentially offset from the first pull wire 1135 by between about 70 degrees and about 110 degrees, such as between about 80 degrees and about 100 degrees, such as between about 85 degrees and about 95 degrees. Referring to Fig. 45B, the width of the spine 2040 (defined by the angle θ) can be any suitable width that allows the delivery catheter 1114 to move to the bent configuration shown in Figures 41 and 42 (and 44A and 44B). In some embodiments, the angle θ between the edges 2204, 2203 of the spine 2040 can between about 45 degrees and about 135 degrees, such as between about 60 degrees and about 120 degrees, such as between about 75 degrees and about 105 degrees such as between about 85 degrees and 95 degrees, such as about 90 degrees. A larger angle θ allows for the spine 2040 to have more control in restricting the movement of the rings 2037, 2038 as compared to a smaller angle θ. The spine 2041 can be made of, for example, nitinol, steel, and/or plastic, or any other suitable material or combination of materials.

Referring to Figure 43B, the proximal flex section 116 extends between the second ring 2038 and the third ring 2039. The second pull wire 1136 is attached to the second ring 2038 at connection point B, and actuation of the second pull wire 1136 causes the proximal flex section 116 to form the sharper bend shown in Figures 41 and 42. Referring to Figures 43B and 43D and 45A and 45C, an optional spine 2041 is connected between the second ring 2038 and the third ring 2039. The spine 2041 is made of a stiffer material than the flexible tube 2030 and, therefore, is configured to restrict the movement between the rings 2038, 2039 when the second pull wire 1136 is actuated. The spine 2041 can be made of, for example, stainless steel, plastic, or any other suitable material that is stiffer than the flexible tube. The flexible tube 2030 can be made out of, for example, nitinol, steel, and/or plastic, or any other suitable material or combination of materials that allow the delivery catheter 1114 to be moved to a flexed configuration (e.g., the flexed configuration shown in Figure 42). In some embodiments, the ratio of Shore D hardness for the spine 2041 to Shore D hardness of the flexible tube 2030 is between about 3:1. In some embodiments the ratio of shore D hardness of the spine 2041 to the flexible tube 2030 is between about 1.5:1 and about 5:1, such as between about 2:1 and about 4:1, such as between about 2.5:1 and about 3.5:1. In some embodiments, the ratio of Shore D hardness of the spine 2041 to the flexible tube 2030 is greater than 5:1 or less than 1.5:1. The stiffness of the spine 2041 can be varied by varying the durometer of the spine. The stiffness can be determined by using a braid, varying the coil, or by using a stent material. The stiffness can also vary along the length of the spine by using one of these options.

In the illustrated embodiment, the spine 2041 is disposed opposite or substantially opposite the second pull wire 1136 such that a center of the spine 2041 is circumferentially offset from the second pull wire 1136 by 180 degrees or approximately 180 degrees. A center of the spine 2041 can be circumferentially offset from the second pull wire 1136 by between about 160 degrees and about 200 degrees, such as between about 170 degrees and about 190 degrees, such as between about 175 degrees and about 185 degrees, or about 180 degrees. Referring to Figure 45C, the width of the spine 2041 (defined by the angle β) can be any suitable width that allows the delivery catheter 1114 to move to a bent configuration having two curves in opposite directions to each other, as illustrated in Figures 17A and 39. In some embodiments, the angle β between the edges 2205, 2207 of the spine 2041 can between about 45 degrees and about 135 degrees, such as between about 60 degrees and about 120 degrees, such as between about 75 degrees and about 105 degrees such as between about 85 degrees and 95 degrees, such as about 90 degrees. A larger angle β allows for the spine 2040 to have more control (i.e., add more stiffness) in restricting the movement of the rings 2037, 2038 as compared to a smaller angle β.

Referring to Figures 43C and 43D, the delivery catheter 1114 includes a lumen 2032 that is sufficiently sized for delivering a valve repair device and/or valve repair device control components therethrough. The lumen 2602 remains sufficiently sized for the valve repair device and/or the valve repair device control components when the first pull wire 1135 and the second pull wire 1136 are actuated to move the delivery catheter 1114 to the bent configuration shown in Figure 42. The lumen 2032 can have, for example, an ovoid cross-section, a circular cross-section, or can have a cross-section with any other appropriate shape the valve repair device control components can move inside the lumen to control the valve repair device.

The connection point B for attaching the second pull wire 1136 to the second ring 2038 is positioned proximal to the connection point A for attaching the first pull wire 1135 to the first ring 2037 and can be offset in a circumferential direction, for example, by 180 degrees or approximately 180 degrees around the distal region 117. A 180-degree offset allows for two bends in opposite directions to each other, in the region, where the respective radii of curvature and directions of articulation of the sections 115, 116 can be different from one another and independent from one another. In some embodiments, the bends in sections 115, 116 can be offset in a circumferential direction by, for example, between about 155 degrees and about 205 degrees, such as between about 165 degrees and about 195 degrees, such as between about 170 degrees and about 190 degrees, such as between about 175 degrees and about 185 degrees or about 180 degrees.

Referring to Figures 43B and 43D, in some embodiments, the wires 2035, 2036 run along a length L of the delivery catheter 1114 such that the wires are parallel or substantially parallel to an axis X that extends through a center of the delivery catheter. In this embodiment, the wires 2035, 2036 are offset in a circumferential direction such that an angle α between the wires 2035, 2036 is 180 degrees. In some embodiments, the angle α between about 155 degrees and about 205 degrees, such as between about 165 degrees and about 195 degrees, such as between about 170 degrees and about 190 degrees, such as between about 175 degrees and about 185 degrees.

Referring to Figures 43A-46, in practice, once the guide sheath 502 is positioned in the atrium, the distal region 117 of the delivery catheter 1114, is advanced out of the distal opening of the guide sheath 502. The portions of the delivery catheter 1114 that extend out of the guide sheath 502 can be positioned in an atrium (e.g., left or right atrium), while in some cases, part of the delivery catheter 1114 can also extend into a ventricle (e.g., left or right ventricle) through the native valve (e.g., native mitral valve). The pull wires 1135, 1136 can be tensioned in order to actuate the distal region 117, i.e. to articulate of the two bends in sections 115, 116 at the distal portions of the delivery catheter 1114.

In one sequence, as shown in Figure 44A, the first pull wire 1135 can be tensioned, to bend section 115 to its curved actuated state, such that the curvature of section 115 causes the distal end 118 to face the mitral valve opening. Then, as shown in Figure 44B, the second pull wire 1136 can then be tensioned, to bend section 116 to its curved actuated state, such that the curvature of section 116 is in an opposite or substantially opposite direction to the curvature of section 115 when section 115 is in its actuated state. In some embodiments, the pull wires 1135, 1136 can be tensioned partially or fully in different amounts and/or orders to properly and safely navigate around or relative to the patient's anatomy during actuation. In some embodiments, actuation of the pull wires or pull wire system can be used in combination with torqueing or rotating the delivery catheter in the sheath to direct the distal region and distal tip of the catheter to a desired position and/or orientation. That is, the catheter can be rotated to point the tip of the delivery catheter 1114 at a target location.

Referring to Figures 45A-45D and 46, in certain embodiments, the delivery catheter 1114 includes a first conduit 2210 (e.g., a tube, sleeve, etc.) for housing the first pull wire 1135 and a second conduit 2212 for housing the second pull wire 1136. In the illustrated embodiment, the conduits 2210, 2212 are defined, at least in part, by a liner 2215 and an inner surface 2216 of the flexible tube 2030. In some embodiments, the conduits 2210, 2212 can take any other suitable form. In some embodiments, conduits are not used to house the pull wires 2035, 2036.

The design of the proximal section 140 and the arrangement of the pull wires 2035, 2036 can provide for an anti-whipping or anti-bending effect through the delivery catheter 1114 when the pull wires 2035 and 2036 are operated. This can allow for maintaining full torqueability of the delivery catheter 1114 through the transseptal bend. This can also facilitate the actuated shape of the distal region 117 to be held and maintained more effectively during torqueing or rotation during delivery. In some embodiments, the delivery catheter 1114 includes a first coil sleeve 2211 that extends around the first pull wire 1135 until it reaches the distal flex section 115 and a second coil sleeve 2213 that extends around the second pull wire 1136 until it reaches the proximal flex section 116. The first pull wire 1135 and the first coil sleeve 2211 around it are floatingly positioned within the first conduit 2210. The second pull wire 1136 and the second coil sleeve 2213 around it are floatingly positioned within the second conduit 2213. The coil sleeves 2211, 2213 are configured to provide for the anti-whipping or anti-bending effect and for maintaining the full torqueability of the delivery catheter 1114.

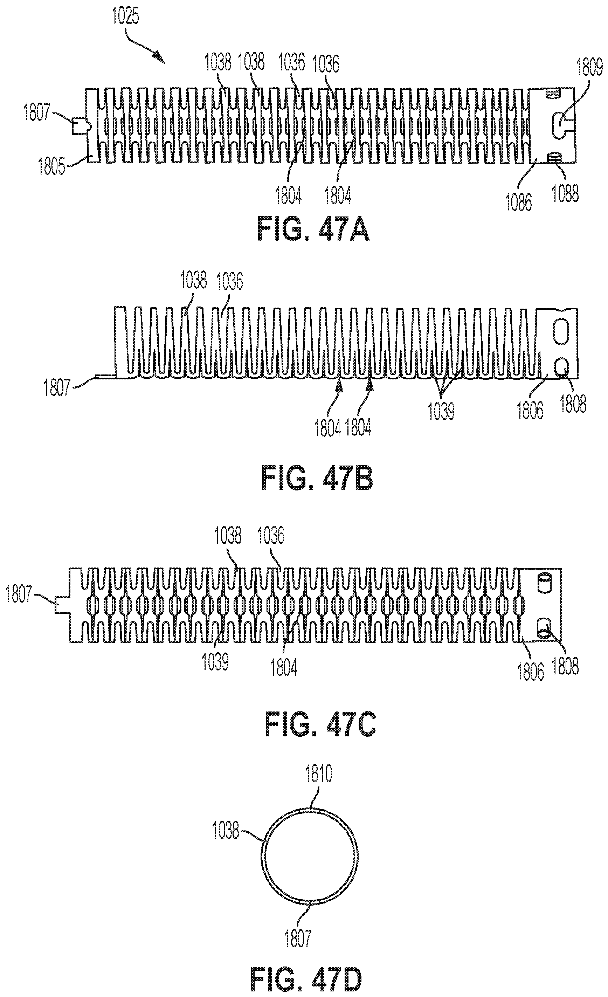

In one embodiment, a delivery catheter for delivering a device to a native valve of a patient's heart can have a flexible tube with a centered main lumen and a pair of control wire lumens. Referring now to Figures 47A-47D, a flexible tube frame 1025 for use in the distal region of a flexible delivery catheter in accordance with an example embodiment is shown. This flexible tube frame 1025 can have an overall cylindrical shape, open at both proximal and distal ends, and provides controlled flexibility to a flexible tube at the distal end of a delivery catheter. As with the other example embodiments described herein, the flexible tube frame, and distal region of the delivery catheter are not limited to a cross-section with a circular shape; the cross-section can also be elliptical or ovoid in shape. In an example embodiment, each flex section can have its own flexible tube frame. For example, a delivery catheter can have two flexible tube frames, one for each of two flex sections in a delivery catheter.

Referring to Figure 47A, a top view of a flexible tube frame of a delivery catheter is illustrated. The flexible tube frame 1025 can be made of links 1038, which are defined by slots or grooves 1036. The links 1038 can also have cut-outs 1804, and slits 1039. Each of the plurality of links 1038 can have a circular shape, and each link can be spaced apart from at least one other link by a slot 1036 (or groove) at the tops and sides of the links, in a circular or other shaped configuration.

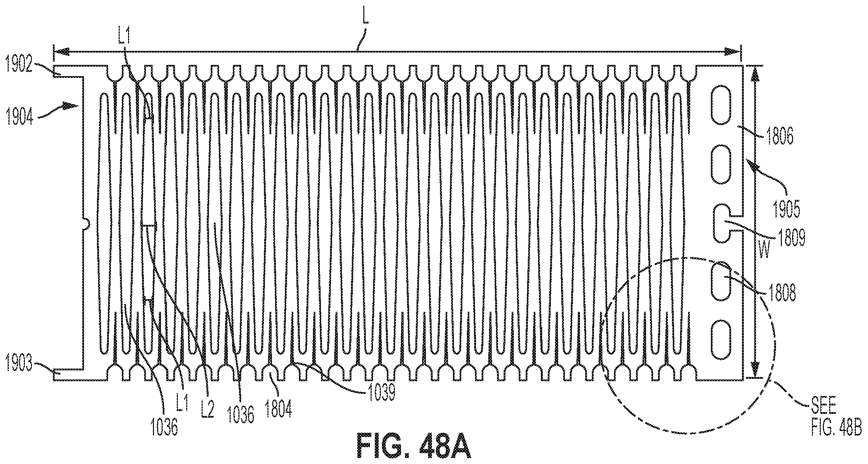

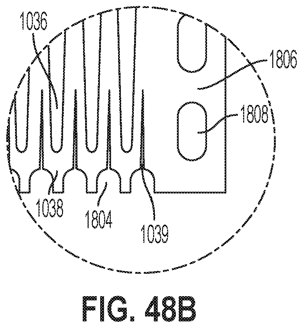

Referring now to Figure 47B, a side view of the flexible tube frame is illustrated, with cut-outs 1804 and slits 1039 positioned near the bottom of the frame. The cut-outs 1804 can be curved in shape, such as a semi-circle or semi-oval cut-out from the frame. There can be a cut-out that corresponds to each of the plurality of links, on each side of the frame 1025 (see Figure 48) so that when the frame is in a tubular configuration, each pair of cut-outs 1804 align to form a circle, oval, or cylindrical shaped opening, positioned along the bottom of the frame, as illustrated in Figure 47C. The slits 1039 as illustrated in Figures 47B and 47C can be cut into the frame such that there are two slits cut into each of the plurality of links, which extend partially upward from the frame bottom into the associated link. There is little to no space created by the slits when the flexible tube frame is straight, but the frame links can each expand at the slits when the frame is moved into a curved configuration.

Referring now to Figure 47D, a distal end of the flexible tube frame 1025 is illustrated. The cut-out 1804 for connecting a pull wire is at the top of the distal end, and the tooth 1807 for securing the anchor ring is at the bottom of the distal end.

Referring now to Figures 48A and 48B, a flat view of the flexible tube frame in a flat configuration is illustrated. The flexible tube of the delivery catheter can have a flexible tube frame made of a plurality of links disposed in the distal region of the flexible tube, the plurality of links positioned between the first ring and the second ring. The plurality of links can be cylindrically shaped, and cut from a single piece of material, such that each link is aligned with and connected to at least one adjacent link, with a slot formed between each pair of adjacent links. In some embodiments, the tube is a hypotube (which can optionally be formed from a flat sheet of material, from a preformed tubular material, from mold, from 3D printing, etc.) having properties similar to or the same as the example embodiments described above and/or illustrated in Figure 35 having the slots 1036, cut-outs 1804, and slits 1039 formed by laser cutting. Figure 48A illustrates an example embodiment of the flexible tube frame in a flat configuration, and Figure 48B illustrates a close-up view of a corner of the frame, of Figure 48A. The frame 1025 can be rectangular or substantially rectangular in shape when in the flat configuration, with the length L modified by cut-outs 1804 and slits 1039. The slots 1036 can be cut out of a central region to form the plurality of links 1038. The slots can have an elongated and/or tapered shape, such that the center region 1901 of each slot 1036 is greater in length L2 than the length L1 of the ends of each slot. The edges of the frame 1025 along each width W can also be altered by additional cut-outs. The first end of the frame can have a cut-out that is rectangular or substantially rectangular in shape with another cut-out that is semi-circular or substantially semi-circular, aligned with a midpoint of the width W of the frame. These cut-outs in the first end create two end pieces 1902, 1903 that when the frame is in a tubular configuration, provide a tooth 1807 attachment point for a pull ring 2037, which is positioned at the bottom of the frame. The tooth 1807, however, is not limited to being formed by two end pieces, but instead can be one end piece that extends distally from distal edge 1904 of the sheet. As with other example embodiments described herein, a frame as illustrated in Figure 48A can be used for each flexible section in the distal region of the delivery catheter.

The cut-outs at a second end of the frame can be a plurality of oval or substantially oval shaped windows 1808. The center window can have an additional proximal cut in it, which can be a proximal slot 1809, so that the center window is open to a proximal edge 1905 of the frame. The proximal slot 1809 can be aligned with the hypotube anchor when the delivery catheter is fully assembled. The windows can be used to provide an opening for adhesive material or polymer material to flow through and adhere to a layer underneath, or interior to, the window, embedding the component (the frame in this instance) and therefore securing it in place.

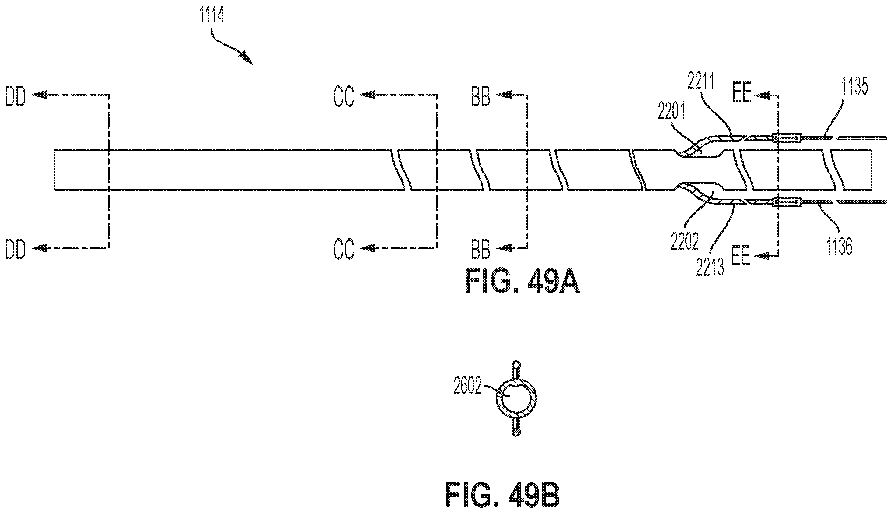

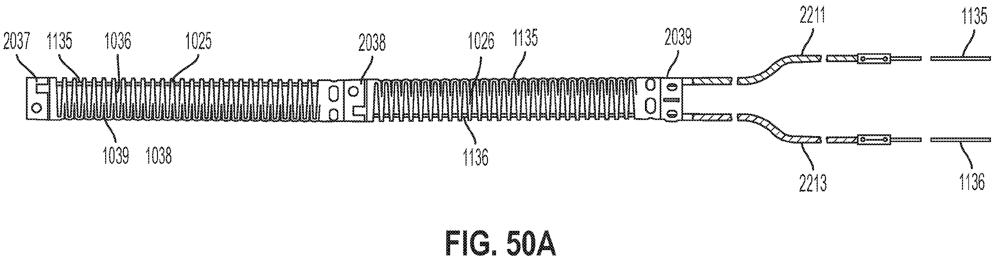

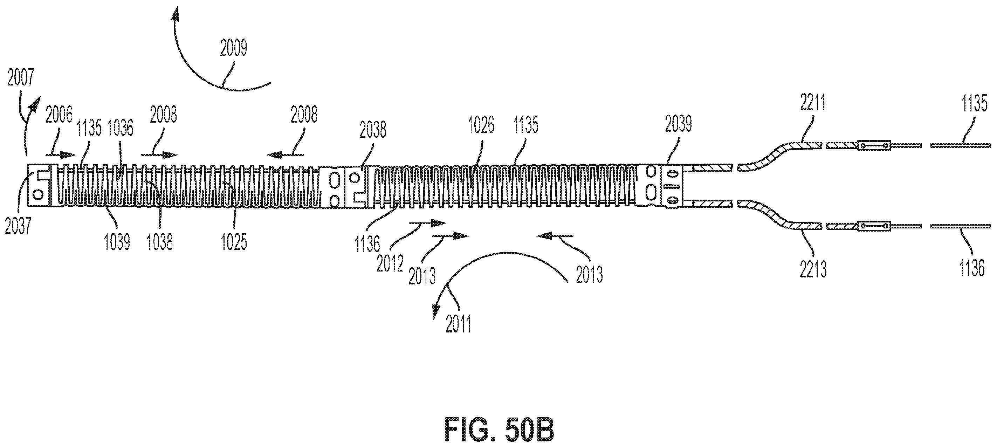

Referring now to Figures 50A and 50B, an example embodiment having two flexible tube frames is illustrated. In this embodiment, a distal section flexible tube frame 1025 is positioned in a distal end of a delivery sheath 1114 (see Fig. 49A), and a proximal section flexible tube frame 1026 is positioned proximal to the distal section flexible tube frame 1025. A first control wire 1135 extends from a proximal end of the delivery catheter, through both the proximal section flexible tube frame 1026 and the distal section flexible tube frame 1025 and can be connected to a distal pull ring 2037. A second control wire 1136 extends from a proximal end of the delivery catheter to the distal end of the proximal section flexible tube frame 1026 and can be connected to a pull ring 2038 at the distal end of the second flexible tube frame. The bending of the flexible tube frames can be controlled using the control wires. The components include a first optional ring added at a distal end of the distal section flex tube frame, a second optional ring added at a proximal end of the distal section flex tube frame, a third optional ring added at a distal end of the proximal section flex tube frame, and a fourth optional ring added at a proximal end of the proximal section flex tube frame.

The first ring at the distal end of the distal section flex tube frame can be a pull ring 2037, connected to the tooth 1807 at the distal end of the frame. The pull ring is fixedly attached to the distal end and can be attached by a weld between the pull ring and the tooth. The pull ring and distal end of the first frame can optionally be attached by any other known technique typically used and that can withstand a tensile load of at least 25 pounds. The pull ring can have cut-outs 1804 which can overlap with cut-outs of the flexible tube frame. The control wire 1135 can be fixedly attached to the pull ring. The third ring at the distal end of the proximal section flex tube frame can be another pull ring 2038, connected to a tooth 33 at the distal end of the proximal frame. As with the pull ring attached to the distal section flex tube frame, the pull ring attached to the proximal section flex tube frame can also be attached by any known technique typically used.

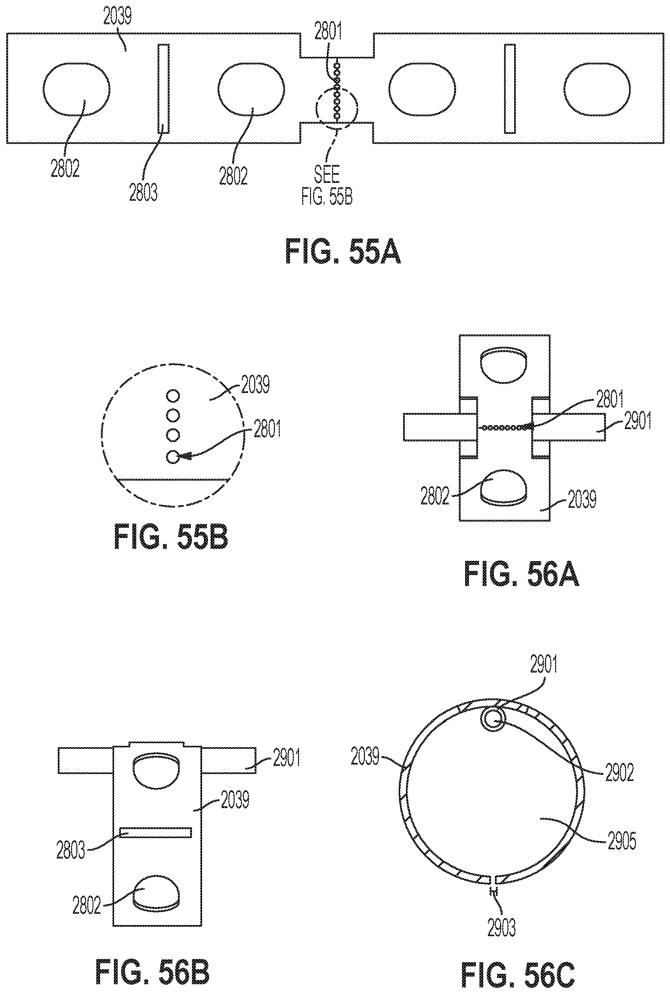

Referring again to Figure 50A, there can also be another ring at the proximal end of the proximal flexible tube frame, e.g., an anchor ring 2039. The anchor ring 2039 is attached to the proximal end of the proximal section flexible tube frame 1026. The anchor ring 2039 can have a hypotube 2901 (see Figures 56A and 56B) attached to the inside surface of it. The anchor ring 2039 can be positioned so that the hypotube 2901 is aligned with the pull wire 1136 that controls the proximal flex section 116. The pull wire 1135 that controls the distal flex section can pass through the central opening 2905 of the anchor ring 2039. The first control wire 1135 for the distal flexible tube passes through the hypotube. There can also be a second anchor ring (not pictured) at the proximal end of the distal flexible tube frame, in addition to, or in place of, the pull ring 2038, having a hypotube attached to it, for the second control wire 1136, that controls the proximal flex tube, to pass through. In such an embodiment, the second anchor ring can be positioned so that the hypotube aligns with the pull wire that controls the distal flex section 115.

Referring to Figures 55A-56C, an anchor ring and hypotube assembly according to the example embodiment of Figures 50A and 50B is illustrated. Figure 55A illustrates a flat view of the anchor ring 2039, for the proximal end of the proximal flexible tube 1036. When flat, the anchor ring can have a rectangular or substantially rectangular shape, with a width the same or about the same as that of the flexible tube frame when in a flat configuration. The anchor ring 2039 can have cut-outs 2803, which can be rectangular in shape. The anchor ring can also have its own holes 2802. The holes 2802 and cut-outs 2803 can be circular or substantially circular and can also be rectangular, or any other shape that permits layering of materials in the assembly of the delivery catheter. The anchor ring can also have weld holes 2801 for the alignment of and welding of a hypotube 2901 to the top inside of the anchor ring. Figure 55B illustrates a close-up view of the weld holes. Figure 56A illustrates a top view of the anchor ring and hypotube 2901 welded together. The hypotube is an anchor for the control wire to slidably pass through. Figure 56B is a side view of the anchor ring 2039 and hypotube 2901 at the top of the anchor ring. The hypotube can be attached by welding or other known techniques. Figure 56C illustrates an end view of the anchor ring. The hypotube can be positioned and welded to the interior surface of the top of the anchor ring. The anchor ring can be curved into a cylindrical configuration to match that of the flexible tube frame; however, the ends of the anchor ring are not required to be in contact with each other to form a complete cylinder or other substantially cylindrical shape. Figure 56C illustrates a gap 2903 which can remain once the components are assembled. The weld between the anchor ring and hypotube should be able to withstand a tensile load of at least 30 pounds, with the load being applied in a coaxial direction to the hypotube.

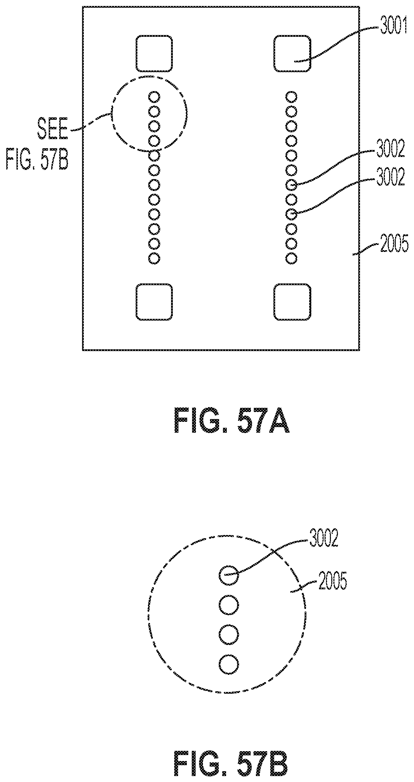

Referring now to Figures 57A-58C, the coil sleeve 2211 and proximal coil stopper 2005 are described in greater detail. Each control wire can have a coil sleeve and a coil stopper in a proximal region of the control wire. Figure 57A illustrates a flat view of the coil stopper 2005. The coil stopper can have windows 3001 and weld holes 3002. The windows allow assembly of the components to be held together with the polymer catheter material, and the weld holes permit the coil stopper to be secured to the coil sleeve. Figure 57B illustrates a close up of the weld holes 3002 which can be used to weld the coil stopper 2005 to the coil sleeve 2211. A second coil stopper 2010 for the second coil sleeve 2213 can be the same as the first coil stopper 2005 for the first coil sleeve 2211.

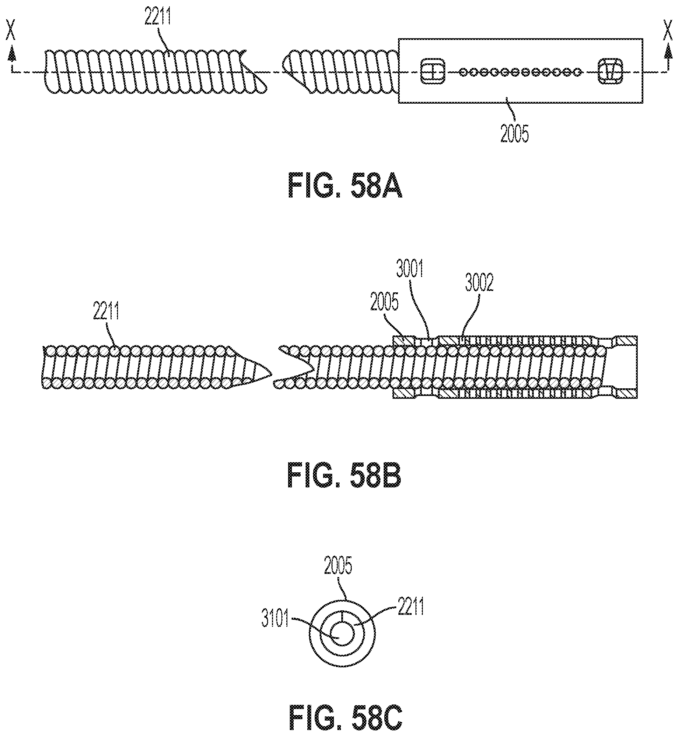

Figures 58A-58C illustrate a proximal region of a coil sleeve 2211 as it is assembled with the first coil stopper 2005. In Figure 58A, a top view is illustrated. Here the assembly windows are positioned so that there is a top proximal window and a top distal window. Each of the assembly windows in Figure 58A can be part of a pair, having a corresponding bottom assembly window, as shown in Figure 58B. The coil stopper is not limited to this particular configuration of assembly windows and weld holes. Figure 58B illustrates a cross-section of the coil sleeve and coil stopper taken along line X-X. A proximal end of the coil sleeve 2211 fits snugly within the proximal coil stopper. The weld holes are on both the top and bottom of the coil stopper. The weld holes are in the coil stopper so that a plurality of rotations of the coil in the coil sleeve can be fixed to the coil stopper. Figure 58C illustrates the coil within the coil stopper, and the channel 3101 in which the control wire slidably extends through. The second coil sleeve 2213 and second coil stopper 2010 can be the same as the first coil sleeve 2211 and first coil stopper 2005.

Referring now to Figures 49A and 49B, the distal portion of a delivery catheter 1114 containing a flexible tube frame is illustrated. Figure 49A illustrates a side view, showing an outer layer of the delivery catheter 1114 can be a polymer coating. The polymer coating can be a thermoplastic elastomer (TPE), which can be a polyether block amide (PEBA). The properties of the polyether block amide can vary along the length of the distal end of the catheter and can be chosen based on the desired flexibility and number of components of each portion of the catheter. At a proximal location along the length of the delivery catheter, the catheter 1114 has a first opening 2201 for a first control wire 1135 to exit from. The first control wire is connected to the control ring 2037 for the distal flex section. A first coil sleeve 2211 surrounds the first control wire until the control wire passes through a first coil stopper 2005 that connects the coil to the ring 2038.

Referring again to Figure 49B, at a proximal location along the length of the delivery catheter, the catheter 1114 has a second opening 2202 for a second control wire 1136 to exit from. The second control wire is connected to the pull ring 2038 for the proximal flex section. A second coil sleeve 2213 surrounds the second control wire until the control wire passes through a second coil anchor 2010 that connects the coil 2213 to the ring 2038. Figure 49B illustrates a distal end view of the delivery catheter.

The components of the catheter can extend various lengths along the delivery catheter in various embodiments. Referring to Figures 46 and 50A, the distal section flexible tube frame 1025 extends along a distal flex section 115 (See Figure 42). The first coil sleeve 2211 extends through the proximal section flexible tube frame 1026 and connects to the ring 2038. The first coil sleeve 2211 extends from the proximal end of the proximal section tube frame 1026 inside the delivery catheter for a length and then exits the delivery catheter and extends in a proximal direction for another length. An optional first control wire lumen liner can extend along the entire length of the first control wire lumen.

Referring to Figures 46 and 50A, the proximal section flexible tube frame 1026 extends along a proximal flex section 116 (See Figure 42) of the distal region 117 of the delivery catheter. The second coil sleeve 2213 is terminates at and is attached to the ring 2039. The second coil sleeve 2213 extends proximally inside the delivery catheter for a length and then exits the delivery catheter and extends in a proximal direction for another length, parallel to the first coil sleeve. The second control wire lumen liner can extend along the entire length of the second control wire lumen, which terminates at the control ring 2038.

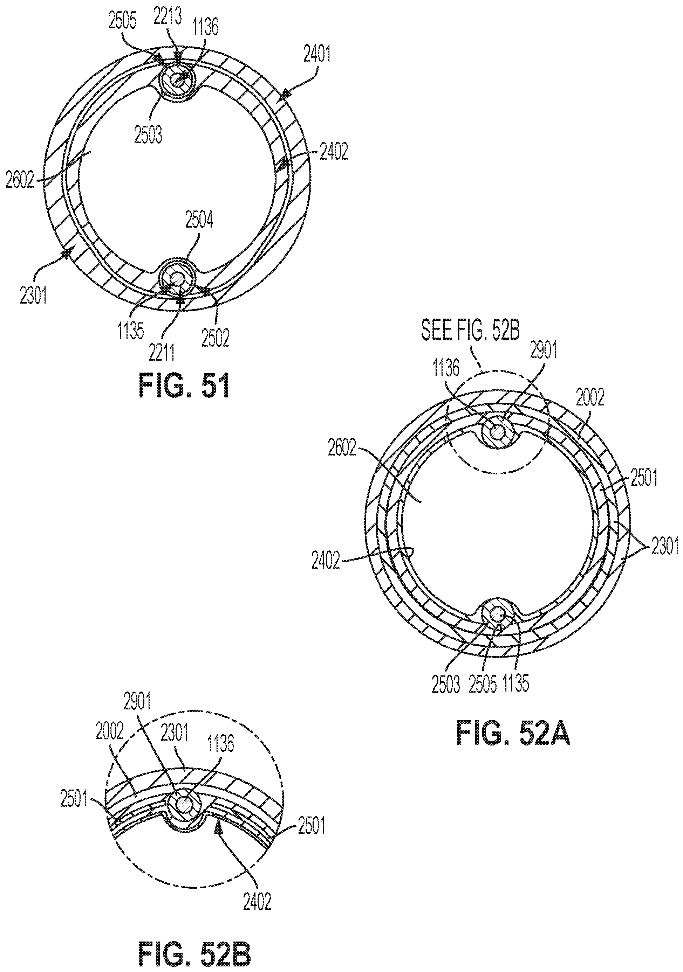

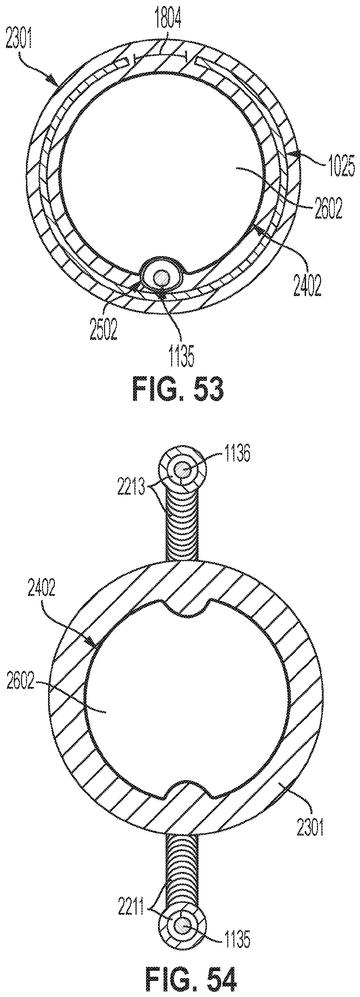

Referring now to Figures 51-54, cross-sections of the delivery catheter are illustrated, taken along various points along the length of the catheter, indicated in Figure 49A. Figures 51-53 illustrate cross-sections from the distal region of the delivery catheter, and include the distal section flexible tube frame 1025 and proximal section flexible tube frame 1026. Figure 54 illustrates a cross-section at section lines EE-EE at a proximal region of the delivery catheter, where the control wires 1135, 1136 are positioned external to the catheter.

Referring to Figure 51, the cross-section of the delivery catheter illustrated is taken along line BB-BB of Figure 49A. This cross-section is at a proximal portion of the distal region 117 having a proximal flex section 116. There is an outer layer 2301 of the catheter, which can be an outer polymer layer having any of the properties described above regarding polymers used for delivery catheters. There is an inner layer that is a primary lumen liner 2402, which can be made of etched PTFE and/or another material, and can extend the full length of the primary lumen of the delivery catheter. The liner can fully surround the interior surface of the catheter lumen. There can be a braid 2401, which provides additional support to the catheter without limiting the flexibility needed to navigate the delivery catheter inside the heart. The optional braid 2401 is embedded in the outer polymer layer. The braid can be made of a flat wire and can have an almond-shaped pattern, or any other pattern known in the art to provide both strength to and retain flexibility in a delivery catheter. There is a first control wire lumen 2503. In the control wire lumen 2503 is an optional control wire lumen liner 2502 lining the control wire lumen, and a coil sleeve 2211 surrounding a first control wire 1135. The first control wire lumen is for the first control wire 1135 to extend through, until it terminates at the ring 2037 at the distal end of the distal section flexible tube frame 1025.

Still referring to Figure 51, a second control wire lumen 2504 is provided. The second control wire lumen 2504 can have a control wire lumen liner 2505 lining it, and a coil sleeve 2213 surrounding the second control wire 1136. A hollow primary lumen 2602 is disposed in the catheter inside the primary catheter lumen liner 2402.

Referring to Figure 52A, the cross-section of the delivery catheter illustrated is taken along line CC-CC in Figure 49A. Note that the orientation of the catheter in Figures 50A and 50B is rotated 180 degrees when compared to Figures 46, 49A, 51, 52A, 52B, 53, and 54. This cross-section is at an intermediate portion of the distal region of the delivery catheter, at a location just proximal to the anchor ring 2039 that is connected to the second flexible tube frame. The first control wire 1135 and second control wire 1136 are each present at this cross-section. Figure 52B illustrates a close-up view of the top of the catheter at the cross section shown in Figure 52A, where the first control wire lumen is located. A hypotube 2901 is attached to anchor ring 2039 (see Figure 46). The coil 2213 is attached to the hypotube 2901 and the control wire 1136 extends through hypotube 2901. The hypotube can be made of stainless steel, nitinol, polymer, and/or other biocompatible material. Figure 52A further illustrates the control wire lumen 2503 with the control wire 1135 and coil 2211 in the lumen 2504 taken along line CC-CC in Figure 49A.

There can also be an optional marker band 2501. The marker band can provide an indicator to the user of the location of the proximal end of the flexible tube frame in the distal region of the delivery catheter. The marker band can be made of platinum iridium or other material that would be readable with the imaging techniques commonly used in conjunction with valve implant delivery catheters. Liner 2402, which is the liner of the primary lumen 2602 of the catheter, can extend along the entire length of the lumen 2602, including at the portion of the distal region of the delivery catheter at line CC-CC.

Referring to Figure 53, the cross-section of the delivery catheter illustrated is taken along line DD-DD in Figure 49A. This cross-section is located in the distal flex section of the delivery catheter. At this cross-section, there is the distal section flexible tube frame 1025 embedded in the outer catheter 2301. This particular cross-section is taken at a part of the catheter having a cut-out in the flexible tube frame, as indicated by the gap 1804. The interior of the primary catheter lumen and the first control wire lumen are each optionally lined with a primary lumen liner 2402 and a control wire lumen liner 2502, respectively. The first control wire lumen is occupied by the first control wire 1135. In an example embodiment, the second control wire does not extend through the distal flexible tube frame. However, in some embodiments, a second empty lumen could optionally be disposed in this space. The outer catheter is made of a polymer as described above.

Figure 54 illustrates the cross-section taken along line EE-EE of Figure 49A, in a proximal region, proximal to both flexible tube frames. The primary catheter lumen 2602 has a liner 2402, which extends along the length of the delivery catheter. The control wire lumens begin at more distal locations in the catheter. At cross-section EE-EE, the first control wire 1135 is exterior to the catheter so that it can be manipulated at its proximal end (not shown) by an operator. The first control wire 1135 is surrounded by a first coil sleeve 2211 and the first coil sleeve is partially covered by the first coil stopper 2005. At cross-section EE-EE, the second control wire 1136 is exterior to the catheter so that it can be manipulated at its proximal end (not shown) by an operator. The second control wire 1135 is surrounded by a second coil sleeve 2211 and the second coil sleeve is partially covered by the second coil stopper 2010.

Referring again to Figures 50A, 50B and 53, the delivery catheter can have a first control wire in the first control wire lumen 2502 (Note again that Figures 50A and 50B is rotated 180 degrees when compared to Figures 46, 49A, 51, 52A, 52B, 53, and 54). The first control wire lumen 2502 can extend along the delivery catheter all the way to the first pull ring 2037. The control wire lumen 2502 can be disposed inside and along the first and second flexible tube frames (i.e., in the interior of the flexible tube frames). The hypotube of the anchor ring and a first coil sleeve 2211 are disposed in the first control wire lumen 2502. The first coil sleeve 2211 is connected to the distal flexible tube frame 1025 by a hypotube 2901 that is connected to the pull ring 2038.

The distal end of the second coil sleeve 2213 can be connected to the anchor ring 2039. The coil surrounds the control wire to protect it and to prevent the plastic material around the coil sleeve from compressing, foreshortening, or buckling when the control wire is pulled. A first coil stopper 2005 can surround the proximal end of the first coil sleeve. The first coil sleeve 2211 has a distal region within the catheter, in the first control wire lumen, and exits the catheter at opening 2201. A proximal region of the first coil sleeve is external to the delivery catheter and extends for a length. At least the portion of the first control wire 1135 that extends from the second ring to the first ring is not covered by the first coil sleeve (see Figure 53). The first coil stopper 2005 maintains the integrity of the proximal end of the coil sleeve 2211 and provides a means for connecting the first coil sleeve 2211. The first coil sleeve 2211 prevents the control wire 1135 from compressing, foreshortening, or buckling delivery catheter in the area of the wire that is covered by the coil sleeve.

The second control wire lumen 2504 can extend to the second pull ring 2038. The second control wire lumen can extend inside and along the proximal flexible tube frame 1026 (i.e., in the interior of the proximal flexible tube frame). A second coil sleeve 2213 is disposed in the second control wire lumen. The coil is located proximal to the second flexible tube frame 1026 and is connected to the anchor ring 2039. There can be a second hypotube to which the coil 2213 is connected or it can be connected directly to the anchor ring 2039. The coil 2213 surrounds the control wire 1136 to protect it and to prevent the plastic material around the coil sleeve from compressing, foreshortening, or buckling when the control wire 1136 is pulled. A second coil stopper 2010 can surround the proximal end of the coil sleeve. The coil sleeve 2211 has a distal region within the catheter, in the control wire lumen, and exits the catheter at opening 2201. A proximal region of the second coil sleeve is external to the delivery catheter and extends for a length. At least the portion of the second control 1136 wire that extends from the anchor ring 2038 to the anchor ring 2039 is not covered by the second coil sleeve 2213. This allows the catheter to flex in the area where the control wire 1136 is not covered. The second coil stopper 2010 maintains the integrity of the proximal end of the second coil sleeve 2213.

The delivery catheter 1114 includes a first control wire lumen 2502 for housing the first control wire 1135, and a second control wire lumen 2212 for housing the second control wire 1136. In the illustrated embodiment, the control wire lumens are each defined, at least in part, by an optional liner. In some embodiments, the control wire lumens can take any other suitable form.

In some embodiments, the delivery catheter 1114 includes a first coil sleeve 2211 that extends around the first control wire until it reaches the distal section flexible tube frame 1025. The delivery catheter can also include a second coil sleeve 2212 that extends around the second control wire until it reaches the proximal section flexible tube frame 1026. The design of the proximal section of the delivery catheter and the arrangement of the first and second control wires 1135, 1136 and first and second coil sleeves 2211, 2213 provides for an anti-whipping or anti-bending effect through the delivery catheter 1114 when the control wires are operated. This can allow for maintaining full torqueability of the delivery catheter 1114 through the transseptal bend. This can also facilitate the actuated shape of the distal region 117 to be held and maintained more effectively during torqueing or rotation during delivery.

Each control wire, control wire lumen, flexible tube frame, pull ring, anchor ring, and coil sleeve operate in a manner similar to a cinch, or a drawstring, where the control wire is the string, and the flexible tube frame allows the distal region of the catheter to be "cinched." Figure 50B illustrates the movement of the components when tension is applied to each of the control wires. At the proximal end of the device, the operator can pull the first control wire 1135 in a proximal direction, as indicated by arrows 2006. The first control wire can be tensioned partially or fully in different amounts to properly and safely navigate around a patient's anatomy. Upon applying this tension in a proximal direction to the first control wire 1135, the first control wire, connected to the first pull ring 2037 at the distal end, applies a force in a proximal direction on the top of the pull ring. This force causes the first pull ring 2037 to move both upward and in a proximal direction, as indicated by arrow 2007. The distal section flexible tube frame 1025 bends and bunches along the slots or grooves 1036, so that the slots 1036 positioned along the top curve of the frame become smaller as the tops of the frame links 1036 are pulled closer together, as indicated by arrows 2008. This is what causes the first flexible tube frame, and corresponding section of the delivery catheter, to flex so that its distal region curves upwards in the direction illustrated with arrow 2007, where the upward direction is defined as the direction that the top of the frame 1025 faces. The result of applying tension to the first control wire 1135 by pulling it in a proximal direction is that the distal flexible tube frame 1025 is curved into a configuration with its proximal and distal ends brought closer together and having a curve such as that provided by the curve 2009 in Figure 50B. In this way, the tension on the control wire is what determines the degree of curvature. As the distal flexible tube frame 1025 bends, the slits 1039 along the bottom of the frame can expand to alleviate force applied to the distal flexible tube frame.

At the proximal end of the device, the operator can pull the second control wire 1135 in a proximal direction, as indicated by arrow 2012. The second control wire can be tensioned partially or fully in different amounts to properly and safely navigate around a patient's anatomy. Upon applying this tension in a proximal direction to the second control wire 1135, the second control wire, connected to the second pull ring 2038, applies a force in a proximal direction on the top of the second pull ring. This force causes the second pull ring 2038 to move both upward and in a proximal direction, as indicated by arrow 2007. The proximal section flexible tube frame 1026 bends and bunches along the slots or grooves 1036, so that the slots 1036 positioned along the top curve of the frame become smaller as the tops of the frame links 1036 are pulled closer together, as indicated by arrows 2013. This is what causes the proximal section flexible tube frame, and corresponding section of the delivery catheter, to flex so that its distal region curves downwards in the direction illustrated with arrow 2011, where the downward direction is defined as the direction that the bottom of the proximal section flexible tube frame 1026 faces. The result of applying tension to the second control wire 1135 by pulling it in a proximal direction is that the proximal section flexible tube frame 1026 is curved into a configuration with its proximal and distal ends brought closer together and having a curve such as that provided by the curve 2011 in Figure 50B. In this way, the tension on the control wire is what determines the degree of curvature. As the proximal section flexible tube frame 1026 bends, the slits 1039 along the bottom of the frame can expand to alleviate force applied to the proximal section flexible tube frame.

Referring again to Figure 50B, the first control wire 1135 is fixedly connected to the pull ring 2036 and is slidably connected to the anchor ring 2038 by passing through a hypotube 2901 that is connected to the anchor ring 2038 (See Figure 46). The first control wire 1135 is also slidably positioned within the first control wire lumen 2502 and the first coil sleeve 2211. The first coil sleeve 2211 prevents cinching and/or bunching of the delivery catheter in regions other than that of the flexible tube. The first coil sleeve provides additional stiffness to the delivery catheter, such that the length of the delivery catheter having the coil sleeve does not flex excessively. As the first control wire 1135 is pulled in the direction of arrow 2006 to flex the distal region of the delivery catheter, a tensile load is applied to the distal end of the first control wire, where it is connected to the first pull ring 2037. The first flexible tube frame bends, but the first coil sleeve 2211 prevents curvature of the first control wire 1135 in the proximal region of the delivery catheter. When the tension in the first control wire is released, the tube frame returns to a straight configuration, the plurality of links 1038 become spaced apart again along the slots and/or grooves 1036, and the slits 1039 close again.

The second control wire 1136 is fixedly connected to the second pull ring 2038 and passes through anchor ring 2039 at the proximal end of the proximal section flexible tube frame 1026. In some embodiments, the second control wire 1136 can be slidably connected to and pass through a second hypotube in the anchor ring 2039. The second control wire 1136 is also slidably positioned within the second control wire lumen 2504 and the second coil sleeve 2213. The second coil sleeve 2213 prevents cinching and/or bunching of the delivery catheter in regions other than that of the flexible tube. The second coil sleeve provides additional stiffness to the delivery catheter, such that the length of the delivery catheter having the coil sleeve does not flex excessively. As the second control wire 1136 is pulled in the direction of arrow 2012 to flex the distal region of the delivery catheter, a tensile load is applied to the distal end of the second control wire, where it is connected to the second pull ring. The proximal flexible tube frame bends, but the second coil sleeve 2211 prevents curvature of the second control wire 1135 in the proximal region of the delivery catheter. When the tension in the second control wire is released, the tube frame returns to a straight configuration, the plurality of links 1038 become spaced apart again along the slots and/or grooves 1036, and the slits 1039 close again.

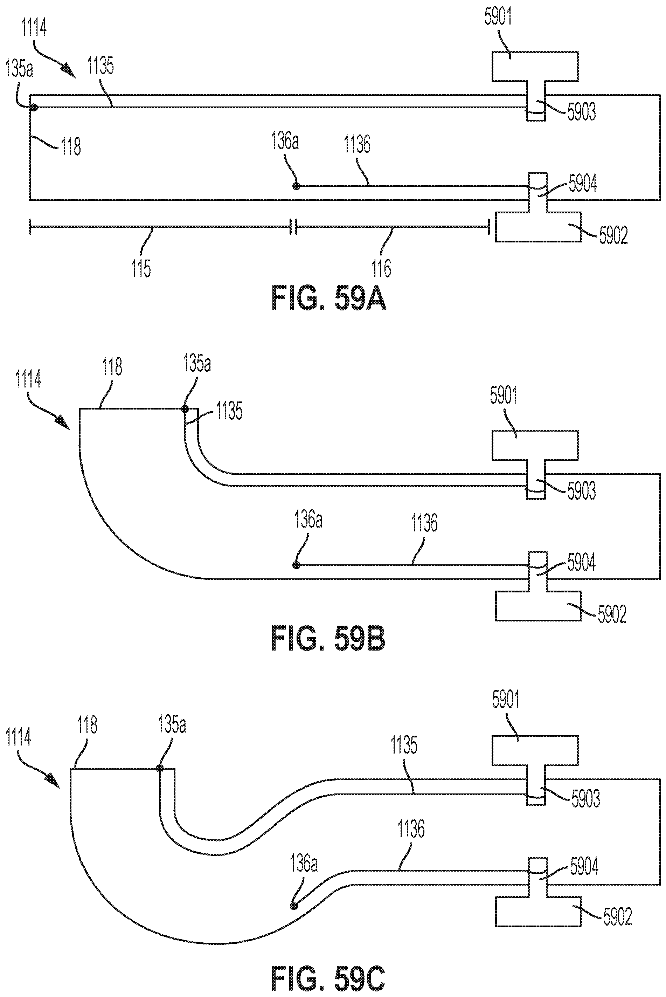

Referring now to Figures 59A-59C, schematics of an example embodiment of a counterflexing steerable catheter are shown. In the example embodiments described herein, the delivery catheter 1114 can be a steerable catheter and the terms "delivery catheter" and "steerable catheter" can be used interchangeably. The steerable catheter has a distal region 117, which has a distal flex section 115 and a proximal flex section 116. The steerable catheter also has two pull wires, where each pull wire has its own control input, such as the illustrated knobs. The control inputs for the wires 1135, 1136 can take a wide variety of different forms. Examples of control inputs include, but are not limited to, knobs, sliders, threaded connections, followers, thread driven sliders, ball and socket connections, etc. Some examples of control inputs are disclosed by US Patent Application Serial No. 15/815385, filed on November 16, 2017. Also, the control inputs are illustrated generically herein as knobs 5901, 5902. In the illustrations, the knobs 5901, 5902 are shown as having a rotational axis that is orthogonal to the axis of the catheter. However, the rotational axis of the knob or knob of any of the embodiments disclosed herein can be parallel to and/or aligned with the axis of the catheter and can optionally drive a component that is connected to the wire along the length of the catheter.

Referring again to Figures 59A-59C, there is a first pull wire 1135, or control wire, that extends from a first control input 5901 to a distal end 118 of the steerable catheter 1114 and is secured at a first actuation point 135a the distal end 118. There is a second pull wire 1136 that extends from a second control input 5902 to an intermediate location in the distal region, located at a distal end of the proximal flex segment, where it is secured at a second actuation point 136a. The first pull wire can be connected to a shaft 5903 connected to the control input 5901, which can be a flex wheel knob. The second pull wire can be connected to a second shaft 5904 connected to a control input 5902, which can be a flex wheel. The pull wires can each spool up around a flex wire as they are tensioned. However, in certain embodiments, the pull wires can each be coupled to a coupler (or to the same coupler in embodiments where there is only one flex wheel). For example, the coupler can be driven along the length of the catheter by rotating a knob. However, as noted above, the control inputs for the wires can take a wide variety of different forms.

Figure 59A illustrates the counterflexing steerable catheter in a straight configuration. Figure 59B illustrates the counterflexing steerable catheter of Figure 59A where the first control wire 1135 has been actuated by using the first control input 5901. By actuating the first control wire, tension is applied to the first control wire, and the distal flex segment is curved because the tension in the first pull wire pulls the steerable catheter from the distal end where it is secured. In Figure 59C, both pull wires have been actuated, and with the actuation of the second pull wire 1136 by the second control input, the tension that is applied pulls the proximal flex section 116 in the opposite direction (e.g., 180 degrees, approximately 180 degrees) that the distal flex section is pulled by its pull wire.

Although Figures 59A-59C illustrate the distal flex section being actuated before the proximal flex section, the segments are not required to flex in that order. In some embodiments, the proximal flex segment can be actuated first, followed by actuation of the distal flex segment. The proximal and distal flex segments can be flexed simultaneously. In a scenario where the distal flex segment is flexed first, the proximal and distal flex segments can then be flexed simultaneously to raise the position of the distal end 118 of the steerable catheter and then adjust the curve in the distal flex segment so that the distal end 118 is facing the same direction as when only the distal flex segment was actuated.

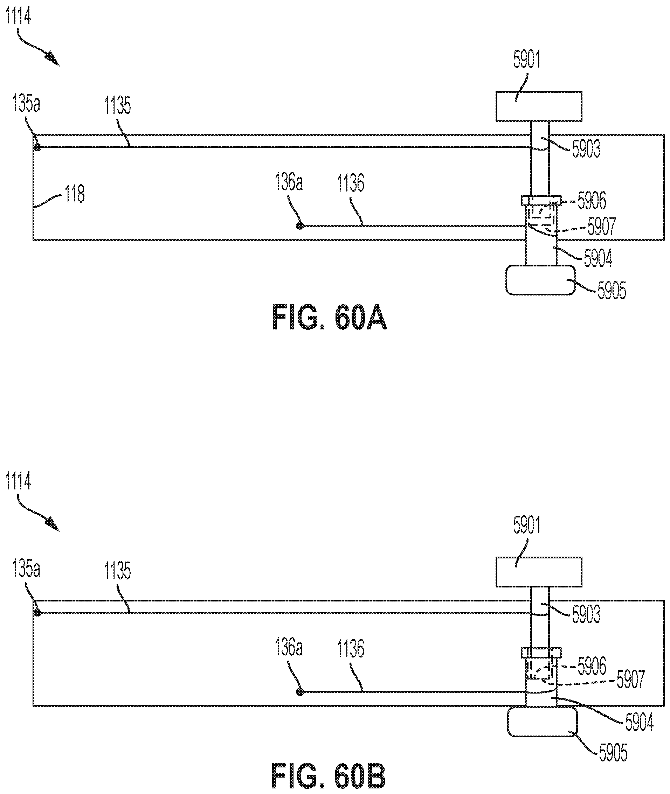

Figures 60A and 60B show a schematic of a counterflexing steerable catheter having a single control wire actuator, with a clutch-type control wire actuating mechanism to control which control wire(s) is being actuated. In this example embodiment, the distal flex section 115 has a first pull wire 1135 associated with it, and the proximal flex section 116 has a second pull wire associated with it. Both the first and second pull wires 1135, 1136 are operated by a single actuator 5901. Figure 60A illustrates the clutch-type mechanism in a disengaged position, where the first shaft 5903 (for actuating the first pull wire) attached to the flex wheel is not connected to the second shaft 5904 (for actuating the second pull wire). The actuator can be a flex wheel 5901 and a shaft attached to the flex wheel. In this embodiment, the first pull wire can be connected to the flex wheel. Turning of the flex wheel in a first direction can apply tension to the first pull wire, thereby causing the distal flex section 115 to flex, and turning of the flex wheel in a second direction can release the tension on the first pull wire 1135, causing the distal flex section to relax and straighten. The second pull wire 1136 can be attached to a second shaft 5904 that is connected to an exterior engagement mechanism 5905. The exterior engagement mechanism can be a button, as illustrated in Figures 60A and 60B, or it can be a switch, lever, or other known clutch-type mechanism. When the button is pressed, as illustrated in Figure 60B, the second screw mechanism is engaged with the first shaft 5903, thereby connecting it to the flex wheel 5901. When engaged, turning of the flex wheel in the first direction also turns the second pull wire, to tension the second pull wire and curve the proximal flex segment, and turning of the flex wheel in the second direction releases the tension in the second pull wire, causing the proximal flex section 116 to straighten.

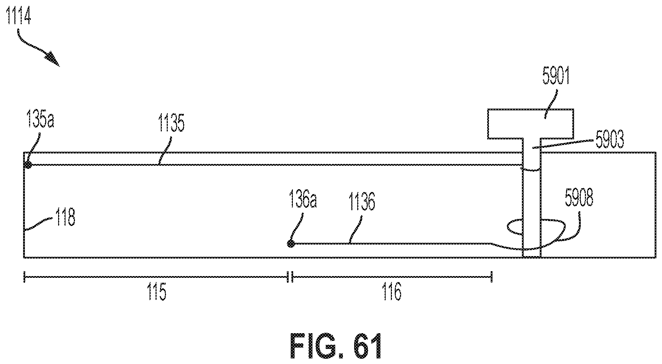

Referring now to Figure 61, a schematic of a counterflexing steerable catheter having one control input and two pull wires, in accordance with an example embodiment, is illustrated. In this embodiment, the first pull wire 1135, which is also referred to as the distal pull wire, extends longitudinally in a distal direction from the single control input to the distal end of the distal flex section 115. The second pull wire 1136, which is also referred to as the proximal pull wire, extends longitudinally in a distal direction from the single control input to a distal end of the proximal flex section 116. In a resting configuration, the second pull wire 1136 can have slack in it, such that operating the single control input to tension the wires will initially tension to the first/distal pull wire. During the initial tensioning of the first pull wire 1135, the slack 5908 in the second pull wire will be reduced by the single control input. Once the slack has been eliminated, the second pull wire will also be tensioned, simultaneously with the first pull wire. When the tension is to be released in the pull wires to straighten the steerable catheter, the straightening of the proximal flex section is completed before the straightening of the distal flex section, due to the slack in the proximal pull wire.

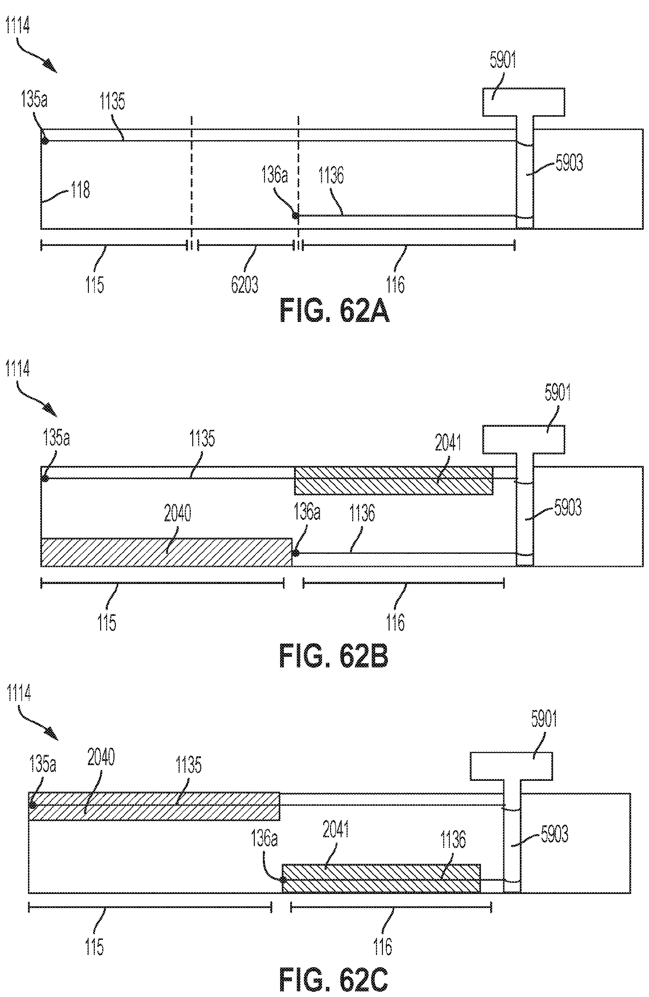

Referring now to Figures 62A-62C, schematics of a counterflexing steerable catheter having different stiffness properties along the length and circumference of the catheter are illustrated. Figure 62A illustrates an example embodiment of regions of varying stiffness. There can be two or more regions; Figure 62A illustrates a schematic having three regions. There can be a distal flex section 115 having a first stiffness section, or first spine 2040, of a first stiffness, a proximal flex section 116 having a second stiffness section, or spine 2041, of a second stiffness, and an intermediate region 6203 having a third stiffness 6203. The first stiffness section can be along all or some of the length of the distal flex section. The second stiffness section can be along all or some of the length of the proximal flex section. The regions can abruptly change or can change gradually according to a gradient. The stiffness can be consistent around the entire circumference of the steerable catheter, or it can be limited to a portion of the circumference, as in the embodiments described above having a spine that is stiffer than the remainder of the circumference of the steerable catheter.

Length of a flexible segment, thickness of the flexible segment, including overall circumference and thickness of steerable catheter wall layers, and/or durometer all affect the extent to which a flexible segment can bend, how much it can curve when actuated, and the rate at which it can bend (i.e., time-dependent bending). The greater the stiffness of a region, the less flexibility it will have. In an example embodiment, the proximal flex section 116 can have a greater stiffness than the distal flex section 115. The stiffness can vary based on the materials used, which can include any combination of metal, polymer, and/or other materials.

Referring now to Figure 62B, a schematic of varying stiffness according to one embodiment is illustrated. A distal flex section 115 has a segment of the steerable catheter, or a spine 2040, positioned opposite the distal pull wire, that is stiffer than the rest of the circumference of the steerable catheter in the distal flex region. The proximal flex section 116 has a segment of the steerable catheter, or another spine 2041, positioned opposite the proximal pull wire, that is stiffer than the rest of the circumference of the steerable catheter in the proximal flex region. The first spine 2040 can extend along a portion of or the entire length of the distal flex section 115. The spine 2040 can be made of a stiffer material than the rest of the steerable catheter tube, and therefore is configured to restrict the movement, such as compression, along the length of the distal flex segment along the portion of the steerable catheter opposite that of the pull wire 1135, when the pull wire is actuated. The spine can be made of, for example, stainless steel, plastic, or any other suitable material that is stiffer than the rest of the steerable catheter in the distal flex region. The steerable catheter can be made out of, for example, nitinol, steel, and/or plastic, or any other suitable material or combination of materials that allow the delivery catheter to be moved to a flexed configuration.

In some embodiments, the ratio of Shore D hardness for the first spine 2040 to Shore D hardness of the steerable catheter is between about 3:1. In certain embodiments, the ratio of Shore D hardness of the spine 2040 to the steerable catheter is between about 1.5:1 and about 5:1, such as between about 2:1 and about 4:1, such as between about 2.5:1 and about 3.5:1. In some embodiments, the ratio of Shore D hardness of the spine to the steerable catheter is greater than 5:1 or less than 1.5:1.

In the example embodiments represented by Figure 62B, the first spine 2040 is disposed opposite or substantially opposite the first pull wire 1135 such that a center of the spine 2040 is circumferentially offset from the first pull wire 1135 by 180 degrees or approximately 180 degrees. A center of the spine 2040 can be circumferentially offset from the first pull wire 1135 by between about 155 degrees and about 205 degrees, such as between about 165 degrees and about 195 degrees, such as between about 170 degrees and about 190 degrees, such as between about 175 degrees and about 185 degrees.

In Figure 62B, the second spine 2041 extends along at least a portion of the length of the proximal flex section 116. Just as with the first spine 2041, the second spine 2041 is made of a material that is stiffer than the rest of the steerable catheter of the proximal flex section, and therefore is configured to restrict the curvature of the proximal flex section when the second pull wire 1136 is actuated. The spine 2041 can be made of, for example, stainless steel, plastic, or any other suitable material that allows the steerable catheter to be moved to a flexed configuration. In certain embodiments, the ratio of Shore D hardness for the spine 2041 to Shore D hardness of the steerable catheter is between about 3:1. In certain embodiments, the ratio of Shore D hardness of the spine 2041 to the steerable catheter is between about 1.5:1 and about 5:1, such as between about 2:1 and about 4:1, such as between about 2.5:1 and about 3.5:1. In some embodiments, the ratio of Shore D hardness of the spine to the steerable catheter is greater than 5:1 or less than 1.5:1.

In the example embodiments represented by Figure 62B, the second spine 2041 is disposed opposite or substantially opposite the second pull wire 1136 such that a center of the spine 2041 is circumferentially offset from the second pull wire 1136 by 180 degrees or approximately 180 degrees. A center of the spine 2041 can be circumferentially offset from the first pull wire 1135 by between about 155 degrees and about 205 degrees, such as between about 165 degrees and about 195 degrees, such as between about 170 degrees and about 190 degrees, such as between about 175 degrees and about 185 degrees.

When the flex wheel 5901 is turned to apply tension to the pull wires, the distal pull wire and stiffness of its corresponding spine 2041 cause the distal flex segment to curve in a controlled manner, and the proximal pull wire and stiffness of its corresponding spine cause the proximal flex section to curve in a controlled manner. The second spine can have a greater stiffness than the first spine. The stiffness of the second spine 2041 can be greater than the stiffness of the first spine 2040 to permit the distal flex section to curve earlier and have a greater overall curve than the proximal flex section. In some embodiments, the stiffness of the distal spine can be greater than that of the proximal spine to permit greater curvature and earlier curving of the proximal flex section. The distal spine and proximal spine can also have the same stiffness.

Referring now to Figure 62C, a schematic of one embodiment is illustrated. In Figure 62C, the properties of the spines remain the same as for the example embodiments represented by the schematic of Figure 62B, except that the first spine is on the same side of the steerable catheter as the first pull wire and the second spine is on the same side as the second pull wire.

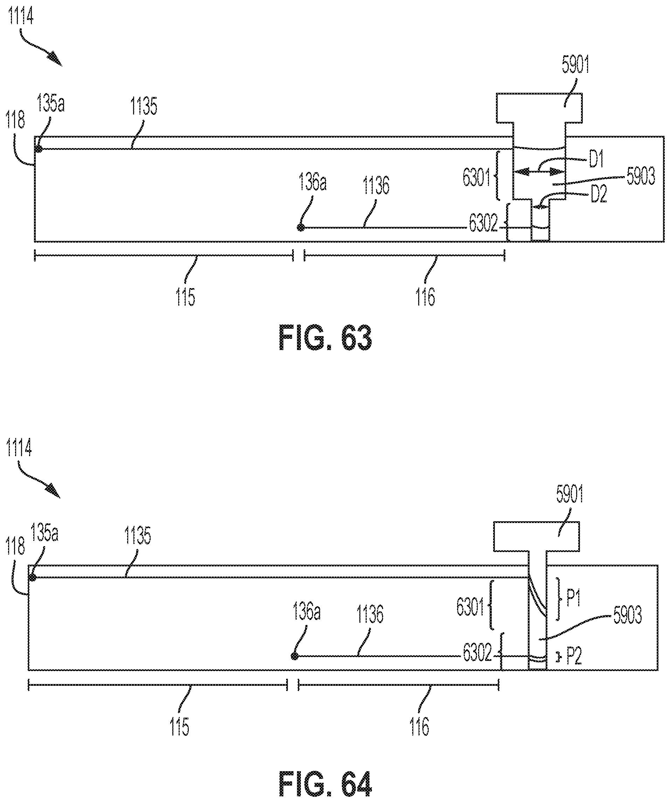

Referring now to Figure 63, a counterflexing steerable catheter having a control input 5901 to control two pull wires 1135, 1136 in accordance with an example embodiment, is illustrated. In this embodiment, the single control input pulls the two wires at two different rates. In the illustrated example, the shaft 5903 of the flex wheel has two shaft regions, each having a shaft diameter; a first shaft region 6301 having a first diameter D1, and a second shaft region 6302 having a second diameter D2. The first pull wire is wrapped around the first shaft region when the flex wheel is turned in a direction to tension the pull wires. This tensioning of the first pull wire, causes the distal flex section to curve in the direction towards the side that the first pull wire is located. The second pull wire is wrapped around the second shaft region when the flex wheel is turned in a direction to tension the pull wires. The tensioning of the second pull wire causes the proximal flex section to curve in the direction towards the side that the second pull wire is located. This can be offset 180 degrees circumferentially from the first pull wire, to curve the proximal flex section in a direction opposite that which the distal flex section is curved. This is referred to as a counterflex.

In the example illustrated by Figure 63, diameter D1 can be greater than diameter D2. When D1 is greater than D2, the distal flex section 115 flexes more rapidly than the proximal flex section 116, due to the greater diameter applying tension to the first pull wire. In certain embodiments, the rate at which each flex section is curved can be controlled by the difference in diameter between D1 and D2. A wide variety of different control inputs can be used to vary the rate at which the flex sections bend.

Referring now to Figure 64, a counter-flexing steerable catheter having a varying screw thread pitches for each of the pull wires in accordance with an example embodiment is illustrated. As with some embodiments, such as illustrated by Figures 59A-64, the control input can be a knob with an axis of rotation that is orthogonal to the length of the catheter and can have a variety of different forms. For example, in the Figure 64 example, a knob can drive a threaded shaft with an axis of rotation in the length direction of the catheter that has two differently threaded portions that drive two different wire couplers along the length of the shaft.

In the schematic illustration of Figure 64, the first pull wire 1135 is associated with a first thread pitch P1 located in a first shaft region 6301 on the shaft 6301 attached to the flex wheel 5901 and the second pull wire 1136 is associated with a second thread pitch P2 located in a second shaft region 6302 on the shaft 5903. The pitches P1 and P2 can be the same if the pull wires are to be actuated at the same rate. For one pull wire to be tensioned more aggressively than the tensioning of the other pull wire, i.e., for its associated flex section to curve earlier or have a greater overall curve, its associated thread pitch will have greater distance between threads, i.e., a greater thread pitch. In the embodiment of Figure 64, the first pull wire 1135 has a greater thread pitch P1 than the thread pitch P2 of the second pull wire 1136. Thus, the first pull wire will apply tension to the distal flex section 115 faster than the second pull wire 1136 will apply tension to the proximal flex section 116, because more of the first pull wire is being pulled with each turn of the flex wheel than of the second pull wire. The result is that the distal flex section curves at a faster rate than the proximal flex section. In some embodiments, the thread pitches P1, P2 can be the same. In some embodiments, the thread pitch P2 of the second pull wire 1136 can be greater than the thread pitch P1 of the first pull wire, so that the proximal flex section can flex at a faster rate than the distal flex section.

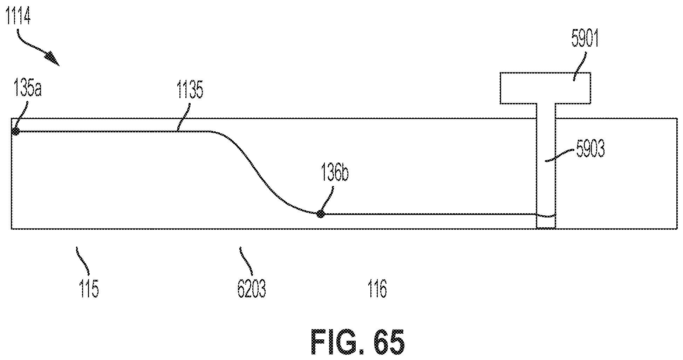

Figure 65 is a schematic illustration of a counter-flexing steerable catheter having a single pull wire to curve each of two flex sections in two different directions, in accordance with an example embodiment. In this embodiment, the pull wire 1135 is attached to the shaft 5903 of the flex wheel 5901, and then extends in one position along the length of the proximal flex section 116. The pull wire can be slidably positioned through the distal end of the proximal flex section. For example, through a hypotube positioned at the distal end of the proximal flex section. The pull wire position along the length of the steerable catheter is rotated around circumferentially, to a new position, in which it extends along the length of the distal flex section 115 to its anchor point 135a. The position of the pull wire along the distal flex section can be rotated circumferentially from the pull wire position in the proximal flex section by 180 degrees. In some embodiments, the position of the pull wire can be rotated circumferentially between about 155 degrees and about 205 degrees, such as between about 165 degrees and about 195 degrees, such as between about 170 degrees and about 190 degrees, such as between about 175 degrees and about 185 degrees.

Still referring to Figure 65, the change in circumferential position of the pull wire can be along a length of the steerable catheter that is an intermediate region 6203 between the proximal flex section 116 and the distal flex section 115. The change in circumferential position of the pull wire can begin in the distal region of the proximal flex section 116 or at the end of the proximal flex section, and it can end at the proximal end of the distal flex section or near the proximal end of the distal flex section. The circumferential position of the pull wire can also change more abruptly and can rotate around the circumference of the delivery catheter in an upward direction, without extending across any length of the delivery catheter 1114. When the flex wheel 5901 is turned to actuate and apply tension to the pull wire 1135, the proximal and distal flex sections can be curved at the same time. In some embodiment, the stiffness of the regions can vary, such as with the various embodiments of spines described herein, which can affect how much each flex section curves and at what rate the curvature occurs.

Referring to Figures 59A-65, the schematics as illustrated show the flex wheel knob 5901 extending upward out of the catheter sheath 1114, positioned orthogonal to the direction in which each of the control wires extends, when in a straightened configuration. However, in practice the flex wheel knob 5901 can be oriented 90 degrees from how it is positioned in the schematics. The flex wheel knob can drive couplers (not shown) attached to the ends of the pull wires that are advanced and/or retracted based on the rotation of the flex wheel knob.

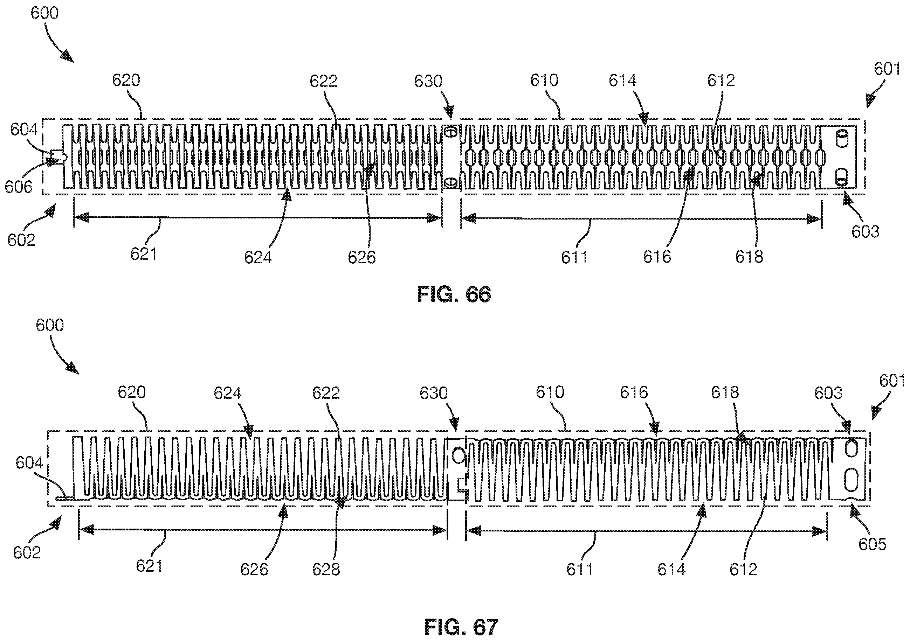

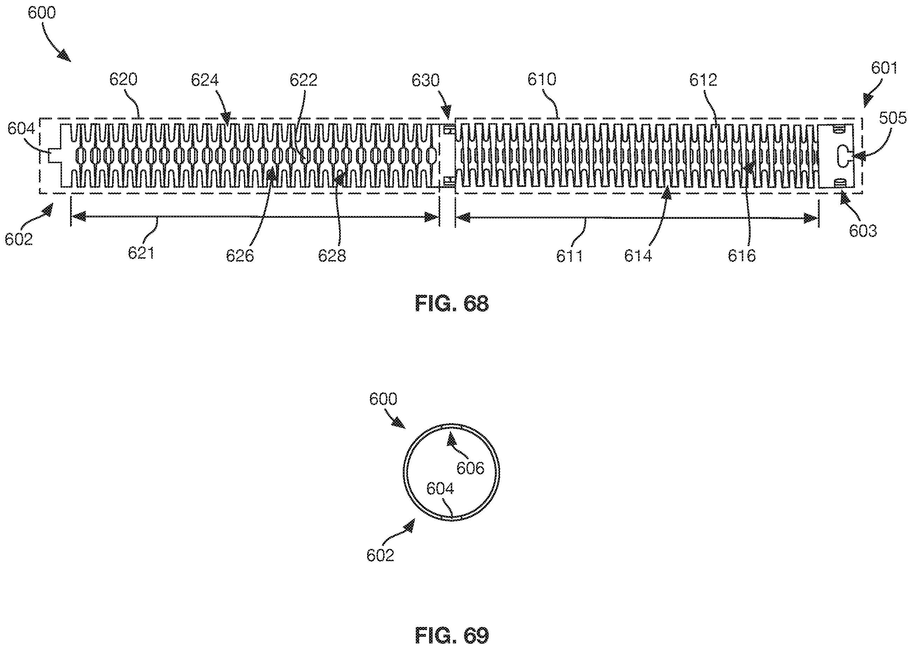

Some embodiments of a delivery catheter for delivering a device to a native valve of a patient's heart can have a single flexible tube having two flexible portions. Referring now to Figures 66-71, an example flexible tube frame 600 of the distal end of a flexible delivery catheter in accordance with an example embodiment having first and second bending portions is shown. The flexible tube frame 600 extends from a proximal end 601 to a distal end 602 and can have an overall cylindrical shape that is open at both the proximal and distal ends 601, 602. The flexible tube frame 600 provides support and controlled flexibility to first and second flexible tube portions 610, 620 (e.g., Figures 66-68) at the distal end of a delivery catheter. In an example embodiment, the flexible tube frame 600 is formed from a single tube and includes two flexible portions so that a delivery catheter can have one flexible tube frame and two flexible portions, one flexible portion for each of two flexible sections of the delivery catheter. As with some embodiments described herein, the flexible tube frame 600 and distal region of the delivery catheter are not limited to a cross-section with a circular shape (e.g., Figure 69); that is, the cross-section can also be elliptical or ovoid in shape.

The proximal end 601 of the flexible tube frame 600 includes a plurality of rounded, oval, and/or substantially oval windows or cut-outs 603 and a center cut-out or proximal slot 605 (Figure 68) that is open to the proximal end 601 of the flexible tube frame 600. The proximal slot 605 can be aligned with a hypotube anchor when the delivery catheter is fully assembled. The plurality of cut-outs 603 are used to provide an opening for adhesive material or polymer material to flow through the flexible tube frame 600 to adhere to other materials, such as a layer underneath or interior to the cut-outs 603 thereby embedding and securing the flexible tube frame 600 in a desired location.

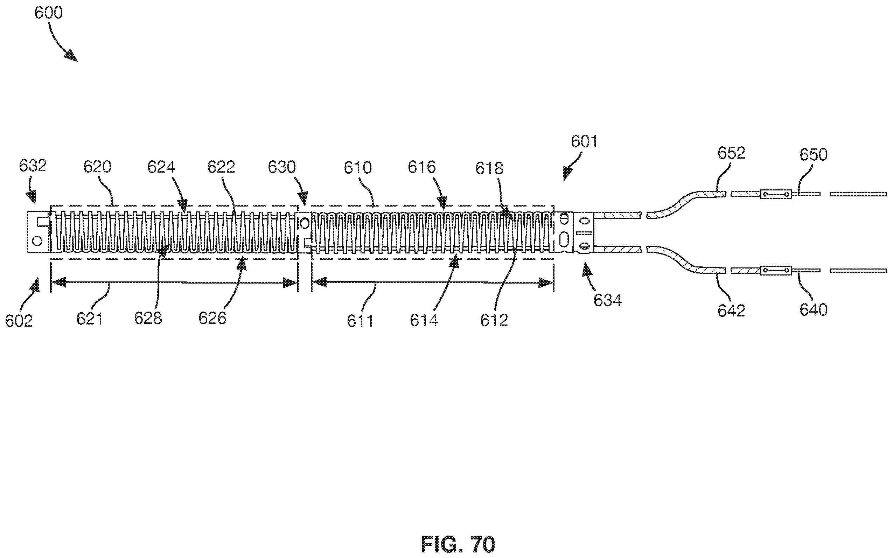

The distal end 602 of the flexible tube frame 600 includes a tooth-shaped attachment portion 604 protruding from the bottom side of the distal end 602 for attaching the flexible tube frame 600 to a distal pull ring 632 (Figures 70-71). The attachment portion 604 can be formed from a single protrusion. Optionally, the attachment portion 604 can be formed from two protrusions that are joined together when the flexible tube frame 600 is formed from a flat sheet of material. The distal end 602 also includes a semi-circular shaped distal cut-out 606 (Figure 66) positioned at the top side of the distal end 602. The distal cut-out 606 attaches to a second control wire 660 (Figures 70-71). The distal end 602 of the flexible tube frame 600 is shown in Figure 69 to show the circular cross-sectional shape of the flexible tube frame 600 and the relative positions of the attachment portion 604 and the cut-out 606.

Referring to Figure 66, a top view of the flexible tube frame 600 is shown. The flexible tube frame 600 has first and second bending portions 610, 620 that each have a length 611, 621. Each of the first and second bending portions 610, 620 is formed from a plurality of links 612, 622 that are defined by slots or grooves 614, 624. The links 612, 622 can also include cut-outs 616, 626 and slits 618, 628. Each of the plurality of links 612, 622 can have a circular shape, and is spaced apart from at least one other link by a slot or groove 614, 624 in a circular configuration. The slots 614, 624 are wider than the slits 618, 628 so that the flexible tube frame 600 bends towards the slots 614, 624 when tension is applied to a control wire extending through and attached to a portion of the flexible tube frame 600.

The cut-outs 616, 626 can have a rounded shape, such as a semi-circle or semioval. Each cut-out 616, 626 corresponds to one of the plurality of links 612, 622. The slits 618, 628 are formed in the flexible tube frame 600 such that there are two slits 618, 628 cut into each of the plurality of links 612, 622. The slits 618, 628 extend partially away from the bottom or top, respectively, of the flexible tube frame 600 and into the particular link 612, 622. The slits 618, 628 operate similar to relief cuts in that the slits 618, 628 are closed or substantially closed when the flexible tube frame 600 is in a straight configuration and can open or expand when the frame links 612, 622 move apart as the flexible tube frame 600 is transitioned into a bent configuration.

As can be seen in Figures 66-68, the slots 614 are positioned near the top of the first flexible portion 610 and the cut-outs 616 and slits 618 are positioned near the bottom of the first flexible portion 610. The slots 624 are positioned near the bottom of the second flexible portion 620 and the cut-outs 626 and slits 628 are positioned near the bottom of the second flexible portion 620. That is, the first flexible portion 610 is configured to bend toward the top of the flexible tube frame 600 and the second flexible portion 620 is configured to bend toward the bottom of the flexible tube frame 600.

The links 612, 622 of the flexible tube frame 600 are formed by laser cutting various shapes in a tube of material. In particular, the shape of the links 612, 622 is defined by the slots 614, 624, cut-outs 616, 626, and slits 618, 628. The slots 614, 624 can have an elongated and/or tapered shape such that the center region of each slot 614, 624 is wider than the ends of the slots 614, 624. The slits 618, 628 extend from the cut-outs 616, 626 toward the center of the slots 614, 624. The proximal end 601 of the tube is cut to form cut-outs 603 and the distal end 602 of the tube is cut to form the attachment projection 604.

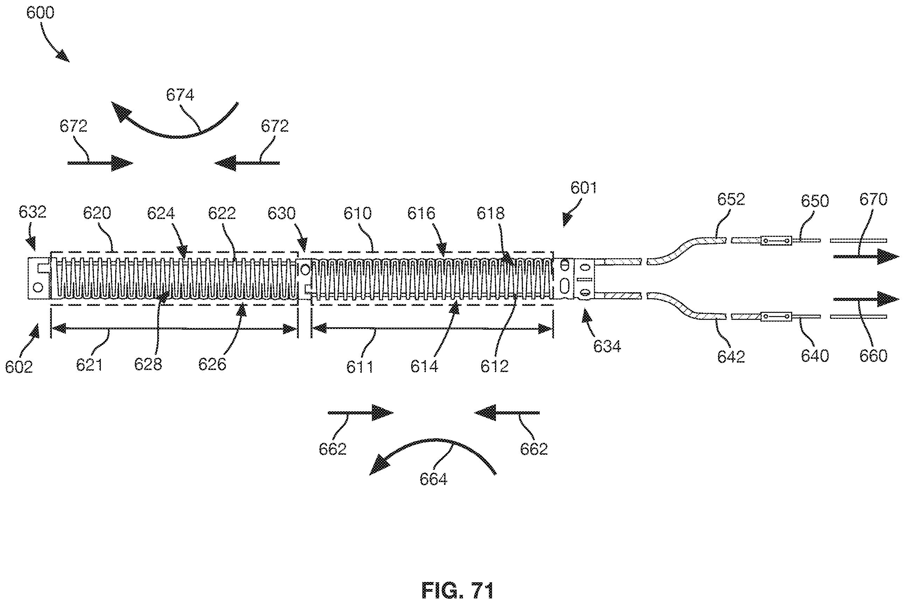

Referring now to Figures 70 and 71, the flexible tube frame 600 is shown assembled to other components of the distal end of the delivery catheter. The distal end assembly includes the flexible tube frame 600, an optional distal pull or moveable ring 632, and an optional proximal stationary, fixed, or anchor ring 634. A first control wire 640 extends through the first flexible portion 610 to the middle portion 630. A second control wire 650 extends through the first and second flexible portions 610, 620 to attach to the distal pull ring 632. Both the first and second control wires 640, 650 extend through first and second coil sleeves 642, 652 that are similar to the coil sleeves described above. The middle portion 630 is integrally formed with the first and second flexible portions 610, 620 and operates similar to the pull ring 632.

The different orientation of the links 612, 622 in the first and second flexible portions 610, 620 contributes to the different bending directions of the first and second bending portions 610, 620. That is, the links 612 in the first flexible portion are oriented toward the bottom of the flexible tube frame 600 so that the first flexible portion 610 bends towards the bottom of the flexible tube frame 600 while the links 622 in the second flexible portion are oriented toward the top of the flexible tube frame 600 so that the second flexible portion 620 bends towards the top of the flexible tube frame 600.

The difference between the bending directions of the first and second flexible portions 610, 620 can be described by an offset angle measured between the first and second flexible portions 610, 620 in the bent condition. The offset angle can be, for example, an angle between about 160 degrees and about 200 degrees, such as between about 170 degrees and about 190 degrees, such as between about 175 degrees and about 185 degrees, or about 180 degrees.

When the first and second flexible portions 610, 620 are in a fully bent condition, the position of the distal end 602 relative to the proximal end 601 of the flexible tube frame 600 depends on the lengths 611, 621 of the first and second flexible portions 610, 620 and their respective bend radii. Increasing the length 611, 621 of one of the flexible portions 610, 620 increases the angle of an arc formed by the flexible portion 610, 620 when the flexible portion 610, 620 is fully bent. Increasing the bend radius of the flexible portion 610, 620 without changing the length 611, 621 has an opposite effect-i.e., the arc angle decreases as the bend radius increases for a given length 611, 621.

In some embodiments, the first and second flexible portions 610, 620 can be assembled with a flexible material, such as a polymer. The durometer or hardness of the flexible material can be the same durometer or hardness along the length of the flexible tube frame 600, or can have different durometers along the length of the flexible tube frame 600. When assembled, the bending characteristics of the first and second flexible portions 610, 620 depend on the stiffness or resistance to bending of the flexible material. That is, using a stiffer material will require more force to bend, thereby forming a greater bending radius in the stiffer section. Similarly, a lower durometer material will be softer and easier to bend, thereby resulting in a reduced bending radius relative to the relatively stiffer portion. Various combinations of soft and hard polymer materials can be used in the first and second flexible portions 610, 620 to provide a large variety of bending radii for first and second bending portions 610, 620.

Referring again to Figures 70 and 71, the bending of the flexible tube frames can be controlled using the control wires 640, 650. Each control wire, flexible tube frame, pull ring, anchor ring, and coil sleeve operate in a manner similar to a cinch, or a drawstring, where the control wire is the string, and the flexible tube frame allows the distal region of the catheter to be "cinched." That is, as tension is applied to one or both of the control wires 640, 650, the length of the control wire 640, 650 disposed within the flexible tube frame 600 is shortened so that portions of the flexible tube frame 600 move closer to each other. Because the slots 614, 624 are wider than the cut-outs 616, 626 and slits 618, 628 and because the control wires 640, 650, the flexible tube frame 600 tends to bend toward the slots 614, 624 when tension is applied to one of the first and second control wires 640, 650.

Referring now to Figure 71, the movement of the components of the flexible tube frame 600 is shown when tension is applied to each of the control wires 640, 650. At the proximal end 601 of the flexible tube frame 600, the operator can pull the first control wire 640 in a proximal direction 660. Because the first control wire 640 is attached to the middle portion 630, application of tension to the first control wire 640 in the proximal direction 660 exerts a force on the middle portion 630 in the proximal direction 660 that, in turn, exerts a compressive force on the first flexible portion 610. Consequently, the links 612 are squeezed together as indicated by opposing arrows 662 such that the gaps between the links formed by the slots 614 are reduced. Because the widest portion of the slots 614 is arranged along one side of the first flexible portion 610, the first flexible portion 610 is bent toward the side where the slots 614 are formed, as is indicated by arrow 664. In this way, the tension applied to the first control wire 640 is what determines the degree of curvature of the first flexible portion 610. Arranging the first control wire 640 along the side of the flexible tube frame 600 closest to the widest portion of the slots 614 enables the first flexible portion 610 to be bent closest to a fully bent or maximum bend condition where adjacent links 612 contact each other and the slots 614 are closed. As the first flexible portion 610 bends and the slots 614 close in response to the compressive force applied by the first control wire 640, the slits 618 can expand to alleviate the resulting tension forces on the opposite side of the first flexible portion 610. When the tension in the first control wire 660 is released, the first flexible portion 610 of the flexible tube frame 600 returns to a straight configuration, the plurality of links 612 become spaced apart again along the slots 614, and the slits 618 close again.

At the proximal end 601 of the flexible tube frame 600, the operator can pull the second control wire 650 in a proximal direction 670. Because the second control wire 650 is attached to the distal pull ring 632, application of tension to the second control wire 650 in the proximal direction 670 exerts a force on the distal pull ring 632 in the proximal direction 670 that, in turn, exerts a compressive force on the second flexible portion 620. Compressive force is not exerted on the first flexible portion 610, due to the sleeve 652 around the wire 650 in the first flexible portion 610. Consequently, the links 622 are squeezed together as indicated by opposing arrows 672 such that the gaps between the links formed by the slots 624 are reduced. Because the widest portion of the slots 624 is arranged along one side of the second flexible portion 620, the second flexible portion 620 is bent toward the side where the slots 624 are formed, as is indicated by arrow 674. In this way, the tension applied to the second control wire 650 is what determines the degree of curvature of the second flexible portion 620. Arranging the second control wire 650 along the side of the flexible tube frame 600 closest to the widest portion of the slots 624 enables the second flexible portion 620 to be bent closest to a fully bent or maximum bend condition where adjacent links 622 contact each other and the slots 624 are closed. As the second flexible portion 620 bends and the slots 624 close in response to the compressive force applied by the second control wire 650, the slits 628 can expand to alleviate the resulting tension forces on the opposite side of the second flexible portion 620. When the tension in the second control wire 670 is released, the second flexible portion 620 of the flexible tube frame 600 returns to a straight configuration, the plurality of links 622 become spaced apart again along the slots 624, and the slits 628 close again.

The various features of each of the embodiments described herein, including those associated with the schematics described above, can be used together in various combinations to achieve the desired differences in tension rate and curvature between the distal flex bend and proximal flex bend, for both timing, and relative rate of curvature.

In some embodiments, a "resting configuration" of the distal region of a counterflexing steerable catheter can be one where the proximal flex segment and the distal flex segment are both curved, such as illustrated in Figure 59C. This can be achieved by using a shape set material and shape setting the flex regions to have a curved resting state. The first pull wire can be tensioned to straighten the curve in the distal flex section. The second pull wire can be tensioned to straighten the curve in the proximal flex section. In use, the pull wires would be relaxed as the user determines the amount of curvature to have each flex region bend.

The various manipulations and controls of the systems and devices described herein can be automated and/or motorized. For example, the controls or knobs described above can be buttons or electrical inputs that cause the actions described with respect to the controls/knobs above. This can be done by connecting (directly or indirectly) some or all of the moving parts to a motor (e.g., an electrical motor, pneumatic motor, hydraulic motor, etc.) that is actuated by the buttons or electrical inputs. For example, the motor can be configured, when actuated, to cause the control wires or pull wires described herein to tension or relax to move the distal region of the catheter. Additionally or alternatively, the motor could be configured, when actuated, to cause the pusher to move translationally or axially relative to the catheter to cause an anchoring or docking device to move within and/or into or out of the catheter. Automatic stops or preventative measures could be built in to prevent damage to the system/device and/or patient, e.g., to prevent movement of a component beyond a certain point.

It should be noted that the devices and apparatuses described herein can be used with other surgical procedures and access points (e.g., transapical, open heart, etc.). It should also be noted that the devices described herein (e.g., the deployment tools) can also be used in combination with various other types of valve repair or replacement devices and/or prosthetic valves different from the examples described herein.

For purposes of this description, certain aspects, advantages, and novel features of the embodiments of this disclosure are described herein. The disclosed methods, apparatuses, and systems should not be construed as limiting in any way. Instead, the present disclosure is directed toward all novel and nonobvious features and aspects of the various disclosed embodiments, alone and in various combinations and subcombinations with one another. The methods, apparatuses, and systems are not limited to any specific aspect or feature or combination thereof, nor do the disclosed embodiments require that any one or more specific advantages be present, or problems be solved.

Although the operations of some of the disclosed embodiments are described in a particular, sequential order for convenient presentation, it should be understood that this manner of description encompasses rearrangement, unless a particular ordering is required by specific language. For example, operations described sequentially can in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms can vary depending on the particular implementation and are readily discernible by one of ordinary skill in the art.

While various inventive aspects, concepts and features of the disclosures may be described and illustrated herein as embodied in combination in the example embodiments, these various aspects, concepts, and features may be used in many alternative embodiments, either individually or in various combinations and subcombinations thereof. Unless expressly excluded herein all such combinations and subcombinations are intended to be within the scope of the present application. Still further, while various alternative embodiments as to the various aspects, concepts, and features of the disclosures-such as alternative materials, structures, configurations, methods, devices, and components, alternatives as to form, fit, and function, and so on-may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein.

Additionally, even though some features, concepts, or aspects of the disclosures may be described herein as being a preferred arrangement or method, such description is not intended to suggest that such feature is required or necessary unless expressly so stated. Still further, example or representative values and ranges may be included to assist in understanding the present application, however, such values and ranges are not to be construed in a limiting sense and are intended to be critical values or ranges only if so expressly stated.

Moreover, while various aspects, features and concepts may be expressly identified herein as being inventive or forming part of a disclosure, such identification is not intended to be exclusive, but rather there may be inventive aspects, concepts, and features that are fully described herein without being expressly identified as such or as part of a specific disclosure, the disclosures instead being set forth in the appended claims. Descriptions of example methods or processes are not limited to inclusion of all steps as being required in all cases, nor is the order that the steps are presented to be construed as required or necessary unless expressly so stated. Further, the treatment techniques, methods, operations, steps, etc. described or suggested herein can be performed on a living animal or on a non-living simulation, such as on a cadaver, cadaver heart, simulator (e.g. with the body parts, tissue, etc. being simulated), etc. The words used in the claims have their full ordinary meanings and are not limited in any way by the description of the embodiments in the specification.

In view of the many possible embodiments to which the principles of the disclosure can be applied, it should be recognized that the illustrated embodiments are only preferred examples of the invention and should not be taken as. Rather, the scope of the disclosure is defined by the following claims.

## Claims

1. A device for delivering an implant to a native valve of a patient's heart, comprising:
a flexible hollow tube (1114) extending from a proximal end to a distal end (118), wherein the flexible hollow tube (1114) comprises:
a first flexible portion (116) extending from the proximal end to a middle portion (620);
a second flexible portion (115) extending from the middle portion (620) to the distal end (118);
a first pull wire (1135) extending through the first flexible portion (116) to attach to the middle portion (620), the first pull wire (1135) extending along a first side of the flexible hollow tube (1114), wherein applying tension to the first pull wire (1135) in a proximal direction (660, 670) creates a first bend in the first flexible portion (116) in a first direction (664);
a second pull wire (1136) extending through the first and second flexible portions (116, 115) to attach to the distal end (118), the second pull wire (1136) extending along a second side of the flexible hollow tube (1114), wherein applying tension to the second pull wire (1136) in a proximal direction (660, 670) creates a second bend in the second flexible portion (115) in a second direction (674);
wherein the second side is offset from the first side by an offset angle (α) so that the second direction (674) of the second bend is also offset from the first direction (664) of the first bend by the offset angle (α); and
wherein the offset angle (α) is in a range of about 160 degrees to about 200 degrees.

2. The device of claim 1, wherein the middle portion (620) comprises a first pull ring (2037) and the distal end (118) comprises a second pull ring (2038); preferably wherein the first pull ring (2037) is integrally formed with the middle portion (620), and/or wherein the second pull ring (2038) is integrally formed with the distal end (118).

3. The device of any of claims 1 or 2, further comprising:
a first conduit (2210) embedded within and extending along the first side of the flexible tube, wherein the first conduit (2210) extends through the first flexible portion (116) and at least a portion of the middle portion (620), and wherein the first pull wire (1135) extends through the first conduit (2210); and
a second conduit (2212) embedded within and extending along the second side of the flexible tube offset from the first conduit (2210) by the offset angle (α), wherein the second conduit (2212) extends through the first flexible portion (116), the middle portion (620), and the second flexible portion (115), and wherein the second pull wire (1136) extends through the second conduit (2212).

4. The device of any of claims 1 to 3, wherein:
a proximal end of the first pull wire (1135) is connected to a first control mechanism (650; 5901) for controlling tension to the first pull wire (1135); and
a proximal end of the second pull wire (1136) is connected to a second control mechanism (660; 5902) for controlling tension to the second pull wire (1136).

5. The device of any of claims 1 to 4, wherein the first bend forms an angle of between zero and 180 degrees, and the second bend forms an angle of between zero and 60 degrees; preferably wherein the first bend forms an angle of between zero and 90 degrees, and the second bend forms an angle of between zero and 45 degrees.

6. The device of any of claims 4 or 5, wherein:
the first control mechanism (650; 5901) comprises a first shaft (5903), the first shaft (5903) being rotatable to wind the first pull wire (1135) to apply tension to the first pull wire (1135); and
the second control mechanism (660; 5902) comprises a second shaft (5904), the second shaft (5904) being rotatable to wind the second pull wire (1136) to apply tension to the second pull wire (1136).

7. The device of any of claims 1 to 6, wherein a proximal end of the first pull wire (1135) and a proximal end of the second pull wire (1136) are connected to a shaft of a control mechanism (5901, 5902).

8. The device of any of claims 1 to 7, wherein:
the second pull wire (1136) comprises a slack portion (5908) when the flexible hollow tube (1114) is in a straight configuration;
the slack portion (5908) is reduced when tension is first applied to the second pull wire (1136); and
the slack portion (5908) is eliminated after continued application of tension to the second pull wire (1136) creates the second bend.

9. The device of claim 7 or claim 8, wherein:
the shaft of the control mechanism has a first portion (6301) having a first diameter (D1) and a second portion (6302) having a second diameter (D2), the first diameter (D1) being greater than the second diameter (D2);
the first pull wire (1135) is connected to the first portion (6301) and the second pull wire (1136) is connected to the second portion (6302); and
a rotation of the shaft increases a tension on the first pull wire (1135) at a greater rate than the rotation increases a tension on the second pull wire (1136).

10. The device of claim 7 or claim 8, wherein:
the shaft of the control mechanism has a first portion (6301) comprising a first thread pitch (P1) and a second portion (6302) comprising a second thread pitch (P2), the first thread pitch (P1) being greater than the second thread pitch (P2);
the first pull wire (1135) is connected to the first portion (6301) and the second pull wire (1136) is connected to the second portion (6302); and
a rotation of the shaft increases a tension on the first pull wire (1135) at a greater rate than the rotation increases a tension on the second pull wire (1136).

11. The device of any of claims 1 to 5, further comprising:
a proximal end of the first pull wire (1135) that is connected to a first shaft (5903) of a control mechanism;
a proximal end of the second pull wire (1136) that is connected to a second shaft (5904) of the control mechanism;
a clutch (5906, 5907) connecting the first shaft (5903) and the second shaft (5904), the clutch (5906, 5907) having a disengaged position and an engaged position;
wherein when the clutch (5906, 5907) is in the disengaged position and the control mechanism is actuated, a tension on the first pull wire (1135) is increased; and
wherein when the clutch (5906, 5907) is in the engaged position and the control mechanism is actuated, the tension on the first pull wire (1135) and a tension on the second pull wire (1136) are increased.

12. The device of any of claims 1 to 11, further comprising:
a first increased stiffness region (2040) extending longitudinally along at least a portion of the first flexible portion (116); and
a second increased stiffness region (2041) extending longitudinally along at least a portion of the second flexible portion (115); preferably wherein:
a longitudinally oriented center of the first increased stiffness region (2040) is circumferentially offset from the first pull wire (1135) by 180 degrees; and
a longitudinally oriented center of the second increased stiffness region (2041) is circumferentially offset from the second pull wire (1136) by 180 degrees.

13. The device of claim 12, wherein:
the first increased stiffness region (2040) extends around a first arc (θ) of a circumference of the flexible tube, the first arc (θ) having an angle between about 45 and about 135 degrees; and
the second increased stiffness region (2041) extends around a second arc (β) of the circumference of the flexible tube, the second arc (β) having an angle between about 45 and about 135 degrees.

14. The device of any of claims 1 to 13, wherein tension can first be applied to the first pull wire (1135) and then the second pull wire (1136), or wherein tension can be simultaneously applied to the first pull wire (1135) and the second pull wire (1136), or wherein tension can first be applied to the second pull wire (1136) and then to the first pull wire (1135).

15. A method of manufacturing a device for delivering an implant to a native valve of a patient's heart, the method comprising:
providing a flexible hollow tube (1114) extending from a proximal end to a distal end (118), the flexible hollow tube (1114) including a middle portion (620) disposed between the proximal end and distal end (118);
cutting a plurality of first slots (125) into the flexible hollow tube (1114) between the proximal end and the middle portion (620) to form a first flexible portion (116) extending from the proximal end to the middle portion (620);
cutting a plurality of second slots (326) into the flexible hollow tube (1114) between the middle portion (620) and the distal end (118) to form a second flexible portion (115) extending from the middle portion (620) to the distal end (118);
attaching a first pull wire (1135) to the middle portion (620), the first pull wire (1135) extending through the first flexible portion (116) along a first side of the flexible hollow tube (1114), wherein applying tension to the first pull wire (1135) in a proximal direction (660, 670) creates a first bend in the first flexible portion (116) in a first direction (664); and
attaching a second pull wire (1136) to the distal end (118), the second pull wire (1136) extending through the first and second flexible portions (116, 115) along a second side of the flexible hollow tube (1114), wherein applying tension to the second pull wire (1136) in a proximal direction (660, 670) creates a second bend in the second flexible portion (115) in a second direction (674);
wherein the second side is offset from the first side by an offset angle (α) so that the second direction (674) of the second bend is also offset from the first direction (664) of the first bend by the offset angle (α); and
wherein the offset angle (α) is in a range of about 160 degrees to about 200 degrees.

## Patentansprüche

1. Vorrichtung zum Zuführen eines Implantats zu einer nativen Klappe des Herzens eines Patienten, umfassend:
eine biegbare hohle Röhre (1114), die sich von einem proximalen Ende zu einem distalen Ende (118) erstreckt, wobei die biegbare hohle Röhre (1114) umfasst:
einen ersten biegbaren Abschnitt (116), der sich von dem proximalen Ende zu einem mittleren Abschnitt (620) erstreckt;
einen zweiten biegbaren Abschnitt (115), der sich von dem mittleren Abschnitt (620) zu dem distalen Ende (118) erstreckt;
einen ersten Zugdraht (1135), der sich durch den ersten biegbaren Abschnitt (116) erstreckt, um an dem mittleren Abschnitt (620) angebracht zu sein, wobei sich der erste Zugdraht (1135) entlang einer ersten Seite der biegbaren hohlen Röhre (1114) erstreckt, wobei das Ausüben einer Zugspannung auf den ersten Zugdraht (1135) in einer proximalen Richtung (660, 670) eine erste Biegung in dem ersten biegbaren Abschnitt (116) in einer ersten Richtung (664) erzeugt;
einen zweiten Zugdraht (1136), der sich durch den ersten und den zweiten biegbaren Abschnitt (116, 115) erstreckt, um an dem distalen Ende (118) angebracht zu sein, wobei sich der zweite Zugdraht (1136) entlang einer zweiten Seite der biegbaren hohlen Röhre (1114) erstreckt, wobei das Ausüben einer Zugspannung auf den zweiten Zugdraht (1136) in einer proximalen Richtung (660, 670) eine zweite Biegung in dem zweiten biegbaren Abschnitt (115) in einer zweiten Richtung (674) erzeugt;
wobei die zweite Seite gegenüber der ersten Seite um einen Versatzwinkel (α) versetzt ist, so dass die zweite Richtung (674) der zweiten Biegung ebenfalls gegenüber der ersten Richtung (664) der ersten Biegung um den Versatzwinkel (α) versetzt ist; und wobei der Versatzwinkel (α) in einem Bereich von etwa 160 Grad bis etwa 200 Grad liegt.

2. Vorrichtung nach Anspruch 1, wobei der mittlere Abschnitt (620) einen ersten Zugring (2037) umfasst und das distale Ende (118) einen zweiten Zugring (2038) umfasst; vorzugsweise, wobei der erste Zugring (2037) einstückig mit dem mittleren Abschnitt (620) ausgebildet ist, und/oder wobei der zweite Zugring (2038) einstückig mit dem distalen Ende (118) ausgebildet ist.

3. Vorrichtung nach einem der Ansprüche 1 oder 2, ferner umfassend:
einen ersten Kanal (2210), der in der ersten Seite der biegbaren Röhre eingebettet ist und sich entlang dieser erstreckt, wobei sich der erste Kanal (2210) durch den ersten biegbaren Abschnitt (116) und wenigstens einen Teil des mittleren Abschnitts (620) erstreckt, und wobei sich der erste Zugdraht (1135) durch den ersten Kanal (2210) erstreckt; und
einen zweiten Kanal (2212), der in die zweite Seite der biegbaren Röhre eingebettet ist und sich entlang dieser erstreckt, die von dem ersten Kanal (2210) um den Versatzwinkel (α) versetzt ist, wobei sich der zweite Kanal (2212) durch den ersten biegbaren Abschnitt (116), den mittleren Abschnitt (620) und den zweiten biegbaren Abschnitt (115) erstreckt, und wobei sich der zweite Zugdraht (1136) durch den zweiten Kanal (2212) erstreckt.

4. Vorrichtung nach einem der Ansprüche 1 bis 3, wobei:
ein proximales Ende des ersten Zugdrahtes (1135) mit einer ersten Steuereinrichtung (650; 5901) verbunden ist, um die Zugspannung auf dem ersten Zugdraht (1135) zu steuern; und
ein proximales Ende des zweiten Zugdrahtes (1136) mit einer zweiten Steuereinrichtung (660; 5902) verbunden ist, um die Zugspannung auf dem zweiten Zugdraht (1136) zu steuern.

5. Vorrichtung nach einem der Ansprüche 1 bis 4, wobei die erste Biegung einen Winkel zwischen null und 180 Grad bildet und die zweite Biegung einen Winkel zwischen null und 60 Grad bildet; vorzugsweise, wobei die erste Biegung einen Winkel zwischen null und 90 Grad bildet und die zweite Biegung einen Winkel zwischen null und 45 Grad bildet.

6. Vorrichtung nach einem der Ansprüche 4 oder 5, wobei:
die erste Steuereinrichtung (650; 5901) einer ersten Wickelwelle (5903) umfasst, wobei die erste Wickelwelle (5903) drehbar ist, um den ersten Zugdraht (1135) aufzuwickeln und eine Zugspannung auf den ersten Zugdraht (1135) auszuüben; und
die zweite Steuereinrichtung (660; 5902) eine zweite Wickelwelle (5904) umfasst, wobei die zweite Wickelwelle (5904) drehbar ist, um den zweiten Zugdraht (1136) aufzuwickeln und eine Zugspannung auf den zweiten Zugdraht (1136) auszuüben.

7. Vorrichtung nach einem der Ansprüche 1 bis 6, wobei ein proximales Ende des ersten Zugdrahtes (1135) und ein proximales Ende des zweiten Zugdrahtes (1136) mit eine Wickelwelle einer Steuereinrichtung (5901, 5902) verbunden sind.

8. Vorrichtung nach einem der Ansprüche 1 bis 7, wobei:
der zweite Zugdraht (1136) einen schlaffen Abschnitt (5908) umfasst, wenn die biegbare hohle Röhre (1114) in einer geraden Konfiguration ist;
der schlaffe Abschnitt (5908) reduziert wird, wenn zum ersten Mal eine Zugspannung auf den zweiten Zugdraht (1136) ausgeübt wird; und
der schlaffe Abschnitt (5908) beseitigt ist, nachdem eine fortgesetzte Ausübung von Zugspannung auf den zweiten Zugdraht (1136) die zweite Biegung erzeugt.

9. Vorrichtung nach Anspruch 7 oder Anspruch 8, wobei:
die Wickelwelle der Steuereinrichtung einen ersten Abschnitt (6301) mit einem ersten Durchmesser (D1) und einen zweiten Abschnitt (6302) mit einem zweiten Durchmesser (D2) aufweist, wobei der erste Durchmesser (D1) größer als der zweite Durchmesser (D2) ist;
der erste Zugdraht (1135) mit dem ersten Abschnitt (6301) verbunden ist und der zweite Zugdraht (1136) mit dem zweiten Abschnitt (6302) verbunden ist; und
eine Drehung der Wickelwelle eine Zugspannung auf den ersten Zugdraht (1135) mit einer höheren Rate steigert als die Drehung eine Zugspannung auf den zweiten Zugdraht (1136) steigert.

10. Vorrichtung nach Anspruch 7 oder Anspruch 8, wobei:
die Wickelwelle der Steuereinrichtung einen ersten Abschnitt (6301) aufweist, der eine erste Gewindesteigung (P1) umfasst, und einen zweiten Abschnitt (6302), der eine zweite Gewindesteigung (P2) umfasst, wobei die erste Gewindesteigung (P1) größer als die zweite Gewindesteigung (P2) ist;
der erste Zugdraht (1135) ist mit dem ersten Abschnitt (6301) verbunden ist und der zweite Zugdraht (1136) ist mit dem zweiten Abschnitt (6302) verbunden; und
eine Drehung der Wickelwelle eine Zugspannung auf den ersten Zugdraht (1135) mit einer höheren Rate steigert als die Drehung eine Zugspannung auf den zweiten Zugdraht (1136) steigert.

11. Vorrichtung nach einem der Ansprüche 1 bis 5, ferner umfassend:
ein proximales Ende des ersten Zugdrahts (1135), das mit einer ersten Wickelwelle (5903) einer Steuereinrichtung verbunden ist;
ein proximales Ende des zweiten Zugseils (1136), das mit einer zweiten Wickelwelle (5904) der Steuereinrichtung verbunden ist;
eine Kupplung (5906, 5907), die die erste Wickelwelle (5903) und die zweite Wickelwelle (5904) verbindet, wobei die Kupplung (5906, 5907) eine ausgerückte Stellung und eine eingerückte Stellung aufweist;
wobei dann, wenn sich die Kupplung (5906, 5907) in der ausgerückten Stellung befindet und die Steuereinrichtung betätigt wird, eine Zugspannung auf dem ersten Zugdraht (1135) erhöht wird; und
wobei dann, wenn sich die Kupplung (5906, 5907) in der eingerückten Stellung befindet und die Steuereinrichtung betätigt wird, die Zugspannung auf dem ersten Zugdraht (1135) und eine Zugspannung auf dem zweiten Zugdraht (1136) erhöht werden.

12. Vorrichtung nach einem der Ansprüche 1 bis 11, ferner umfassend:
eine erste Region (2040) mit erhöhter Steifigkeit, die sich in Längsrichtung entlang wenigstens eines Abschnitts des ersten biegbaren Abschnitts (116) erstreckt; und
eine zweite Region (2041) mit erhöhter Steifigkeit, die sich in Längsrichtung entlang wenigstens eines Abschnitts des zweiten biegbaren Abschnitts (115) erstreckt;
wobei vorzugsweise:
eine in Längsrichtung orientierte Mitte der ersten Region mit erhöhter Steifigkeit (2040) in Umfangsrichtung um 180 Grad gegenüber dem ersten Zugdraht (1135) versetzt ist; und
eine in Längsrichtung orientierte Mitte der zweiten Region (2041) mit erhöhter Steifigkeit in Umfangsrichtung um 180 Grad gegenüber dem zweiten Zugdraht (1136) versetzt ist.

13. Vorrichtung nach Anspruch 12, wobei:
die erste Region (2040) mit erhöhter Steifigkeit sich um einen ersten Bogen (θ) des Umfangs der biegbaren Röhre erstreckt, wobei der erste Bogen (θ) einen Winkel zwischen etwa 45 und etwa 135 Grad aufweist; und
die zweite Region (2041) mit erhöhter Steifigkeit sich um einen zweiten Bogen (β) des Umfangs der biegbaren Röhre erstreckt, wobei der zweite Bogen (β) einen Winkel zwischen etwa 45 und etwa 135 Grad aufweist.

14. Vorrichtung nach einem der Ansprüche 1 bis 13, wobei eine Zugspannung zuerst auf den ersten Zugdraht (1135) und dann auf den zweiten Zugdraht (1136) ausgeübt werden kann, oder wobei eine Zugspannung gleichzeitig auf den ersten Zugdraht (1135) und den zweiten Zugdraht (1136) ausgeübt werden kann, oder wobei eine Zugspannung zuerst auf den zweiten Zugdraht (1136) und dann auf den ersten Zugdraht (1135) ausgeübt werden kann.

15. Verfahren zum Herstellen einer Vorrichtung zum Zuführen eines Implantats zu einer nativen Klappe des Herzens eines Patienten, wobei das Verfahren umfasst:
Bereitstellen einer biegbaren hohlen Röhre (1114), die sich von einem proximalen Ende zu einem distalen Ende (118) erstreckt, wobei die biegbare hohle Röhre (1114) einen mittleren Abschnitt (620) aufweist, der zwischen dem proximalen Ende und dem distalen Ende (118) angeordnet ist;
Schneiden mehrerer erster Schlitze (125) in die biegbare hohle Röhre (1114) zwischen dem proximalen Ende und dem mittleren Abschnitt (620), um einen ersten biegbaren Abschnitt (116) zu bilden, der sich von dem proximalen Ende zu dem mittleren Abschnitt (620) erstreckt;
Schneiden mehrerer zweiter Schlitze (326) in die biegbare hohle Röhre (1114) zwischen dem mittleren Abschnitt (620) und dem distalen Ende (118), um einen zweiten biegbaren Abschnitt (115) zu bilden, der sich von dem mittleren Abschnitt (620) zu dem distalen Ende (118) erstreckt;
Anbringen eines ersten Zugdrahtes (1135) an dem mittleren Abschnitt (620), wobei sich der erste Zugdraht (1135) durch den ersten biegbaren Abschnitt (116) entlang einer ersten Seite der biegbaren hohlen Röhre (1114) erstreckt, wobei das Ausüben von Zugspannung auf den ersten Zugdraht (1135) in einer proximalen Richtung (660, 670) eine erste Biegung in dem ersten biegbaren Abschnitt (116) in einer ersten Richtung (664) erzeugt; und
Anbringen eines zweiten Zugdrahtes (1136) am distalen Ende (118), wobei sich der zweite Zugdraht (1136) durch den ersten und den zweiten biegbaren Abschnitt (116, 115) entlang einer zweiten Seite der biegbaren hohlen Röhre (1114) erstreckt, wobei das Ausüben von Zugspannung auf den zweiten Zugdraht (1136) in einer proximalen Richtung (660, 670) eine zweite Biegung in dem zweiten biegbaren Abschnitt (115) in einer zweiten Richtung (674) erzeugt;
wobei die zweite Seite gegenüber der ersten Seite um einen Versatzwinkel (α) versetzt ist, so dass die zweite Richtung (674) der zweiten Biegung ebenfalls gegenüber der ersten Richtung (664) der ersten Biegung um den Versatzwinkel (α) versetzt ist; und
wobei der Versatzwinkel (α) in einem Bereich von etwa 160 Grad bis etwa 200 Grad liegt.

## Revendications

1. Dispositif de pose d'un implant au niveau d'une valvule native du cœur d'un patient, comprenant:
un tube creux flexible (1114) s'étendant depuis une extrémité proximale à une extrémité distale (118), le tube creux flexible (1114) comprenant:
une première partie flexible (116) s'étendant depuis l'extrémité proximale à une partie centrale (620);
une seconde partie flexible (115) s'étendant depuis la partie centrale (620) à l'extrémité distale (118);
un premier fil de traction (1135) s'étendant à travers la première partie flexible (116) pour se fixer à la partie centrale (620), le premier fil de traction (1135) s'étendant le long d'un premier côté du tube creux flexible (1114), l'application d'une tension au premier fil de traction (1135) dans un sens proximal (660, 670) créant un premier coude dans la première partie flexible (116) dans un premier sens (664);
un second fil de traction (1136) s'étendant à travers les première et seconde parties flexibles (116, 115) pour se fixer à l'extrémité distale (118), le second fil de traction (1136) s'étendant le long d'un second côté du tube creux flexible (1114), l'application d'une tension au second fil de traction (1136) dans un sens proximal (660, 670) créant un second coude dans la seconde partie flexible (115) dans un second sens (674);
le second côté étant décalé d'un angle de décalage (α) par rapport au premier côté de telle sorte que le second sens (674) du second coude est également décalé de l'angle de décalage (α) par rapport au premier sens (664) du premier coude; et
l'angle de décalage (α) étant situé dans une plage d'environ 160 degrés à environ 200 degrés.

2. Dispositif selon la revendication 1, la partie centrale (620) comprenant une première bague de traction (2037) et l'extrémité distale (118) comprenant une seconde bague de traction (2038); de préférence la première bague de traction (2037) étant formée d'un seul tenant avec la partie centrale (620) et/ou la seconde bague de traction (2038) étant formée d'un seul tenant avec l'extrémité distale (118).

3. Dispositif selon l'une quelconque des revendications 1 ou 2, comprenant en outre:
un premier conduit (2210) intégré à l'intérieur et s'étendant le long du premier côté du tube flexible, le premier conduit (2210) s'étendant à travers la première partie flexible (116) et au moins une partie de la partie centrale (620) et le premier fil de traction (1135) s'étendant à travers le premier conduit (2210); et
un second conduit (2212) intégré à l'intérieur et s'étendant le long du second côté du tube flexible décalé de l'angle de décalage (α) par rapport au premier conduit (2210), le second conduit (2212) s'étendant à travers la première partie flexible (116), la partie centrale (620) et la seconde partie flexible (115) et le second fil de traction (1136) s'étendant à travers le second conduit (2212).

4. Dispositif selon l'une quelconque des revendications 1 à 3:
une extrémité proximale du premier fil de traction (1135) étant reliée à un premier mécanisme de commande (650; 5901) afin de commander la tension sur le premier fil de traction (1135); et
une extrémité proximale du second fil de traction (1136) étant reliée à un second mécanisme de commande (660; 5902) afin de commander la tension sur le second fil de traction (1136).

5. Dispositif selon l'une quelconque des revendications 1 à 4, le premier coude formant un angle entre zéro et 180 degrés et le second coude formant un angle entre zéro et 60 degrés; de préférence le premier coude formant un angle entre zéro et 90 degrés et le second coude formant un angle entre zéro et 45 degrés.

6. Dispositif selon l'une quelconque des revendications 4 ou 5:
le premier mécanisme de commande (650; 5901) comprenant une première tige (5903), la première tige (5903) pouvant tourner pour enrouler le premier fil de traction (1135) afin d'appliquer une tension au premier fil de traction (1135); et
le second mécanisme de commande (660; 5902) comprenant une seconde tige (5904), la seconde fige (5904) pouvant tourner pour enrouler le second fil de traction (1136) afin d'appliquer une tension au second fil de traction (1136).

7. Dispositif selon l'une quelconque des revendications 1 à 6, une extrémité proximale du premier fil de traction (1135) et une extrémité proximale du second fil de traction (1136) étant reliées à une tige d'un mécanisme de commande (5901, 5902).

8. Dispositif selon l'une quelconque des revendications 1 à 7:
le second fil de traction (1136) comprenant une partie lâche (5908) lorsque le tube creux flexible (1114) est dans une configuration droite;
la partie lâche (5908) étant réduite lorsqu'une tension est d'abord appliquée au second fil de traction (1136); et
la partie lâche (5908) étant éliminée après la création du second coude par l'application continue de tension au second fil de traction (1136).

9. Dispositif selon la revendication 7 ou la revendication 8:
la tige du mécanisme de commande présentant une première partie (6301) présentant un premier diamètre (D1) et une seconde partie (6302) présentant un second diamètre (D2), le premier diamètre (D1) étant supérieur au second diamètre (D2);
le premier fil de traction (1135) étant relié à la première partie (6301) et le second fil de traction (1136) étant relié à la seconde partie (6302); et
une rotation de la tige augmentant une tension sur le premier fil de traction (1135) à un taux supérieur à l'augmentation d'une tension sur le second fil de traction (1136) par la rotation.

10. Dispositif selon la revendication 7 ou la revendication 8:
la tige du mécanisme de commande présentant une première partie (6301) comprenant un premier pas de filetage (P1) et une seconde partie (6302) comprenant un second pas de filetage (P2), le premier pas de filetage (P1) étant supérieur au second pas de filetage (P2);
le premier fil de traction (1135) étant relié à la première partie (6301) et le second fil de traction (1136) étant relié à la seconde partie (6302); et
une rotation de la tige augmentant une tension sur le premier fil de traction (1135) à un taux supérieur à l'augmentation d'une tension sur le second fil de traction (1136) par la rotation.

11. Dispositif selon l'une quelconque des revendications 1 à 5, comprenant en outre:
une extrémité proximale du premier fil de traction (1135) qui est reliée à une première tige (5903) d'un mécanisme de commande;
une extrémité proximale du second fil de traction (1136) qui est reliée à une seconde tige (5904) du mécanisme de commande;
un embrayage (5906, 5907) reliant la première tige (5903) et la seconde tige (5904), l'embrayage (5906, 5907) présentant une position débrayée et une position embrayée;
où, lorsque l'embrayage (5906, 5907) est dans la position débrayée et que le mécanisme de commande est actionné, une tension sur le premier fil de traction (1135) est augmentée; et
où, lorsque l'embrayage (5906, 5907) est dans la position embrayée et que le mécanisme de commande est actionné, la tension sur le premier fil de traction (1135) et une tension sur le second fil de traction (1136) sont augmentées.

12. Dispositif selon l'une quelconque des revendications 1 à 11, comprenant en outre:
une première région (2040) à rigidité accrue s'étendant longitudinalement le long d'au moins une partie de la première partie flexible (116); et
une seconde région (2041) à rigidité accrue s'étendant longitudinalement le long d'au moins une partie de la seconde partie flexible (115);
de préférence:
un centre orienté longitudinalement de la première région (2040) à rigidité accrue étant décalé de manière circonférentielle de 180 degrés par rapport au premier fil de traction (1135); et
un centre orienté longitudinalement de la seconde région (2041) à rigidité accrue étant décalé de manière circonférentielle de 180 degrés par rapport au second fil de traction (1136).

13. Dispositif selon la revendication 12,:
la première région (2040) à rigidité accrue s'étendant autour d'un premier arc (θ) d'une circonférence du tube flexible, le premier arc (θ) présentant un angle entre environ 45 et environ 135 degrés; et
la seconde région (2041) à rigidité accrue s'étendant autour d'un second arc (β) de la circonférence du tube flexible, le second arc (β) présentant un angle entre environ 45 et environ 135 degrés.

14. Dispositif selon l'une quelconque des revendications 1 à 13, une tension pouvant d'abord être appliquée au premier fil de traction (1135) puis au second fil de traction (1136), ou une tension pouvant être appliquée simultanément au premier fil de traction (1135) et au second fil de traction (1136) ou une tension pouvant d'abord être appliquée au second fil de traction (1136) puis au premier fil de traction (1135).

15. Procédé de fabrication d'un dispositif de pose d'un implant au niveau d'une valvule native du cœur d'un patient, le procédé comprenant:
la fourniture d'un tube creux flexible (1114) s'étendant depuis une extrémité proximale à une extrémité distale (118), le tube creux flexible (1114) comprenant une partie centrale (620) disposée entre l'extrémité proximale et l'extrémité distale (118);
la découpe d'une pluralité de premières fentes (125) dans le tube creux flexible (1114) entre l'extrémité proximale et la partie centrale (620) afin de former une première partie flexible (116) s'étendant depuis l'extrémité proximale à la partie centrale (620);
la découpe d'une pluralité de secondes fentes (326) dans le tube creux flexible (1114) entre la partie centrale (620) et l'extrémité distale (118) afin de former une seconde partie flexible (115) s'étendant depuis la partie centrale (620) à l'extrémité distale (118);
la fixation d'un premier fil de traction (1135) à la partie centrale (620), le premier fil de traction (1135) s'étendant à travers la première partie flexible (116) le long d'un premier côté du tube creux flexible (1114), l'application d'une tension au premier fil de traction (1135) dans un sens proximal (660, 670) créant un premier coude dans la première partie flexible (116) dans un premier sens (664); et
la fixation d'un second fil de traction (1136) à l'extrémité distale (118), le second fil de traction (1136) s'étendant à travers les première et seconde parties flexibles (116, 115) le long d'un second côté du tube creux flexible (1114), l'application d'une tension au second fil de traction (1136) dans un sens proximal (660, 670) créant un second coude dans la seconde partie flexible (115) dans un second sens (674);
le second côté étant décalé d'un angle de décalage (α) par rapport au premier côté de telle sorte que le second sens (674) du second coude est également décalé de l'angle de décalage (α) par rapport au premier sens (664) du premier coude; et
l'angle de décalage (α) étant situé dans une plage d'environ 160 degrés à environ 200 degrés.
